# EUROPEAN PATENT SPECIFICATION

(11) **EP 4 562 158 B1**
(45) Date of publication and mention of the grant of the patent: **28.10.2026**
(21) Application number: 24740290.2
(22) Date of filing: 13.06.2024
(51) Int. Cl.: C12N 15/113, A61K 31/713

(54) **ADVANCED RNA TARGETING (ARNATAR) FOR ANGIOTENSINOGEN**
VERBESSERTES RNA-TARGETING (ARNATAR) FÜR ANGIOTENSINOGEN
CIBLAGE D'ARN AVANCÉ (ARNATAR) POUR ANGIOTENSINOGÈNE

(30) Priority: 13.06.2023 US 202363472727 P; 13.06.2023 US 202363472780 P; 17.08.2023 US 202363533283 P
(43) Date of publication of application: 04.06.2025
(73) Proprietor: Arnatar Therapeutics, Inc, San Diego, CA 92121 (US)
(72) Inventor: LIEU, Yen, San Diego, CA 92121 (US); ZHANG, Lingdi, San Diego, CA 92121 (US); LIANG, Xuehai, San Diego, CA 92121 (US); WANG, Yanfeng, San Diego, CA 92121 (US)
(74) Representative: Schiener, Jens
(86) International application number: PCT/US2024/033852
(87) International publication number: WO 2024/259134

(56) References cited:
- WO-A1-2015/179724
- WO-A1-2019/222166
- WO-A1-2023/014765
- WO-A1-2024/137543
- JEFFREY OLEARCZYK ET AL: "Targeting of hepatic angiotensinogen using chemically modified siRNAs results in significant and sustained blood pressure lowering in a rat model of hypertension", HYPERTENSION RESEARCH, vol. 37, no. 5, 12 December 2013 (2013-12-12), pages 405 - 412, XP055206991, ISSN: 0916-9636, DOI: 10.1038/hr.2013.155
- OLEARCZYK JEFFREY ET AL: "Supplementary information: Targeting of hepatic angiotensinogen using chemically modified siRNAs results in significant and sustained blood pressure lowering in a rat model of hypertension", HYPERTENSION RESEARCH, 12 December 2013 (2013-12-12), XP093205115, Retrieved from the Internet <URL:https://static-content.springer.com/esm/art%3A10.1038%2Fhr.2013.155/MediaObjects/41440_2014_BFhr2013155_MOESM6_ESM.doc>
- REN LIWEI ET AL: "Targeting angiotensinogen with RNA-based therapeutics :", CURRENT OPINION IN NEPHROLOGY & HYPERTENSION, vol. 29, no. 2, March 2020 (2020-03-01), GB, pages 180 - 189, XP055981374, ISSN: 1062-4821, DOI: 10.1097/MNH.0000000000000586

## Description

### FIELD

Certain embodiments are directed to compounds for modulating angiotensinogen (AGT) gene expression by Advanced RNA Targeting (ARNATAR). Such compounds are useful for reducing expression of AGT, thereby treating diseases, disorders and/or conditions related to AGT in a subject.

### BACKGROUND

Angiotensinogen (also known as AGT, SERPINA8 or ANHU), a member of the serpin family, is a component of the renin-angiotensin system (RAS) pathway (also known as the renin-angiotensin-aldosterone system (RAAS)). It is primarily produced in the liver and is released into the circulation where renin converts it into angiotensin I. Angiotensin converting enzyme (ACE) then converts angiotensin I into angiotensin II.

Angiotensin II, a peptide hormone, has multiple effects on the circulatory system that controls blood pressure and may contribute to hypertension. Angiotensin II causes vasoconstriction which, in turn, can increase blood pressure. Angiotensin II stimulates secretion of the hormone aldosterone from the adrenal cortex. Aldosterone causes the kidneys to increase reabsorption of sodium and modulation of the glomerular filtration rate which can lead to an increase of fluid volume in a body which, in turn, can increase blood pressure. Excess angiotensin II can lead to dysregulation of the RAAS pathway and high blood pressure.

Chronic high blood pressure is known as hypertension. High blood pressure in a hypertensive subject requires the heart to work harder to circulate blood through the blood vessels. Hypertension can lead to many deleterious conditions in a body such as increased oxidative stress, increased inflammation, hypertrophy, fibrosis in the heart, fibrosis in the kidneys, and fibrosis in the arteries, and result in, e.g., left ventricular fibrosis, arterial remodeling, and glomerulosclerosis.

Hypertension is known as a major risk factor for various diseases, disorders and conditions such as shortened life expectancy due to, for example, cardiovascular morbidity, chronic kidney disease, stroke, myocardial infarction, heart failure, aneurysms of the blood vessels (e.g. aortic aneurysm), peripheral artery disease, heart damage (e.g., heart enlargement or hypertrophy) and other cardiovascular related diseases, disorders and/or conditions.

The prevalence of resistant hypertension (RHTN), hypertension resistant to drug treatment, has steadily increased in number likely due to an ageing population and an ever-increasing incidence of obesity despite the number of anti-hypertensive drugs commercially available. Also, commercially available anti-hypertensive therapies targeting various RAAS pathway components have been ineffective in completely inhibiting or blocking the RAAS pathway. The mechanism(s) for this ineffective inhibition has not been fully elucidated, but may occur by ACE escape and/or aldosterone escape pathways. Thus, there is a need for new therapeutics.

The use of therapeutic oligomeric compounds (e.g., oligonucleotides) was first proposed over forty years ago by Stephenson and Zamecnik (Inhibition of Rous Sarcoma Viral RNA Translation by a Specific Oligodeoxyribonucleotide, PNAS, 1978, 75:285-288).

Sequence-specific silencing of gene expression, RNA interference (RNAi), was discovered in 1998 by Fire et al. (Potent and Specific Genetic Interference by Double-Stranded RNA in Caenorhabditis elegans, Nature, 1998, 391:806-811). RNAi utilizes double-stranded RNA (dsRNA) to inhibit gene expression via the RNA-induced silencing complex (RISC).

RISC comprises a complex of multiple proteins interacting with an oligomeric compound to inhibit gene expression. The oligomeric compound acts as a template for RISC to recognize complementary messenger RNA (mRNA) transcripts to target a specific mRNA transcript for cleavage. Cleavage of the target mRNA blocks translation of the target mRNA and silences the target gene. Oligomeric compounds utilized by RISC include, but are not limited to: single stranded oligomeric compounds such as microRNAs (miRNAs), certain oligonucleotides, and single strand siRNAs (Lima et al., Single-stranded siRNAs activate RNAi in animals. Cell. 2012, 150(5):883-94), and double stranded RNA (dsRNA) compounds such as short hairpin RNAs (shRNAs) and small interfering RNAs (siRNAs).

In 2001, Elbashir et al., showed that 21-nucleotide long siRNA duplexes specifically suppress expression of endogenous and heterologous genes in mammalian cell lines and theorized that siRNA may eventually be used as a gene-specific therapeutic (Duplexes of 21-Nucleotide RNAs Mediate RNA Interference in Cultured Mammalian Cells, Nature, 2001, 411:494-498).

The field of oligomeric therapeutic compounds is still maturing and improvements in delivery, stability, specificity, safety and potency are still being sought to improve the therapeutic efficacy of the oligomeric compound.

For example, international application WO 2023/014765 A1 discloses iRNA compositions and methods for siliencing angiotensinogen (AGT).

Currently, several clinical trials are underway assessing nucleic acid-based therapeutics targeting AGT to treat hypertension. In clinical trials, both an siRNA therapeutic and an antisense oligonucleotide (ASO) therapeutic are being assessed (Ranasinghe et al., J Am Heart Assoc, 2022, 11(20); Cruz-López et al., Hypertension, 2022, 79:2115-2126; Morgan et al., Clinical Res, 2021, 6(6):4865-96), however, neither type of nucleic acid therapeutic has been given regulatory approval for patient treatment. Thus, there is still an unmet need for nucleic acid-based therapeutics targeting AGT. Disclosed herein are dsRNA compounds targeting AGT improved with Advanced RNA Targeting (ARNATAR) abilities that enhance their gene silencing activity.

### SUMMARY

Several embodiments provided herein relate to the discovery of certain ARNATAR design dsRNA compounds targeting AGT transcripts that can enhance their effectiveness in modulating AGT gene expression. In several aspects, the dsRNA compound is a modified shRNA or siRNA compound. The double-stranded RNA compound comprises a sense strand and an antisense strand. The antisense strands can be fully or substantially complementary to a target nucleic acid.

In some embodiments, a double stranded ribonucleic acid (dsRNA) compound for inhibiting expression of angiotensinogen (AGT) in a cell comprises a sense strand and an antisense strand forming a double stranded portion, as defined in the appended claims.

In certain embodiments, the dsRNA compound comprises a conjugate. In certain embodiments, the conjugate is N-Acetylgalactosamine (GalNAc). In certain embodiments, the conjugate is attached to the 3' end of the sense strand of a dsRNA compound targeting AGT. In certain embodiments, the conjugate is attached to the 3' end of the sense strand of a siRNA compound targeting AGT.

In certain embodiments, a kit comprising the dsRNA compound or a pharmaceutical composition described herein, and optionally, a label, is provided.

Certain embodiments provide the double stranded ribonucleic acid (dsRNA) compound for use in medicine.

Certain embodiments provide the double stranded ribonucleic acid (dsRNA) compound for use in treating or preventing a RAAS associated disease, disorder and/or condition in a subject.

### BRIEF DESCRIPTION OF THE DRAWINGS

**FIGURE 1****:** shows AGT siRNA inhibition screen in human primary hepatocyte cells after 48 hours free uptake.
**FIGURE 2****:** shows the dose response curves of AGT siRNAs on AGT expression (percent) after transfection of AGT siRNAs into Hep3B for 24 hours to compare ARNATAR modifications to Third Party modifications of the same sequences without GalNAc.
**FIGURE 3****:** shows ARNATAR AGT siRNA ATsi483 with comparable inhibition activity as reference AGT siRNA ATXL-G in human primary hepatocyte cells after 42 hours free uptake.
**FIGURE 4****:** shows *in vivo* AGT protein levels via ELISA in blood plasma after siRNA dosing, illustrating comparable activity and durability of ARNATAR AGT siRNAs compared to reference AGT siRNA ATXL-G over a >4-week time period.
**FIGURE 5****:** shows *in vivo* plasma AGT protein levels after dosing with further modifications of ARNATAR AGT siRNAs, demonstrating comparable activity and durability as reference AGT siRNA ATXL-G over a time course of 4 weeks.
**FIGURE 6****:** shows *in vitro* inhibition activity via 42 hours free uptake by human primary hepatocytes of ARNATAR modified AGT siRNAs and reference ATXL-G.
**FIGURE 7****:** shows *in vivo* plasma AGT protein levels of some of ARNATAR AGT siRNAs that have lower activity *in vitro* but comparable or better activity than reference ATXL-G *in vivo.*
**FIGURE 8****:** shows dose response curves of AGT siRNAs on AGT expression (percent) after transfection of ARNATAR AGT siRNAs into Hep3B for 8 or 12 hours, demonstrating better activity than reference ATXL-G for some ARNATAR siRNAs.
**FIGURE 9****:** shows percent AGT protein levels in mouse plasma at different times after siRNA dosing at 3 mg/kg, showing similar or better activity at several time points than reference ATXL-G.
**FIGURE 10****:** shows percent AGT protein levels in mouse plasma of AGT-AAV transgenic mice at different times after siRNA dosing at 3 mg/kg with compound ATsi786 showing more potent knockdown than reference ATXL-G.

### DETAILED DESCRIPTION

It is to be understood that both the foregoing general description and the following detailed description are exemplary and explanatory only and are not restrictive of the invention, as claimed. Herein, the use of the singular includes the plural unless specifically stated otherwise. As used herein, the use of "or" means "and/or" unless stated otherwise. Furthermore, the use of the term "including" as well as other forms, such as "includes" and "included", is not limiting. Also, terms such as "element" or "component" encompass both elements and components comprising one unit and elements and components that comprise more than one subunit, unless specifically stated otherwise.

The section headings used herein are for organizational purposes only and are not to be construed as limiting the subject matter described.

### Definitions

Unless specific definitions are provided, the nomenclature utilized in connection with, and the procedures and techniques of, analytical chemistry, synthetic organic chemistry, and medicinal and pharmaceutical chemistry described herein are those well-known and commonly used in the art. Standard techniques may be used for chemical synthesis, and chemical analysis.

Unless otherwise indicated, the following terms have the following meanings:
"2'-O-methoxyethyl" (also 2'-MOE and 2'-O(CH₂)₂-OCH₃) refers to an O-methoxy-ethyl modification at the 2' position of a furanose ring. A 2'-O-methoxyethyl modified sugar is a modified sugar.

"2'-MOE nucleoside" (also 2'-O-methoxyethyl nucleoside) means a nucleoside comprising a 2'-MOE modified sugar moiety. "2'-MOE nucleotide" (also 2'-O-methoxyethyl nucleotide) means a nucleotide comprising a 2'-MOE modified sugar moiety.

"2'-O-methyl" (also 2'-OCH₃ and 2'-OMe) refers to an O-methyl modification at the 2' position of a furanose ring. A 2'-O-methyl modified sugar is a modified sugar.

"2'-OMe nucleoside" (also 2'-O-methyl nucleoside) means a nucleoside comprising a 2'-OMe modified sugar moiety. "2'-OMe nucleotide" (also 2'-O-methyl nucleotide) means a nucleotide comprising a 2'-OMe modified sugar moiety.

"2'-substituted nucleoside" means a nucleoside comprising a substituent at the 2'-position of the furanose ring other than H or OH. In certain embodiments, 2' substituted nucleosides include nucleosides with a fluoro (2'-F), O-methyl (2'-OMe), O-methoxyethyl (2'-MOE) or bicyclic sugar modifications.

"5-methylcytosine" means a cytosine modified with a methyl group attached to the 5 position. A 5-methylcytosine is a modified nucleobase.

"About" means within ±7% of a value. For example, if it is stated, "the compounds affected at least about 70% inhibition of mRNA", it is implied that the mRNA levels are inhibited within a range of 63% and 77%.

"Animal" refers to a human or non-human animal, including, but not limited to, mice, rats, rabbits, dogs, cats, pigs, and non-human primates, including, but not limited to, monkeys and chimpanzees.

"Antibody" refers to a complete antibody molecule or any fragment or portion thereof, such as the heavy chain, the light chain, F_{ab} portion, and F_{c} portion.

"Antisense oligonucleotide" or "ASO" means a single-stranded oligonucleotide having a nucleobase sequence that permits hybridization to a corresponding region or segment of a target nucleic acid. The antisense oligonucleotide can comprise one or more ribonucleoside (RNA) residues and/or deoxyribonucleoside (DNA) residues.

"Base complementarity" refers to the capacity for the base pairing of nucleobases of an oligonucleotide with corresponding nucleobases in a target nucleic acid (i.e., hybridization), and is mediated by Watson-Crick, Hoogsteen or reversed Hoogsteen hydrogen binding between corresponding nucleobases. Base complementarity also refers to canonical (e.g., A:U, A:T, C:G) or non-canonical base pairings (e.g., A:G, A:U, G:U, I:U, I:A, I:C).

"Bicyclic sugar" means a furanose ring modified by the bridging of two non-geminal carbon atoms. A bicyclic sugar is a modified sugar.

"Cap structure" or "terminal cap moiety" means chemical modifications, which have been incorporated at either terminus of an oligomeric compound.

"Chemical modification" means modification of molecular structure or element from naturally occurring molecules. For example, siRNA compounds are composed of linked ribonucleosides (also sometimes referred to herein as RNA), therefore, substitution of a deoxyribonucleoside (also sometimes referred to herein as DNA) for a ribonucleoside is considered a chemical modification of the siRNA compound.

"Chemically distinct portion" refers to a portion of an oligomeric compound that is in some way chemically different than another portion of the same oligomeric compound. For example, a portion having 2'-OMe nucleotides is chemically distinct from a portion having nucleotides without 2'-OMe modifications.

"Chimeric oligomeric compounds" means oligomeric compounds that have at least 2 chemically distinct portions, each portion having a plurality of subunits. For example, as disclosed herein, siRNA can comprise a peripheral portion and a central portion. The peripheral portion comprises motifs with various modified or unmodified nucleobases so as to confer increased stability, specificity, safety and potency, while the central portion comprises various modified or unmodified nucleobases to serve as substrate for RISC mediated degradation.

"Complementarity" means the capacity for pairing between nucleobases of a first nucleic acid and a second nucleic acid.

"Comply" in the context of a therapy means the adherence with a recommended therapy by a subject.

"Comprise", "comprises" and "comprising" will be understood to imply the inclusion of a stated member without the exclusion of other (e.g., non-stated) members. For example, the inclusion of a stated step or element or group of steps or elements but not the exclusion of any other step or element or group of steps or elements, respectively.

"Contiguous nucleobases" means nucleobases adjacent to each other.

"Deoxyribonucleotide" means a nucleotide having a hydrogen at the 2' position of the sugar portion of the nucleotide. A sequence of deoxyribonucleotides is sometimes referred to as "DNA" herein. A deoxyribonucleotide is sometimes referred to as "DNA nucleotide", "d nucleotide" or "D" herein. Deoxyribonucleotides may be modified with any of a variety of substituents.

"Designing" or "designed" in the context of an oligomeric compound refer to the process of making/engineering an oligomeric compound that specifically hybridizes with a target nucleic acid molecule. Designing generally encompasses providing mutations and/or modifications to a e.g., natural sequence.

"Efficacy" means the ability to produce a desired effect.

"Expression" generally includes all the functions by which a gene's coded information is converted into structures present and operating in a cell. Such structures include, but are not limited to the products of transcription and/or translation. Within the present disclosure, expression refers to protein as the products of expression.

"Fully complementary" or "100% complementary" means each nucleobase of a first nucleic acid molecule has a complementary nucleobase in a second nucleic acid molecule. A first nucleic acid molecule can be an oligomeric compound and a target nucleic acid molecule is a second nucleic acid molecule.

"Fully modified motif" refers to an oligomeric compound comprising a contiguous sequence of nucleosides wherein each nucleoside has a chemical modification.

"GalNAc" refers to the compound N-Acetylgalactosamine or to a compound comprising a N-Acetylgalactosamine compound. A GalNAc-comprising moiety (or sometimes only "GalNAc" or "GalNAc moiety" used herein) describes a compound comprising at least one N-Acetylgalactosamine compound usually attached to one or more spacers and/or linkers for the attachment to an oligonucleotide compound.

"Hybridization" means the annealing of complementary nucleic acid molecules. Complementary nucleic acid molecules can include, but are not limited to, a combination of an oligomeric compound and a nucleic acid molecule target. Complementary nucleic acid molecules can include, but are not limited to, a combination of an siRNA and a nucleic acid molecule target, particularly an mRNA target molecule.

"Induce", "inhibit", "potentiate", "elevate", "increase", "decrease" or the like, generally denotes an action to obtain quantitative differences between two states.

"Inhibiting the expression or activity" refers to a reduction or blockade of the expression or activity and does not necessarily indicate a total elimination of expression or activity.

"Internucleoside linkage" refers to the chemical bond between two adjacent nucleosides. The linkage may be a naturally occurring linkage, i.e., a phosphate linkage, or an artificial linkage, such as a phosphorothioate (also known as thiophosphate or PS) linkage.

"Linked nucleosides" means adjacent nucleosides (e.g., A, G, C, T, U, and I) linked together by an internucleoside linkage. Examples of linked nucleosides include a sequence of deoxyribonucleosides (sometimes referred to as DNA herein) or a sequence of ribonucleosides (sometimes referred to as RNA herein).

"Mismatch" or "non-complementary nucleobase" refers to the case when a nucleobase of a first nucleic acid is not capable of pairing with the corresponding nucleobase of a second or target nucleic acid through Watson-Crick base-pairing (e.g., A:T, A:U, C:G).

"Modified internucleoside linkage" refers to a substitution or any change from a naturally occurring internucleoside bond (i.e., a phosphodiester internucleoside bond).

"Modified nucleobase" means any nucleobase other than adenine, cytosine, guanine, thymidine, or uracil. An "unmodified nucleobase" means the purine bases adenine (A) and guanine (G), and the pyrimidine bases thymine (T), cytosine (C) and uracil (U).

"Modified nucleoside" means a nucleoside having a modified sugar moiety and/or a modified nucleobase. As used herein, where the oligomeric compound is RNA-based, a substitution of a deoxyribonucleoside (sometimes referred to as DNA nucleoside herein) for a ribonucleoside is considered a modification of the oligomeric compound. Also, where the oligomeric compound is DNA-based, a substitution of a ribonucleoside (sometimes referred to as RNA nucleoside herein) for a deoxyribonucleoside is considered a modification of the oligomeric compound.

"Modified nucleotide" means a nucleotide having at least one of a modified sugar moiety, a modified internucleoside linkage, a deoxyribonucleoside (sometimes referred to as DNA nucleotide herein) for a ribonucleoside (sometimes referred to as RNA nucleotide herein) substitution, a ribonucleoside (sometimes referred to as RNA nucleoside herein) for a deoxyribonucleoside (sometimes referred to as DNA nucleoside herein) substitution, and a modified nucleobase.

"Modified oligonucleotide" means an oligonucleotide comprising at least one of a modified internucleoside linkage, a modified sugar, a deoxyribonucleoside (sometimes referred to as DNA nucleoside herein) for a ribonucleoside (sometimes referred to as RNA nucleoside herein) substitution, a ribonucleoside (sometimes referred to as RNA nucleoside herein) for a deoxyribonucleoside (sometimes referred to as DNA nucleoside herein) substitution, and/or a modified nucleobase.

"Modified sugar" means substitution and/or any change from a natural sugar moiety of a nucleotide found in DNA or RNA.

"Moiety" means one of the portions into which something is divided i.e., a part or component of something. For example, a sugar moiety of a nucleotide is the sugar component of the nucleotide.

"Monomer" refers to a single unit of an oligomer or a single unit for forming an oligomer. Monomers include, but are not limited to, nucleosides and nucleotides, whether naturally occuring or modified.

"Motif" means a pattern of modification in an oligomeric compound. For example, as disclosed herein, ARNATAR designed oligomeric compounds comprising motifs with various modified nucleobases and internucleoside linkages in order to improve delivery, stability, specificity, safety and potency of the compounds.

"Natural sugar (moiety)" or generally "sugar (moiety)" means a sugar (moiety) found in DNA (2'-H) or RNA (2'-OH), i.e., 2-deoxy-beta-D-ribofuranose or beta-D-ribofuranose, respectively.

"Naturally occurring internucleoside linkage" means a 3' to 5' phosphodiester linkage.

"Non-complementary nucleobase" refers to a pair of nucleobases that do not form hydrogen bonds with one another or otherwise support hybridization.

"Nucleic acid (molecule)" refers to a sequence of monomeric nucleotides. A nucleic acid molecule includes, but is not limited to, ribonucleic acids (RNA), messenger RNA (mRNA), deoxyribonucleic acids (DNA), single-stranded nucleic acids, double-stranded nucleic acids, small interfering ribonucleic acids (siRNA), hairpin ribonucleic acids (shRNA) and microRNAs (miRNA).

"Nucleobase" means any unmodified nucleobase as defined above, any modified nucleobase and/or any artificial nucleobase that may generally be any heterocyclic moiety capable of pairing with a base of a nucleic acid molecule.

"Nucleobase complementarity" refers to the ability of a nucleobase of base pairing (also known as being complementary) with another nucleobase. If a nucleobase at a certain position of an oligomeric compound is capable of hydrogen bonding with a nucleobase at a certain position of a target nucleic acid molecule, then the position of hydrogen bonding between the oligomeric compound and the target nucleic acid molecule is considered to be complementary at that nucleobase pair. For example, in DNA, adenine (A) is complementary to thymine (T); in RNA, adenine (A) is complementary to uracil (U); and, guanine (G) is complementary to cytosine (C) in both DNA and RNA. Base pairs, or complementary nucleobases, are usually canonical Watson-Crick base pairs (C:G, A:U, A:T), but, non-canonical base pairs such as Hoogsteen base pairs (e.g., A:G, A:U), Wobble base pairs (e.g., G:U, I:U, I:A, I:C, wherein I is hypoxanthine) and the like are also included. Nucleobase complementarity facilitates hybridization of the oligomeric compounds described herein to their target nucleic acids.

"Nucleobase sequence" means the order of contiguous nucleobases independent of any sugar, linkage, and/or nucleobase modification.

"Nucleoside" means a nucleobase linked to the natural or modified sugar as defined above.

"Nucleoside mimetic" includes those structures used to replace the sugar or the sugar and the base and not necessarily the linkage at one or more positions of an oligomeric compound such as for example nucleoside mimetics having morpholino, cyclohexenyl, cyclohexyl, tetrahydropyranyl, bicyclo or tricyclo sugar mimetics, *e.g*., non furanose sugar units. Nucleotide mimetic includes those structures used to replace the nucleoside and the linkage at one or more positions of an oligomeric compound such as for example peptide nucleic acids or morpholinos (morpholinos linked by -N(H)-C(=O)-O- or other non-phosphodiester linkage). Sugar surrogate overlaps with the slightly broader term nucleoside mimetic but is intended to indicate replacement of the sugar unit (furanose ring) only. The tetrahydropyranyl rings provided herein are illustrative of an example of a sugar surrogate wherein the furanose sugar group has been replaced with a tetrahydropyranyl ring system. "Mimetic" refers to groups that are substituted for a sugar, a nucleobase, and/or internucleoside linkage. Generally, a mimetic is used in place of the sugar or sugar-internucleoside linkage combination, and the nucleobase is maintained for hybridization to a selected target.

"Nucleotide" means a nucleoside having a linkage group (e.g., a phosphate (p) or phosphorothioate (PS) group) covalently linked to the sugar portion of the nucleoside. Nucleotides include ribonucleotides and deoxyribonucleotides with phosphate and/or phosphorothioate linkages. A sequence of linked nucleotides, e.g., ribonucleotides, deoxyribonucleotides and/or a mixture thereof, form an oligonucleotide.

"Off-target effect" refers to an unwanted or deleterious biological effect associated with modulation of RNA or protein expression of a gene other than the intended target nucleic acid.

"Oligomeric activity" means any detectable or measurable activity attributable to the hybridization of an oligomeric compound to its target nucleic acid. Oligomeric activity can be measured as a decrease in the amount or expression of a target nucleic acid. Oligomeric activity can be modulated by an oligomeric compound such as a dsRNA. Oligomeric activity can be modulated by an oligomeric compound such as an siRNA.

"Oligomeric compound" means a compound of linked monomeric subunits (also known as "subunits" herein) that is capable of undergoing hybridization to at least a region of a target nucleic acid through hydrogen bonding. The monomeric subunits are particularly modified or unmodified nucleotides or nucleosides, and the oligomeric compound is particularly an oligonucleotide. The oligomeric compound acts as a template for RISC to recognize complementary messenger RNA (mRNA) transcripts to target a specific mRNA transcript for cleavage. Cleavage of the target mRNA blocks translation of the target mRNA and silences the target gene. Examples of oligomeric compounds include single-stranded and double-stranded compounds, such as, antisense oligonucleotides, ssRNAs, siRNAs, shRNAs and miRNAs. Generally, oligomeric compounds are distinguished from polymeric compounds based on their number of monomers, wherein an oligomer often has about 5 to about 100 monomeric units.

"Oligomeric inhibition" means reduction of target nucleic acid (e.g., mRNA) levels in the presence of an oligomeric compound complementary to a target nucleic acid compared to target nucleic acid levels in the absence of the oligomeric compound.

"Oligomeric mechanisms" include RISC or RNase H related mechanisms involving hybridization of an oligomeric compound with target nucleic acid (e.g., mRNA), wherein the outcome or effect of the hybridization is target degradation and inhibition of gene expression.

"Oligonucleotide" as used herein means a sequence of linked nucleosides and/or nucleotides, each of which can be modified or unmodified, independently from each other. Oligonucleotides can have a linking group other than a phosphate group (e.g., a phosphorothioate group) used as a linking moiety between nucleosides. An oligonucleotide can comprise one or more ribonucleoside (RNA) residues and/or deoxyribonucleoside (DNA) residues.

"Phosphorothioate linkage" or "PS" means a linkage between nucleosides where the phosphodiester bond is modified by replacing one of the non-bridging oxygen atoms with a sulfur atom. A phosphorothioate linkage is a modified internucleoside linkage.

"Portion" means a defined number of contiguous (i.e., linked) nucleobases of a nucleic acid. A portion can be a defined number of contiguous nucleobases of a target nucleic acid. A portion can be a defined number of contiguous nucleobases of an oligomeric compound.

"Region" is defined as a portion of the target nucleic acid having at least one identifiable structure, function, or characteristic.

"RNA" or "ribonucleic acid" consists of ribose nucleotides or ribonucleotides (nitrogenous bases attached to a ribose sugar) linked by phosphodiester bonds, forming sequences of varying lengths. The nitrogenous bases in RNA are adenine, guanine, cytosine, and uracil.

"Ribonucleotide" means a nucleotide having a hydroxy at the 2' position of the sugar portion of the nucleotide. Ribonucleotides can be modified with any of a variety of substituents and may be connected by covalent linkages other than naturally occurring phosphodiester such as phosphorothioate. A ribonucleotide is sometimes referred to as RNA, "R" or "r" herein.

"Segments" are defined as smaller or sub-portions of regions within a nucleic acid molecule, particularly a target nucleic acid.

"Sites" as used herein are defined as unique nucleobase positions within a nucleic acid, particularly a target nucleic acid.

"Specifically hybridizable" refers to an oligomeric compound having a sufficient degree of complementarity between an oligomeric compound (e.g., siRNA) and a target nucleic acid (e.g., mRNA) to induce a desired effect, while exhibiting minimal or no effects on non-target nucleic acids under conditions in which specific binding is desired, i.e., under physiological conditions in the case of *in vivo* assays and therapeutic treatments.

"Stringent hybridization conditions" or "stringent conditions" refer to conditions under which an oligomeric compound will hybridize to its target nucleic acid, with minimal hybridization to other nucleic acid molecules.

"Subject" means a human or non-human animal, particularly a human or non-human animal selected for treatment or therapy.

"Target" generally refers to a protein or nucleic acid molecule, the modulation of which is desired. As used herein, "target" particularly refers to a nucleic acidmolecule (e.g., mRNA), the modulation of which is desired.

"Target gene" refers to a gene encoding a target.

"Targeting" means the process of design and selection of an oligomeric compound that will specifically hybridize to a target nucleic acid and induce a desired effect.

"Target nucleic acid," "target RNA," "target RNA transcript" and "nucleic acid target" all mean a nucleic acid capable of being targeted by oligomeric compounds.

"Target region" means a portion of a target nucleic acid to which one or more oligomeric compounds is targeted.

"Target segment" means the sequence of nucleotides of a target nucleic acid to which an oligomeric compound is targeted. "5' target site" refers to the 5'-most nucleotide of a target segment. "3' target site" refers to the 3'-most nucleotide of a target segment. A target segment can be at least a 13-nucleobase portion (i.e. at least 13 consecutive nucleobases) of a target region to which an oligomeric compound is targeted.

"Therapeutic efficacy" or "therapeutically effective" refers to the effectiveness of a compound or composition such as an oligomeric compound described herein in a therapeutic application. Therapeutic efficacy can be increased by improvements in delivery, stability, specificity, safety and/or potency of the therapeutic compound.

"Unmodified" RNA nucleobases mean the purine bases adenine (A) and guanine (G), and the pyrimidine bases cytosine (C) and uracil (U). "Unmodified" DNA nucleobases mean the purine bases adenine (A) and guanine (G), and the pyrimidine bases thymine (T) and cytosine (C). An unmodified RNA nucleobase is considered modified when a DNA nucleobase is substituted for the RNA nucleobase in an oligomeric compound such as an siRNA compound. Aan unmodified DNA nucleobase is considered modified when an RNA nucleobase is substituted for the DNA nucleobase in a DNA sequence.

"Unmodified nucleoside" means herein a nucleoside composed of a naturally occuring nucleobase and a naturally occuring sugar moiety. An unmodified nucleoside is an RNA nucleoside in an oligomeric compound such as an siRNA compound.

"Unmodified nucleotide" means herein a nucleotide composed of a naturally occuring nucleobase, a naturally occuring sugar moiety, and an internucleoside linkage, wherein the internucleotide linkage may be a naturally occuring linkage (i.e., a phosphate linkage) or an artificial linkage (e.g., a phosphorothioate linkage). An unmodified nucleotide is an RNA nucleotide in an oligomeric compound such as an siRNA compound.

Disclosed herein are improved compounds targeting a nucleic acid encoding angiotensinogen (AGT) with Advanced RNA Targeting (ARNATAR) designs that enhance their gene silencing activity. According to the invention, the compound is a double stranded ribonucleic acid (dsRNA) compound. In an embodiment, the dsRNA compound is an shRNA or an siRNA compound.

According to the invention, the double stranded ribonucleic acid (dsRNA) compound for inhibiting expression of angiotensinogen (AGT) in a cell comprises a sense strand and an antisense strand forming a double stranded portion, as defined by the appended claims.

In one embodiment of the disclosure, a double-stranded ribonucleic acid (dsRNA) compound for inhibiting expression of angiotensinogen (AGT) in a cell comprises a sense strand and an antisense strand forming a double stranded portion, wherein the sense strand comprises any one of the sense nucleotide sequences of the dsRNA compound according to the disclosure in any one of Tables 2, 5, 10 or 13, and wherein the antisense strand comprises any one of the antisense nucleotide sequences of the dsRNA compound according to the disclosure in any one of Tables 2, 5, 10 or 13. In certain embodiments, the dsRNA compound is an siRNA compound, wherein the sense strand comprises any one of the sense nucleotide sequences of the dsRNA compound according to the disclosure in any one of Tables 2, 5, 10 or 13, and wherein the antisense strand comprises any of the antisense nucleotide sequences of the dsRNA compound according to the disclosure in any one of Tables 2, 5, 10 or 13. Accordingly, the dsRNA (e.g., siRNA) compound comprises the antisense sequence of SEQ ID NOs: 9 or 19, and the sense sequence of SEQ ID NOs: 8, 33 or 55. In a preferred embodiment, the sense strand comprises the sequence of SEQ ID NO: 55, and the antisense strand comprises the sequence of SEQ ID NOs: 9 or 19.

Accordingly, all of the nucleotides of the sense strand and all of the nucleotides of the antisense strand are modified nucleotides. Modifications are selected from the group consisting of phosphorothioate internucleotide (PS) linkage, modifications of the furanose ring (e.g., 2' substituted nucleosides including nucleosides with a fluoro (2'-F), O-methyl (2'-OMe), H (deoxyribonucleotide), and combinations thereof.

According to the invention, the dsRNA comprises a strand comprising phosphorothioate internucleotide (PS) linkages. The dsRNA comprises a strand comprising a phosphorothioate internucleotide (PS) linkage adjacent to a deoxyribonucleoside (D). The dsRNA comprises a phosphorothioate internucleotide (PS) linkage adjacent to the deoxyribonucleoside (D) on the 5' side, the 3' side or both sides. The dsRNA comprises a strand comprising a phosphorothioate internucleotide (PS) linkage adjacent to 2 nucleosides at the 5' end of the strand and/or 2 nucleosides at the 3' end of the strand.

In certain embodiments, the dsRNA comprises an siRNA. In a preferred embodiment of the disclosure, the siRNA comprises any one of the ARNATAR designed siRNAs according to the disclosure listed in any one of Tables 2, 5, 10 or 13. In a preferred embodiment, the dsRNA comprises the nucleotide sequence and chemical modifications of ATsi786 (SEQ ID NOs: 9 and 55) or ATsi787 (SEQ ID NOs: 19 and 55). In a preferred embodiment, the siRNA comprises the nucleotide sequence and chemical modifications of ATsi786 (SEQ ID NOs: 9 and 55) or ATsi787 (SEQ ID NOs: 19 and 55).

dsRNA can be trafficked into target cells by a variety of modalities. In certain embodiments, dsRNA compounds enter cells via viral delivery vectors, lipid-based delivery, polymer-based delivery, and/or conjugate-based delivery.

In certain embodiments, the dsRNA compound described herein further comprises a conjugate moiety. In certain embodiments of the disclosure, the siRNA according to the disclosure listed in any one of Tables 2, 5, 10 or 13 comprises a conjugate moiety. The conjugate can be selected from cholesterols, lipids, carbohydrates, phospholipids, biotin, phenazine, folate, phenanthridine, anthraquinone, acridine, fluoresceins, rhodamines, coumarins, and dyes. In a preferred embodiment, the conjugate moiety is an N-Acetylgalactosamine (GalNAc)-comprising moiety. It is to be understood that the GalNAc-comprising moiety will usually contain at least one GalNAc compound and a linker for attachment to the dsRNA. In an embodiment, the conjugate can be attached to the 3' end of a sense strand. In a preferred embodiment, the dsRNA compound is an siRNA conjugated to a GalNAc-comprising moiety.

Certain embodiments disclosed herein provide a compound comprising an siRNA for inhibiting expression of AGT in a cell, wherein the siRNA comprises a sense strand and an antisense strand forming a duplex, wherein the sense strand (ATs786 or ATs787 as shown in Table 13, SEQ ID NO: 55) comprises the formula:

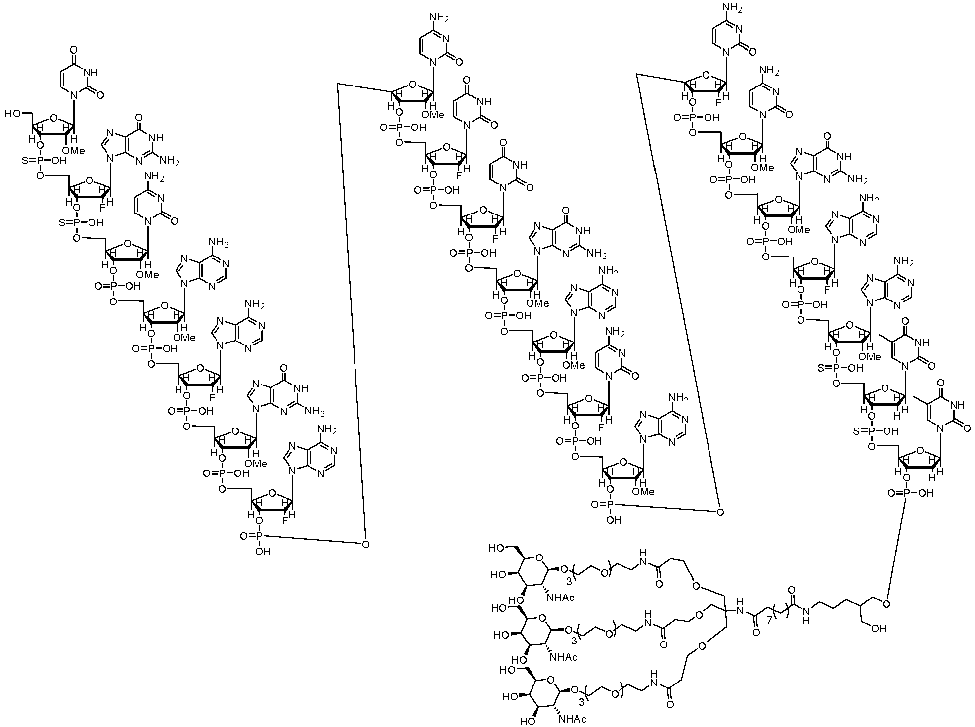

Certain embodiments disclosed herein provide a compound comprising an siRNA for inhibiting expression of AGT in a cell, wherein the siRNA comprises an antisense strand and an antisense strand forming a duplex, wherein the antisense strand (ATa786 as shown in Table 13, SEQ ID NO: 9) comprises the formula:

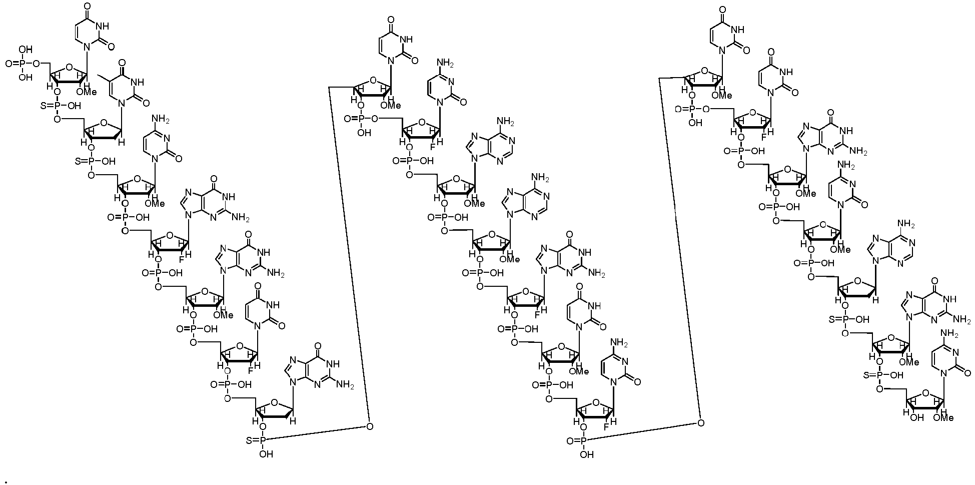

Certain embodiments disclosed herein provide a compound comprising an siRNA for inhibiting expression of AGT in a cell, wherein the siRNA comprises an antisense strand and an antisense strand forming a duplex, wherein the antisense strand (ATa787 as shown in Table 13, SEQ ID NO: 19) comprises the formula:

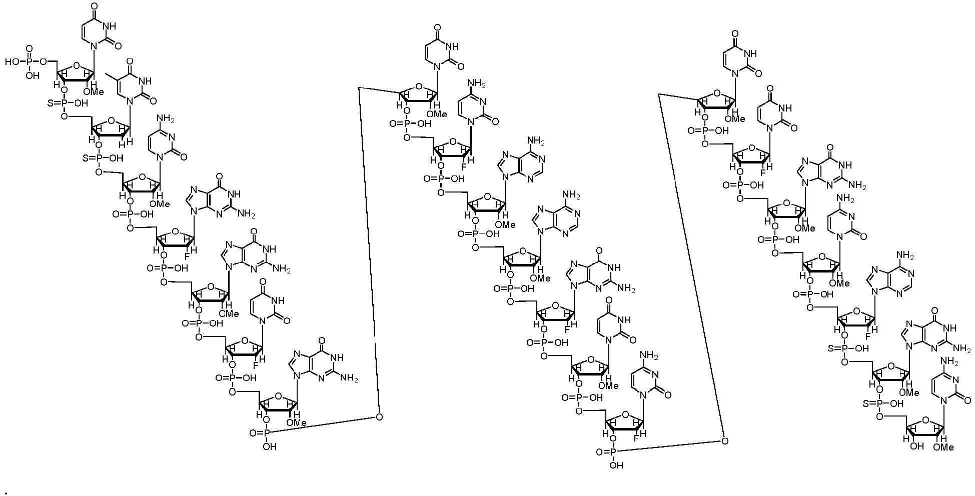

Certain embodiments disclosed herein provide a compound comprising an siRNA for inhibiting expression of AGT in a cell, wherein the siRNA comprises a sense strand and an antisense strand forming a duplex, wherein the sense strand (ATs786 as shown in Table 13, SEQ ID NO: 55) comprises the formula: 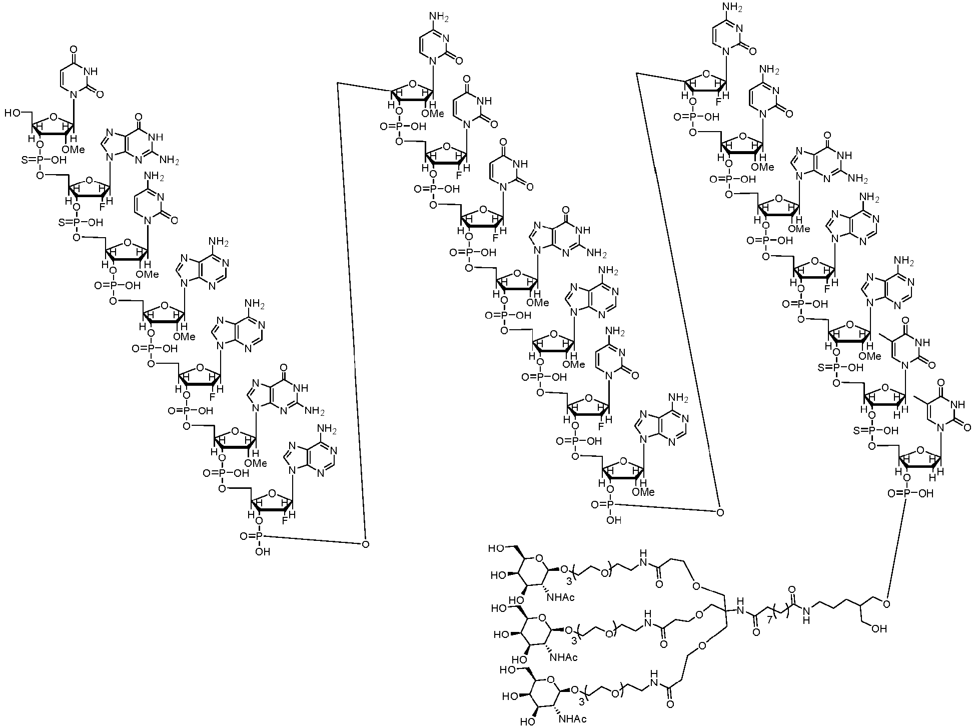 , and the antisense strand (ATa786 as shown in Table 13, SEQ ID NO: 9) comprises the formula:

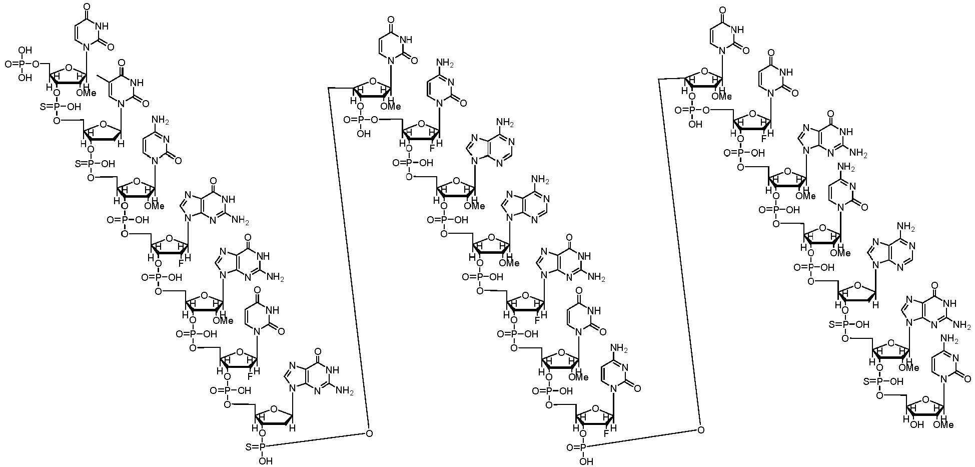

Certain embodiments disclosed herein provide a compound comprising an siRNA for inhibiting expression of AGT in a cell, wherein the siRNA comprises a sense strand and an antisense strand forming a duplex, wherein the sense strand (ATs787 as shown in Table 13, SEQ ID NO: 55) comprises the formula: 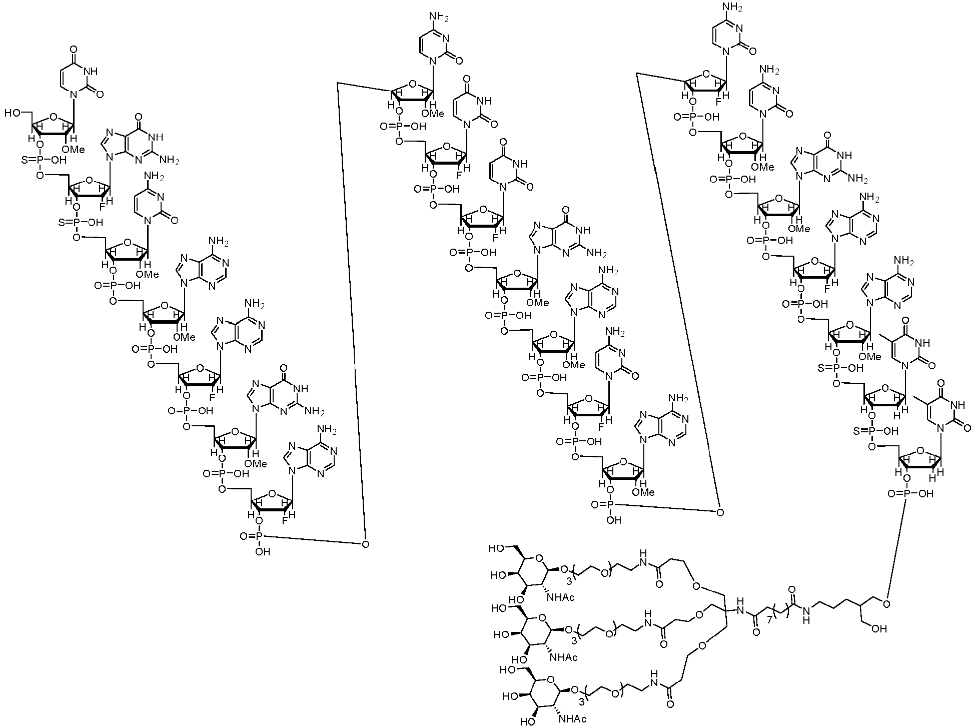 , and the antisense strand (ATa787 as shown in Table 13, SEQ ID NO: 19) comprises the formula:

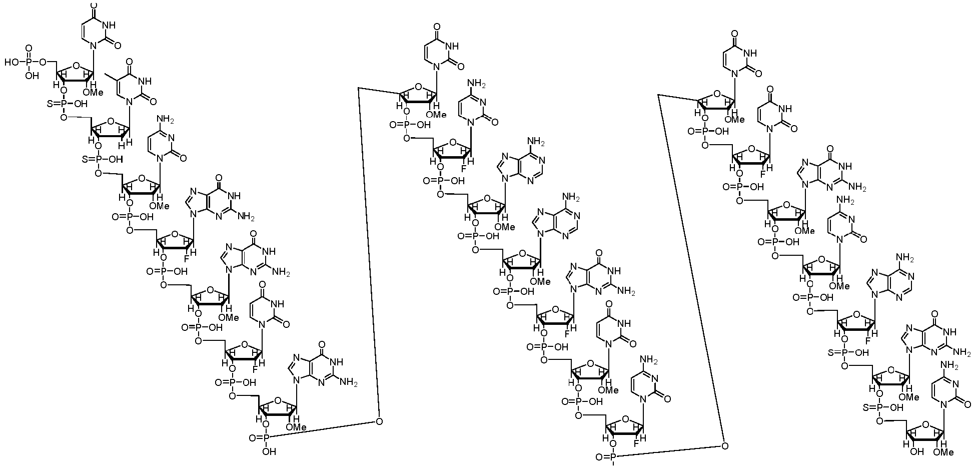

In certain embodiments, the compounds or compositions disclosed herein comprise a salt of the dsRNA. In certain embodiments of the disclosure, the compounds or compositions disclosed herein comprise a salt of the siRNAs according to the disclosure disclosed in Tables 2, 5, 10 and 13.

Described herein is a method of inhibiting expression of angiotensinogen (AGT) in a cell comprising contacting the cell with a dsRNA compound or a pharmaceutical composition described herein in an amount sufficient to inhibit expression of AGT, thereby inhibiting expression of AGT in the cell. The compounds or compositions comprise a dsRNA that inhibits expression of angiotensinogen (AGT) in a cell by at least about 70%, 75%, 80%, 85%, 90%, 95%, 98% or 99%. The dsRNA can be an siRNA that inhibits expression of angiotensinogen (AGT) by at least about 70%, 75%, 80%, 85%, 90%, 95%, 98% or 99%. The compound or composition that inhibits expression of AGT can be provided in Tables 2, 5, 7, 9, 10 or 13. The sense strand of the siRNA can comprise the nucleotide sequence and chemical modification of SEQ ID NOs: 8, 33 or 55). The antisense strand of the siRNA can comprise the nucleotide sequence and chemical modification of SEQ ID NOs: 9 or 19). The siRNA can comprise the nucleotide sequence and chemical modifications of ATsi786 (SEQ ID NOs: 9 and 55) or ATsi787 (SEQ ID NOs: 19 and 55). The amount of angiotensinogen (AGT) expression inhibition can be generally measured by assessing the mRNA and/or protein levels in a cell after contacting the cells with a dsRNA compound or a pharmaceutical composition and comparing the mRNA or protein levels to cells which did not contact the dsRNA.

The siRNA compound that inhibits expression of angiotensinogen (AGT) can inhibit expression of AGT by at least about 85%.

The siRNA compound that inhibits expression of angiotensinogen (AGT) can inhibit expression of AGT by at least about 90%.

Described herein is an assay to determine the level of AGT inhibition in a sample from a subject. The AGT assay can comprise: a) administering a compound or composition disclosed herein to a subject in an amount sufficient to inhibit expression of AGT; b) removing a sample from a subject; c) determining the amount of AGT protein present in the sample; thereby determining the amount of AGT inhibition by the compound or composition. The sample can be from blood, serum, urine and/or liver. The amount of AGT protein present in the sample can be determined by isolating the AGT protein from the sample, Western Blotting the protein and probing with an AGT specific monoclonal antibody to assess the amount of AGT protein present. The amount of AGT protein present in the sample can be determined using ELISA.

In certain embodiments, a pharmaceutical composition for inhibiting expression of angiotensinogen (AGT) in a cell comprises a dsRNA, alone or in combination with a pharmaceutically acceptable carrier, diluent and/or excipient. In certain embodiments, the dsRNA is in a buffer solution. The buffer solution can comprise acetate, citrate, prolamine, carbonate, or phosphate or any combination thereof. In one embodiment, the buffer solution is phosphate buffered saline (PBS). In certain embodiments, the dsRNA in the pharmaceutical composition is an siRNA. In certain embodiments, the siRNA comprises the nucleotide sequence and chemical modifications of ATsi786 (SEQ ID NOs: 9 and 55) or ATsi787 (SEQ ID NOs: 19 and 55).

Described herein is a method for treating a RAAS associated disease, disorder and/or condition in a subject, comprising administering to the subject in need thereof a therapeutically effective amount of a compound or composition disclosed herein in an amount sufficient to inhibit expression of AGT, whereby inhibiting expression of AGT in the subject treats the RAAS associated disease, disorder and/or condition in the subject. The compound or composition that inhibits expression of an AGT is as provided in Tables 2, 5, 7, 9, 10 or 13. The siRNA can comprise the nucleotide sequences of SEQ ID NOs: 61 and 62. The siRNA can comprise the nucleotide sequence and chemical modifications of ATsi786 (SEQ ID NOs: 9 and 55) or ATsi787 (SEQ ID NOs: 19 and 55).

Described herein is a method for treating a RAAS associated disease, disorder and/or condition in a subject, comprising administering to the subject in need thereof a therapeutically effective amount of a compound comprising the ARNATAR designed dsRNA listed in Tables 2, 5, 7, 9, 10 or 13, or a pharmaceutical composition comprising the dsRNA listed in Tables 2, 5, 7, 9, 10 or 13, in an amount sufficient to inhibit expression of AGT whereby inhibiting expression of AGT in the subject treats the RAAS associated disease, disorder and/or condition in the subject. The dsRNA for treating a RAAS associated disease, disorder and/or condition in the subject can be an siRNA listed in Tables 2, 5, 7, 9, 10 or 13. The siRNA can comprise the nucleotide sequence of SEQ ID NOs: 61 and 62. The siRNA can comprise the nucleotide sequence and chemical modifications of ATsi786 (SEQ ID NOs: 9 and 55) or ATsi787 (SEQ ID NOs: 19 and 55).

A symptom of a RAAS associated disease, disorder and/or condition can be treated in a subject comprising administering to the subject in need thereof a therapeutically effective amount of a compound comprising the ARNATAR designed dsRNA listed in Tables 2, 5, 7, 9, 10 or 13, or a pharmaceutical composition comprising the dsRNA listed in Tables 2, 5, 7, 9, 10 or 13 in an amount sufficient to inhibit expression of AGT whereby inhibiting expression of AGT in the subject treats the symptom of the RAAS associated disease, disorder and/or condition. The dsRNA for treating the symptom in the subject is an siRNA listed in Tables 2, 5, 7, 9, 10 or 13. The siRNA can comprise the nucleotide sequence of SEQ ID NOs: 61 and 62. The siRNA can comprise the nucleotide sequence and chemical modifications of ATsi786 (SEQ ID NOs: 9 and 55) or ATsi787 (SEQ ID NOs: 19 and 55).

In certain embodiments, the RAAS associated disease, disorder and/or condition is selected from the group consisting of high blood pressure, hypertension, borderline hypertension, primary hypertension, secondary hypertension isolated systolic or diastolic hypertension, pregnancy-associated hypertension, diabetic hypertension, resistant hypertension, refractory hypertension, paroxysmal hypertension, renovascular hypertension, Goldblatt hypertension, hypertension associated with low plasma renin activity or plasma renin concentration, ocular hypertension, glaucoma, pulmonary hypertension, portal hypertension, systemic venous hypertension, systolic hypertension, labile hypertension; hypertensive heart disease, hypertensive nephropathy, atherosclerosis, arteriosclerosis, vasculopathy, diabetic nephropathy, diabetic retinopathy, chronic heart failure, cardiomyopathy, diabetic cardiac myopathy, glomerulosclerosis, coarctation of the aorta, aortic aneurism, ventricular fibrosis, heart failure, myocardial infarction, angina, stroke, renal disease, renal failure, systemic sclerosis, intrauterine growth restriction (IUGR), fetal growth restriction, obesity, liver steatosis/ fatty liver, non-alcoholic steatohepatitis (NASH), non-alcoholic fatty liver disease (NAFLD), glucose intolerance, type 2 diabetes (non-insulin dependent diabetes), and metabolic syndrome. In a preferred embodiment, the RAAS associated disease, disorder and/or condition is hypertension.

Described herein is a method for treating hypertension in a subject, comprising administering to the subject in need thereof a therapeutically effective amount of a compound comprising the ARNATAR designed dsRNA listed in Tables 2, 5, 7, 9, 10 or 13, or a pharmaceutical composition comprising the dsRNA listed in Tables 2, 5, 7, 9, 10 or 13, in an amount sufficient to inhibit expression of AGT whereby inhibiting expression of AGT in the subject treats the hypertension in the subject. The dsRNA for treating hypertension in the subject can be an siRNA listed in Tables 2, 5, 7, 9, 10 or 13. The siRNA can comprise the nucleotide sequence of SEQ ID NOs: 61 and 62. The siRNA can comprise the nucleotide sequence and chemical modifications of ATsi786 (SEQ ID NOs: 9 and 55) or ATsi787 (SEQ ID NOs: 19 and 55). Types of hypertension include primary, secondary, resistant, malignant and isolated hypertension.

In certain embodiments, the subject in need of therapeutic treatment with a compound or composition disclosed herein is a human subject. In certain embodiments, the subject has: a systolic blood pressure of at least 130 mm Hg and a diastolic blood pressure of at least 80 mm Hg; or, a systolic blood pressure of at least 140 mm Hg and a diastolic blood pressure of at least 80 mm Hg. In certain embodiments, the subject has systolic blood pressure above about 120 mm Hg, 125 mm Hg, 130 mm Hg, 135 mmHg or 140 mmHg. In certain embodiments, the subject has systolic blood pressure between 130-160 mm Hg, 130-165 mm Hg, 130-170 mm Hg, 130-180 mm Hg, 135-160 mm Hg, 135-165 mm Hg, 135-170 mm Hg, 140-170 mm Hg, 145-180 mm Hg, 155-180 mm Hg. In certain embodiments, the subject is part of a group susceptible to salt sensitivity, is overweight, is obese, and/or is pregnant.

Described herein is a method for treating a subject having a disease, disorder and/or condition that would benefit from reduction in angiotensinogen (AGT) expression, comprising administering to the subject in need thereof a therapeutically effective amount of a compound comprising the dsRNA listed in Tables 2, 5, 7, 9, 10 or 13, or a pharmaceutical composition comprising any of the dsRNA listed in Tables 2, 5, 7, 9, 10 or 13, in an amount sufficient to inhibit expression of AGT whereby inhibiting expression of AGT in the subject treats the subject having the disorder that would benefit from reduction in AGT expression. The dsRNA can be an siRNA listed in Tables 2, 5, 7, 9, 10 or 13. The siRNA can comprise the nucleotide sequence of SEQ ID NOs: 61 and 62. The siRNA can comprise the nucleotide sequence and chemical modifications of ATsi786 (SEQ ID NOs: 9 and 55) or ATsi787 (SEQ ID NOs: 19 and 55). The disease, disorder and/or condition can be a RAAS associated disease, disorder and/or condition.

Described herein is a method of preventing or reducing at least one symptom in a subject having a disorder that would benefit from reduction in angiotensinogen (AGT) expression, comprising administering to the subject in need thereof a prophylactically effective amount of a compound comprising the dsRNA listed in Tables 2, 5, 7, 9, 10 or 13, or a pharmaceutical composition comprising any of the dsRNA listed in Tables 2, 5, 7, 9, 10 or 13, in an amount sufficient to inhibit expression of AGT whereby inhibiting expression of AGT in the subject prevents or reduces at least one symptom in the subject having the disorder that would benefit from reduction in AGT expression. The dsRNA can be an siRNA listed in Tables 2, 5, 7, 9, 10 or 13. The siRNA can comprise the nucleotide sequence of SEQ ID NOs: 61 and 62. The siRNA can comprise the nucleotide sequence and chemical modifications of ATsi786 (SEQ ID NOs: 9 and 55) or ATsi787 (SEQ ID NOs: 19 and 55). The at least one symptom can be a RAAS associated symptom.

Described herein is a method for inhibiting AGT expression in a subject comprising the step of administering the compound or composition comprising the dsRNA described herein to the subject, in an amount sufficient to inhibit expression of AGT. The dsRNA compound is administered subcutaneously or intravenously to the subject. The dsRNA can inhibit expression of angiotensinogen (AGT) by at least about 70%, 75%, 80%, 85%, 90%, 95%, 98% or 99%.

Certain embodiments provide a double stranded ribonucleic acid (dsRNA) compound according to the invention and described herein for use in medicine. Certain embodiments provide the double stranded ribonucleic acid (dsRNA) compound according to the invention and described herein for use in treating or preventing a RAAS associated disease, disorder and/or condition in a subject. In certain embodiments, the RAAS associated disease, disorder and/or condition is as described above.

In certain embodiments, the dsRNA is administered to the subject at a dose of about 0.01 mg/kg to about 50 mg/kg. In certain embodiments, the dsRNA is an siRNA that inhibits expression of angiotensinogen (AGT) by at least about 70%, 75%, 80%, 85%, 90%, 95%, 98% or 99%. In certain embodiments, the dsRNA is an siRNA that is administered to the subject at a dose of about 0.01 mg/kg to about 50 mg/kg. In certain embodiments, a preferred dose is selected from 700mg, 800mg and 900mg. In certain embodiments, a therapeutically effective amount of the compound or composition comprising the dsRNA described herein is dosed at about 150mg, 300mg or 600mg once every 3 months. In certain embodiments, a therapeutically effective amount of the compound or composition comprising the dsRNA described herein is dosed at about 150mg, 300mg or 600mg once every 6 months.

In certain embodiments, after administration of the compound or composition comprising dsRNA to a subject, the level of AGT in a sample(s) from the subject is determined. In certain embodiments, the level of AGT in the subject sample(s) is an AGT nucleic acid (e.g., mRNA) level or protein level in a blood, plasma, urine or liver tissue sample(s). In certain embodiments, the dsRNA is an siRNA.

In certain embodiments, after administration of the compound or composition comprising dsRNA to a subject, additional factors are assessed. In certain embodiments, the level of bradykinin, prekallikrein, or blood pressure in the subject is determined. In certain embodiments, the dsRNA is an siRNA.

In certain embodiments, the compound or composition comprising dsRNA is administered alone or in combination with additional therapeutic agent(s) to a subject for treatment of a RAAS associated disease, disorder and/or condition, or symptom thereof. In certain embodiments, the siRNA comprises the nucleotide sequence and chemical modifications of ATsi786 (SEQ ID NOs: 9 and 55) or ATsi787 (SEQ ID NOs: 19 and 55). In certain embodiments, the additional therapeutic agent is selected from the group consisting of a diuretic, an angiotensin converting enzyme (ACE) inhibitor, an angiotensin II receptor antagonist (also known as an angiotensin II receptor blocker (ARB)), a beta-blocker, a vasodilator, a calcium channel blocker, an aldosterone antagonist, an alpha2-agonist, a renin inhibitor, an alpha-blocker, a peripheral acting adrenergic agent, a selective DI receptor partial agonist, a nonselective alpha-adrenergic antagonist, a synthetic, a steroidal antimineralocorticoid agent, an angiotensin receptor-neprilysin inhibitors (ARNi), Entresto^{®}, sacubitril/valsartan; or an endothelin receptor antagonist (ERA), sitaxentan, ambrisentan, atrasentan, BQ-123, zibotentan, bosentan, macitentan, and tezosentan; a combination of any of the foregoing; and a hypertension therapeutic agent formulated as a combination of agents. In certain embodiments, the additional therapeutic agent comprises an angiotensin II receptor antagonist. In certain embodiments, the angiotensin II receptor antagonist is selected from the group consisting of losartan, valsartan, olmesartan, eprosartan, and azilsartan. In certain embodiments, the additional therapeutic agent(s), when used in combination with the compounds or compositions comprising a dsRNA described herein, may provide a synergistic or additive effect in lowering blood pressure in a subject.

Described herein is a process for preparing the sense and/or antisense strand of the double stranded ribonucleic acid (dsRNA) compound, wherein the process comprises the steps of: (a) preparing the sense and/or antisense strand by sequential coupling of modified and/or unmodified nucleotides via the phosphoramidite oligonucleotide synthesis on a solid support; (b) optionally, coupling a GalNAc-comprising moiety to the sense and/or antisense strand on the solid support via the phosphoramidite oligonucleotide synthesis; (c) detaching the sense and/or antisense strand from the solid support and removing the solid support; and (d) optionally, further purifying the sense and/or antisense strand, optionally using chromatography. Phosphoramidite oligonucleotide synthesis is generally known in the art. By this process a sense or antisense strand may be prepared that may contain a GalNAc-comprising moiety as conjugate at its 5' end. Preferably, this process is used to prepare the antisense strand that does not contain any conjugate (e.g., the antisense strand of SEQ ID NO: 9 or 19).

Described herein is a process for preparing the sense and/or anisense strand of the double stranded ribonucleic acid (dsRNA) compound, wherein the process comprises the steps of: (a) coupling a GalNAc-comprising moiety to a solid support via the phosphoramidite oligonucleotide synthesis, (b) coupling a modified and/or unmodified nucleotide via the phosphoramidite oligonucleotide synthesis to the GalNAc-comprising moiety on the solid support; (c) sequentially coupling additional modified and/or unmodified nucleotides via the phosphoramidite oligonucleotide synthesis to prepare the sense and/or anisense strand; (d) detaching the sense and/or antisense strand from the solid support and removing the solid support; and (e) optionally, further purifying the sense and/or antisense strand, optionally using chromatography. By this process a sense or antisense strand is prepared that contains a GalNAc-comprising moiety as conjugate at its 3' end. Preferably, this process is used to prepare the sense strand containing a 3' end GalNAc conjugate (e.g., the sense strand of SEQ ID NO: 55).

Described herein is a process of preparing the double stranded ribonucleic acid (dsRNA) compound, comprising: (a) contacting the sense prepared according any one of the above processes with the antisense strand prepared according any one of the above processes in equimolar concentrations in a solution; (b) optionally heating the solution to a temperature of about 94°C; and (c) optionally reducing the temperature of the solution to about 25°C. Preferably, the sense strand is a sense strand containing a 3' end GalNAc conjugate and the antisense strand is an antisense strand that does not contain any conjugate. Described herein is a process for preparing an oligonucleotide (of the sense or antisense strand) for use in forming a compound or composition described herein comprising synthesizing an oligonucleotide on solid support by: (a) attaching a modified or unmodified nucleotide to the solid support by phosphoramidite chemistry; (b) removing excess phosphoramidite reaction solution; (c) removing an acid labile protecting group from the nucleotide with an acid solution; (d) removing the acid solution; (e) coupling a modified or unmodified nucleotide, GalNAc-comprising moiety, or lipophilic moiety by phosphoramidite chemistry to the unprotected nucleotide attached to the solid support to form an elongating oligonucleotide attached to the solid support; (f) removing excess phosphoramidite reaction solution; (g) capping unreacted amines or alcohols on the oligonucleotide attached to the solid support with an acetic anhydride solution to reduce branching and truncation; (h) removing the acetic anhydride solution; (i) introducing a phosphodiester linkage by an oxidizing reagent and/or a phosphorothioate linkage by a sulfurizing reagent; (j) removing the oxidizing and/or sulfurizing reagent; (k) repeating steps (c)-(j) until the desired length of the oligonucleotide is achieved; (l) removing the oligonucleotide from the solid support with an alkaline solution; (m) removing the solid support from the oligonucleotide solution; and (n) optionally, purifying the oligonucleotide using chromatography.

Described herein is a process for preparing an oligonucleotide (of the sense or antisense strand) for use in forming a compound or composition described herein comprising synthesizing an oligonucleotide on a moiety loaded solid support by: (a) attaching a modified or unmodified nucleotide to the moiety on the solid support by phosphoramidite chemistry; (b) removing excess phosphoramidite reaction solution; (c) removing an acid labile protecting group from the nucleotide with an acid solution; (d) removing the acid solution; (e) coupling a modified or unmodified nucleotide by phosphoramidite chemistry to the unprotected nucleotide attached to the solid support to form an elongating oligonucleotide attached to the solid support; (f) removing excess phosphoramidite reaction solution; (g) capping unreacted amines or alcohols on the oligonucleotide attached to the solid support with an acetic anhydride solution to reduce branching and truncation; (h) removing the acetic anhydride solution; (i) introducing a phosphodiester linkage by an oxidizing reagent and/or a phosphorothioate linkage by a sulfurizing reagent; (j) removing the oxidizing and/or sulfurizing reagent; (k) repeating steps (c)-(j) until the desired length of the oligonucleotide is achieved; (l) removing the oligonucleotide from the solid support with an alkaline solution; (m) removing the solid support from the oligonucleotide solution; and (n) optionally, purifying the oligonucleotide using chromatography. The moiety loaded on the solid support can be selected from a GalNAc-comprising moiety, cholesterol, lipid, carbohydrate, phospholipid, biotin, phenazine, folate, phenanthridine, anthraquinone, acridine, fluorescein, rhodamine, coumarin, dye, and the like. The moiety loaded on the solid support can be a GalNAc-comprising moiety.

The present invention also provides kits comprising any of the compounds or any of the pharmaceutical compositions disclosed herein, and optionally, a label (e.g., instructions for use). The present invention provides a vial comprising any of the compounds or any of the pharmaceutical compositions disclosed herein. The present invention provides a syringe comprising any of the compounds or any of the pharmaceutical compositions disclosed herein. In one embodiment, the invention provides a kit for performing a method of inhibiting expression of AGT in a subject by administering to the subject in need thereof an amount effective to inhibit expression of the AGT in the subject. The kit comprises a dsRNA compound and instructions for use and, optionally, means for administering the dsRNA to a subject. In certain embodiments of the disclosure, the compound or pharmaceutical composition is a dsRNA according to the disclosure listed in Tables 2, 5, 10 or 13. In certain embodiments, the dsRNA is an siRNA according to the disclosure listed in Tables 2, 5, 10 or 13. In a preferred embodiment, the siRNA of the kit comprises the nucleotide sequence and chemical modifications of ATsi786 (SEQ ID NOs: 9 and 55) or ATsi787 (SEQ ID NOs: 19 and 55).

The following description applies to all of the above embodiments.

### Oligomeric Compounds

Oligomeric compounds include, but are not limited to, double stranded RNA (dsRNA) compounds such as short hairpin RNAs (shRNAs) and small interfering RNAs (siRNAs) target the AGT gene by targeting the AGT mRNA. An oligomeric compound comprises an "antisense strand" to a target nucleic acid, meaning that is is capable of undergoing hybridization to a target nucleic acid through hydrogen bonding.

An oligomeric compound can have a nucleobase sequence that, when written in the 5' to 3' direction, comprises the reverse complement of the target segment of a target nucleic acid to which it is targeted. For example, an siRNA compound can comprise an antisense strand which has a nucleobase sequence that, when written in the 5' to 3' direction, comprises the reverse complement of the target segment of a target nucleic acid to which it is targeted.

An oligomeric compound can have 12-30 subunits in length. The oligomeric compound can be an siRNA.

It is possible to increase or decrease the length of an oligomeric compound, such as an siRNA compound, and/or introduce base mismatch(s) without eliminating activity (US Patent 7,772,203). For example, it is possible to introduce non-canonical base pairings (e.g., A:G, A:C, G:U, I:U, I:A, I:C) into an oligomeric compound without eliminating activity. Designing oligomeric compounds with one or more non-canonical base pairings, i.e., mismatch(s), can enhance the activity of the oligomeric compound.

The oligomeric compound can comprise a mismatch(es) with the target, between the oligomeric strands within the duplex, or combinations thereof. The mismatch may occur throughout the siRNA such as in the overhang (a portion of the sense or antisense strand at the 5' and/or 3' end of a duplexed siRNA that has no complementary strand) or the duplex portion.

### Oligomeric Compound Motifs

A motif refers to a pattern of modification of an oligomeric compound. Various motifs have been described in the art (e.g., US Patent 11,203,755; US Patent 10,870,849; EP Patent 1,532,248; US Patent 11,406,716; US Patent 10,668,170; US Patent 9,796,974; US Patent 8,754,201; US Patent 10,837,013; US Patent 7,732,593; US Patent 7,015,315; US Patent 7,750,144; US Patent 8,420,799; US Patent 8,809,516; US Patent 8,796,436; US Patent 8,859,749; US Patent 9,708,615; US Patent 10,233,448; US Patent 10,273,477; US Patent 10,612,024; US Patent 10,612,027; US Patent 10,669,544; US Patent 11,401,517; USSN 2020/0031862; USSN 2016/0272970). However, new and improved motifs are constantly being sought.

Oligomeric compounds disclosed herein, such as siRNAs, can have chemically modified subunits arranged into motifs to confer on to the oligomeric compounds beneficial properties including, but not limited to: enhanced inhibitory activity to increase potency; increased binding affinity to increase specificity for a target nucleic acid, thereby limiting off-target effects and increasing safety; or enhanced resistance to degradation by *in vivo* nucleases thereby increasing stability and durability. The oligomeric compounds can be chimeras where the peripheral nucleobases of the oligomeric compounds comprise motifs with various modified or unmodified nucleobases so as to confer increased stability, specificity, safety and potency, while the central portion of the compound comprises various modified or unmodified nucleobases to serve as substrates for RISC mediated degradation. Each distinct portion can comprise uniform sugar moieties, modified, or alternating sugar moieties. Each portion can comprise a varied pattern of phosphate and phosphorothioate linkages.

The oligomeric compounds targeted to an AGT nucleic acid can comprise a sense strand with sequence and chemical modification motif as shown in Tables 2, 5, 7, 9, 10 or 13. The oligomeric compounds targeted to an AGT nucleic acid can comprise an antisense strand with sequence and chemical modification motif as shown in Tables 2, 5, 7, 9, 10 or 13.

### Target Nucleic Acids, Target Regions and Nucleotide Sequences

Described herein are methods of modulating gene expression by dsRNA inhibition.

A method of inhibiting angiotensinogen (AGT) gene expression in a cell can comprise administering to the cell a dsRNA compound targeted to an mRNA (or its corresponding cDNA) transcript of AGT (GenBank NM_001382817.3, incorporated herein as SEQ ID NO:1).

The nucleic acid sequence and chemical modification motifs of dsRNA targeting the AGT transcript are shown in Tables 2, 5, 7, 9, 10 and 13. It is understood that the sequence set forth in each SEQ ID NO in the examples contained herein is independent of any modification to a sugar moiety, an internucleoside linkage, or a nucleobase. As such, siRNA compounds defined by a SEQ ID NO may comprise, independently, one or more modifications to a sugar moiety, an internucleoside linkage, or a nucleobase. siRNA compounds denoted by ARNATAR designations indicate a combination of sequence and motif.

### Hybridization

Hybridization can occur between an oligomeric compound disclosed herein and an mRNA. The most common mechanism of hybridization involves hydrogen bonding (e.g., Watson-Crick, Hoogsteen or reversed Hoogsteen hydrogen bonding) between complementary nucleobases of the nucleic acid molecules.

In Watson-Crick canonical base pairings, adenine (A) is complementary to thymine (T) in DNA, adenine (A) is complementary to uracil (U) in RNA, and guanine (G) is complementary to cytosine (C) in both DNA and RNA. Base pairs, or complementary nucleobases, are usually Watson-Crick base pairs (C:G, A:U, A:T), but, non-canonical base pairs such as Hoogsteen base pairs (e.g., A:G, A:U), Wobble base pairs (e.g., G:U, I:U, I:A, I:C, wherein I is hypoxanthine) and the like are also permitted during hybridization of the oligomeric compound to a target nucleic acid or target region. Wobble base pairs in RNAi agents have previously been described (see e.g., US Patent 7,732,593; US Patent 7,750,144).

Nucleobase complementarity facilitates hybridization of the oligomeric compounds described herein to their target nucleic acids with the stronger the pairing (e.g., the more base pairs and/or the stronger the hydrogen bond), the stronger the hybridization of the oligomeric compound to the target. Hybridization can occur under varying conditions. Stringent conditions are sequence-dependent and are determined by the nature and composition of the oligomeric compound to be hybridized.

Methods of determining whether a sequence is specifically hybridizable to a target nucleic acid are well known in the art. The oligomeric compounds described herein can be specifically hybridizable with a target mRNA with little to no off-target binding.

### Complementarity

An oligomeric compound and a target nucleic acid are complementary to each other when a sufficient number of nucleobases of the oligomeric compound can hybridize with the corresponding nucleobases of the target nucleic acid, such that a desired effect will occur (e.g., inhibition of a target nucleic acid, such as an mRNA nucleic acid).

Non-complementary nucleobases between an oligomeric compound and an mRNA nucleic acid may be tolerated provided that the oligomeric compound remains able to specifically hybridize to a target nucleic acid. Moreover, an oligomeric compound may hybridize over one or more segments of an mRNA nucleic acid such that intervening or adjacent segments is not involved in the hybridization event (e.g., a loop structure, mismatch or hairpin structure).

The oligomeric compounds described herein, or a specified portion thereof, are, or are at least, 70%, 80%, 85%, 86%, 87%, 88%, 89%, 90%, 91%, 92%, 93%, 94%, 95%, 96%, 97%, 98%, 99%, or 100% complementary to an mRNA nucleic acid, a target region, target segment, or specified portion thereof. Percent complementarity of an oligomeric compound with a target nucleic acid can be determined using routine methods.

For example, an oligomeric compound in which 18 of 20 nucleobases of the antisense strand of the oligomeric compound are complementary to a target region, and would therefore specifically hybridize, would represent 90 percent complementarity. In this example, the remaining non-complementary nucleobases may be clustered or interspersed with complementary nucleobases and need not be contiguous to each other or to complementary nucleobases. Percent complementarity of an oligomeric compound with a region of a target nucleic acid can be determined routinely using BLAST programs (basic local alignment search tools) and PowerBLAST programs known in the art (Altschul et al., 1990, J. Mol. Biol., 215:403-410; Zhang and Madden, 1997, Genome Res., 7:649-656) and available through the website for the National Center for Biotechnology Information (NCBI, https://blast.ncbi.nlm.nih.gov/Blast.cgi). Percent homology, sequence identity or complementarity, can be determined by, for example, NCBI Blast (Johnson et al., Nucleic Acids Res. 2008, 36 (Web Server issue): W5-W9).

The oligomeric compounds described herein, or specified portions thereof, are fully complementary (i.e. 100% complementary) to a target nucleic acid, or specified portion thereof. For example, an oligomeric compound may be fully complementary to an mRNA nucleic acid, or a target region, or a target segment or target sequence thereof. As used herein, "fully complementary" means each nucleobase of an oligomeric compound is capable of precise base pairing with the corresponding nucleobases of a target nucleic acid. For example, a 20-nucleobase oligomeric compound is fully complementary to a target sequence that is 400 nucleobases long, so long as there is a corresponding 20 nucleobase portion of the target nucleic acid that is fully complementary to the oligomeric compound.

Fully complementary can also be used in reference to a specified portion of the oligomeric compound or the nucleic acid target. For example, a 20-nucleobase portion of a 30-nucleobase oligomeric compound can be "fully complementary" to a target sequence that is 400 nucleobases long. The 20-nucleobase portion of the 30-nucleobase oligomer is fully complementary to the target sequence, if the target sequence has a corresponding 20 nucleobase portion wherein each nucleobase is complementary to the 20-nucleobase portion of the oligomeric compound. At the same time, the entire 30 nucleobase oligomeric compound may or may not be fully complementary to the target sequence, depending on whether the remaining 10 nucleobases of the oligomeric compound are also complementary to the target sequence.

The location of a non-complementary nucleobase may be at the 5' end or 3' end of the oligomeric compound. Alternatively, the non-complementary nucleobase or nucleobases may be at an internal position of the oligomeric compound. When two or more non-complementary nucleobases are present, they may be contiguous (i.e., linked) or non-contiguous.

Oligomeric compounds that are, or are up to 12, 13, 14, 15, 16, 17, 18, 19, 20 or 21 nucleobases in length can comprise no more than 6, no more than 5, no more than 4, no more than 3, no more than 2, or no more than 1 non-complementary nucleobase(s) relative to a target nucleic acid, such as an mRNA nucleic acid, or specified portion thereof.

Oligomeric compounds that are, or are up to 12, 13, 14, 15, 16, 17, 18, 19, 20, 21, 22, 23, 24, 25, 26, 27, 28, 29, or 30 nucleobases in length can comprise no more than 8, no more than 7, no more than 6, no more than 5, no more than 4, no more than 3, no more than 2, or no more than 1 non-complementary nucleobase(s) relative to a target nucleic acid, such as an mRNA nucleic acid, or specified portion thereof.

The oligomeric compounds described herein also include those which are complementary to a portion of a target nucleic acid. As used herein, "portion" refers to a defined number of contiguous (i.e. linked) nucleobases within a region or segment of a target nucleic acid. A "portion" can also refer to a defined number of contiguous nucleobases of an oligomeric compound. The oligomeric compounds, can be complementary to at least an 8-nucleobase portion of a target segment. The oligomeric compounds can be complementary to at least a 9-nucleobase portion of a target segment. The oligomeric compounds can be complementary to at least a 10-nucleobase portion of a target segment. Tthe oligomeric compounds can be complementary to at least an 11-nucleobase portion of a target segment. The oligomeric compounds can be complementary to at least a 12-nucleobase portion of a target segment. Tthe oligomeric compounds can be complementary to at least a 13-nucleobase portion of a target segment. The oligomeric compounds can be complementary to at least a 14-nucleobase portion of a target segment. The oligomeric compounds can be complementary to at least a 15-nucleobase portion of a target segment. Also contemplated are oligomeric compounds that are complementary to at least a 9, 10, 11, 12, 13, 14, 15, 16, 17, 18, 19, 20, or more nucleobase portion of a target segment, or a range defined by any two of these values.

### Identity

The oligomeric compounds described herein may also have a defined percent identity to a particular nucleotide sequence, SEQ ID NO, or compound represented by a specific ARNATAR number, or portion thereof. As used herein, an oligomeric compound is identical to the sequence disclosed herein if it has the same nucleobase pairing ability. For example, an RNA which contains uracil in place of thymidine in a disclosed DNA sequence would be considered identical to the DNA sequence since both uracil and thymidine pair with adenine. Shortened and lengthened versions of the oligomeric compounds described herein as well as compounds having non-identical bases relative to the oligomeric compounds described herein also exist. The non-identical bases may be adjacent to each other or dispersed throughout the oligomeric compound. Percent identity of an oligomeric compound is calculated according to the number of bases that have identical base pairing relative to the sequence to which it is being compared.

A portion of the oligomeric compound is compared to an equal length portion of the target nucleic acid. An 8, 9, 10, 11, 12, 13, 14, 15, 16, 17, 18, 19, 20, 21, 22, 23, 24, or 25 nucleobase portion of the oligomeric compound can be compared to an equal length portion of the target nucleic acid.

### Chemical Modifications

A nucleoside is a base-sugar combination. The nucleobase (also known as base) portion of the nucleoside is normally a heterocyclic base moiety. Nucleotides are nucleosides that further include a covalent linkage (e.g., phosphate group or a chemically modified linkage as described *infra*) to the sugar portion of the nucleoside. Oligonucleotides are formed through the covalent linkage of adjacent nucleotides to one another, to form a linear sequence of linked nucleotides. Within the oligonucleotide structure, the linkage groups are commonly referred to as forming the internucleoside linkages of the oligonucleotide. Oligomeric compounds are made up of one (e.g., ssRNAs, antisense oligonucleotides or miRNAs) or more oligonucleotides (e.g., dsRNAs such as siRNAs or shRNAs)

Modifications to oligomeric compounds encompass substitutions or changes to nucleobases, internucleoside linkages or sugar moieties. Modified oligomeric compounds as disclosed herein are often preferred over native or unmodified forms because of desirable properties such as, for example, enhanced delivery (e.g., increased cellular uptake), enhanced specificity or affinity for a nucleic acid target, increased stability in the presence of nucleases, enhanced safety (e.g., fewer side effects after administration of the compound to a subject) or increased potency (e.g., inhibitory activity).

### Nucleobase Modifications

A nucleobase is a heterocyclic moiety capable of base pairing with a nucleobase of another nucleic acid. Modifications to nucleobases can be advantageous to an oligomeric compound for various reasons including, but not limited to, increase stability of the oligomeric compound, increase specificity, decreased immunogenicity of the oligomeric compound, increase affinity of the oligomeric compound, increase potency of the oligomeric compound and other desirable features.

Examples of nucleobase modifications and their advantages are well known in the art (Friedrich and Aigner, Therapeutic siRNA: State-of-the-Art and Future Perspectives, 2022, BioDrugs, 36(5):549-571; Hu et al., Therapeutic siRNA: State of the Art, Signal Transduction and Targeted Therapy, 2020, 5:101). Nucleobase modifications can comprise substituting nucleobases with nucleobase analogs or modification of a part of the nucleobase. Examples of nucleobase modifications include, but are not limited to, pseudouridine, 2'-thiouridine, N6'-methyladenosine, and 5'-methylcytidine, 5'-fluoro-2'-deoxyuridine, N-ethylpiperidine 5' triazole-modified adenosine, 5'-nitroindole, 2',4'-difluorotolylribonucleoside, N-ethylpiperidine 7'-EAA triazole-modified adenosine, 6'-phenylpyrrolocytosine and the like.

Oligomeric compounds targeted to an mRNA nucleic acid can comprise one or more modified nucleobases. The modified nucleobases can be, for example, deoxyribonucleosides (D) substituted for ribonucleosides (R). A modified nucleobase can be a thymine substitution for a uracil. Multiple nucleobases of an oligomeric compound can be modified. IEach nucleobase of an oligomeric compound can be modified.

### Internucleoside Linkage Modifications

The naturally occuring internucleoside linkage of RNA and DNA is a 3' to 5' phosphodiester linkage. For nucleosides that include a furanose sugar, the phosphate group can be linked to the 2', 3' or 5' hydroxyl moiety of the sugar. Oligomeric compounds having one or more modified, i.e., non-naturally occurring, internucleoside linkages are often selected over oligomeric compounds having naturally occurring internucleoside linkages because of desirable properties such as, for example, enhanced cellular uptake, enhanced affinity for target nucleic acids, decrease toxicity, increased stability and durability, decreased degradation and other desirable features for an oligomeric compound. Modified internucleoside linkages and their advantages are well known in the art (Friedrich and Aigner, Therapeutic siRNA: State-of-the-Art and Future Perspectives, 2022, BioDrugs, 36(5):549-571; Hu et al., Therapeutic siRNA: State of the Art, Signal Transduction and Targeted Therapy, 2020, 5:101).

Oligomeric compounds having modified internucleoside linkages include internucleoside linkages that retain a phosphorus atom as well as internucleoside linkages that do not have a phosphorus atom. Representative phosphorus containing internucleoside linkages include, but are not limited to, phosphodiesters, phosphotriesters, methylphosphonates (e.g., 5'-methylphosphonate (5'-MP)), phosphoramidate, and phosphorothioates (e.g., phosphorodithioate Rp isomer (PS,Rp), phosphorodithioate Rp isomer (PS,Sp), 5'-phosphorothioate (5'-PS)), methoxypropylphosphonate, (*S*)-5'-C-methyl with Phosphate, peptide nucleic acid (PNA), 5'-(E)-vinylphosphonate.

Oligomeric compounds targeted to an mRNA nucleic acid can comprise one or more modified internucleoside linkages. The modified internucleoside linkages can be phosphorothioate (PS) linkages. One or more internucleoside linkage of an oligomeric compound can be a phosphorothioate internucleoside linkage. The PS linkage is adjacent to a deoxyribonucleoside (sometimes referred to as DNA or "D" herein) or a ribonucleoside (sometimes referred to as RNA, "R" or "r" herein). Each internucleoside linkage of an oligomeric compound can be a phosphorothioate internucleoside linkage.

### Sugar Modifications

Natural sugars are sugar moieties found in DNA (2'-H) or RNA (2'-OH), i.e., 2-deoxy-beta-D-ribofuranose or beta-D-ribofuranose, respectively. Oligomeric compounds described herein can contain one or more nucleosides wherein the natural sugar moiety has been modified. Such sugar modified nucleosides may impart desirabe features such as increased stability, increased durability (e.g., increased half-life), increased binding affinity, decreased off-target effects, decreased immunogenicity, decreased toxicity, increased potency, or some other beneficial biological property to the oligomeric compounds. Sugar modifications and their advantages are known in the art (Friedrich and Aigner, 2022, BioDrugs, 36(5):549-571; Hu et al., Therapeutic siRNA: State of the Art, Signal Transduction and Targeted Therapy, 2020, 5:101; Chiu and Rana, 2003, RNA, 9:1034-1048; Choung et al., Biochem Biophys Res Commun, 2006, 342:919-927; Amarzguioui et al., 2003, Nucleic Acids Res, 31(2):589-595; Braasch et al., 2003, Biochemistry, 42(26):7967-7975; Czauderna et al., 2003, Nucleic Acids Res, 31(11):2705-2716; Allerson et al., 2005, J Med Chem, 48:901-904; Layzer et al., 2004, RNA, 10:766-771; Ui-Tei, et al., 2008, Nucleic Acids Res, 36(7):2136-51; Bramsen and Kjems, 2012, Frontiers in Genetics, 3(154):1-22; Bramsen et al., 2010, Nucleic Acids Res, 38(17):5761-5773; Muhonen et al., 2007, Chem & Biodiversity, 4:858-873).

Nucleosides can comprise a chemically modified ribofuranose ring moiety. Examples of chemically modified ribofuranose rings can include, without limitation, addition of substitutent groups (e.g., 5' sugar modifications, 2' sugar modifications); bridging of non-geminal ring atoms to form bicyclic nucleic acids (BNA); replacement of the ribosyl ring oxygen atom with S, N(R), or C(R) (R = H, C₁-C₁₂ alkyl or a protecting group); nucleoside mimetic; and combinations thereof.

A 2'-modified sugar refers to a furanosyl sugar modified at the 2' position. A 2'-modified nucleoside refers to a nucleoside comprising a sugar modified at the 2' position of a furanose ring. Such modifications can include substituents selected from: a halide, including, but not limited to substituted and unsubstituted alkoxy, substituted and unsubstituted thioalkyl, substituted and unsubstituted amino alkyl, substituted and unsubstituted alkyl, substituted and unsubstituted allyl, and substituted and unsubstituted alkynyl. 2' modifications can be selected from substituents including, but not limited to: O[(CH₂)ₙO]ₘCH₃, O(CH₂)ₙNH₂, O(CH₂)ₙCH₃, O(CH₂)ₙONH₂, OCH₂C(=O)N(H)CH₃, and O(CH₂)ₙON[(CH₂)ₙCH₃]₂, where n and m are from 1 to about 10. Other 2'- substituent groups can also be selected from: C₁-C₁₂ alkyl, substituted alkyl, alkenyl, alkynyl, alkaryl, aralkyl, O-alkaryl or O-aralkyl, SH, SCH₃, OCN, Cl, Br, CN, CF₃, OCF₃, SOCH₃, SO₂CH₃, NO₂, N₃, NH₂, heterocycloalkyl, heterocycloalkaryl, aminoalkylamino, polyalkylamino, substituted silyl, an RNA cleaving group, a reporter group, an intercalator, a group for improving pharmacokinetic properties, and a group for improving the pharmacodynamic properties of an oligomeric compound, and other substituents having similar properties.

Further examples of nucleosides having modified sugar moieties include, without limitation, nucleosides comprising 5'-vinyl, 5'-methyl (R or S), 2'-F-5'-methyl, 4'-S, 2'-deoxy-2'-fluoro (2'-F), 2'-OCH₃ (2'-O-methyl, 2'-OMe), 2'-O(CH₂)₂OCH₃ (2'-O-methoxyethyl, 2'-O-MOE, 2'-MOE), 2'-O-methyl-4-pyridine, phosphorodiamidate morpholino (PMO), tricyclo-DNA (tcDNA), 2'-arabino-fluoro, 2'-O-benzyl, glycol nucleic acid (GNA), and unlocked nucleic acid (UNA) substituent groups. The substituent at the 2' position can also be selected from allyl, amino, azido, thio, O-allyl, O-C₁-C₁₀ alkyl, OCF₃, O(CH₂)₂SCH₃, O(CH₂)₂-O-N(Rm)(Rn), and O-CH₂-C(=O)-N(Rm)(Rn), where each Rm and Rn is, independently, H or substituted or unsubstituted C₁-C₁₀ alkyl. 2'-OMe or 2'-OCH₃ or 2'-O-methyl each refers to a nucleoside comprising a sugar comprising an -OCH₃ group at the 2' position of the sugar ring. 2'-F refers to a sugar comprising a fluoro group at the 2' position. 2'-O-methoxyethyl or 2'-O-MOE or 2'-MOE each refers to a nucleoside comprising a sugar comprising an -O(CH₂)₂OCH₃ group at the 2' position of the sugar ring.

BNAs refer to modified nucleosides comprising a bicyclic sugar moiety wherein a bridge connecting two carbon atoms of the sugar ring connects the 2' carbon and another carbon of the sugar ring. Examples of bicyclic nucleosides include, without limitation, nucleosides comprising a bridge between the 4' and the 2' ribosyl ring atoms such as in locked nucleic acid (LNA). Oligomeric compounds described herein can include one or more bicyclic nucleosides wherein the bridge comprises a 4' to 2' bicyclic nucleoside. LNAs and UNAs have been described by Campbell and Wengel (Chem Soc Rev, 2011, 40(12):5680-9).

Oligomeric compounds can comprise one or more nucleotides having modified sugar moieties. The modified sugar moiety can have a 2'-OMe modification. The modified sugar moiety can have a 2'-F modification. The 2'-OMe and/or 2'-F modified nucleotides can be arranged in a motif. The modifications can be arranged in Arnatar motifs as disclosed in PCT/US2023/084688.

The oligomeric compounds targeted to a nucleic acid can comprise a sense strand with motif described by the one of the following formulas:

Formula (I): 5' M-(Y)n-Z-(Y)r-D-D 3',

Formula (II): 5' Y-Z-(Y)q-FFNM-(Y)q-M-(Y)v-D-D 3',

Formula (III): 5' M*F*MMN*MN*MFFNMN*MN*MMFM*D*D 3',

Formula (X): 5' MFMMNMNMFFNMNMNMMNMDD 3',

or

Formula (XI): 5' MFMMNMNMFFMMNMNMMFMDD 3',

wherein
each D is a deoxyribonucleoside (D is a modification of R),
each R is a ribonucleoside,
each N is a nucleoside, modified or unmodified (e.g., D, R, M, F, UNA modified, or LNA modified),
each M is a 2'-OMe modified nucleoside,
each F is a 2'-F modified nucleoside,
each * is a phosphorothioate (PS) linkage,
each Y is two adjacent nucleosides with different modifications (e.g., MD, DM, DF, FD, MF or FM) or a modified nucleoside adjacent to an unmodified nucleoside (e.g., DR, RD, MR or RM),
each Z is two adjacent unmodified nucleosides or two adjacent nucleosides with the same modification or two adjacent unmodified nucleosides (e.g., MM, DD, RR, or FF),
each n is 6-8,
each q is 2-3,
each r is 1-2,
each v is 0-1, and
wherein no single modification type modifies more than two consecutive nucleotides. The FFNM can be FFRM or FFMM.

The oligomeric compounds targeted to a nucleic acid can comprise an antisense strand with motif described by one of the following formulas:

Formula (IV): 5'-L-M-(D)v-(Y)s-(Z)t-(Y)u-Z-N-(Z)r 3',

Formula (V): 5' L-(Y)p-NM-(FMM)r-(Y)p-(Z)r 3',

Formula (VI): 5' L-M*N*MNMFNMFMMNMFMFMMN*M*M 3',

Formula (VII): 5' L-M*D*MFMFNMFMMFMFMFMMN*M*M 3',

Formula (VIII): 5' L-MNMNMFNMFMMNMFMFMMNMM 3',

Formula (IX): 5' L-M-(Y)p-Z-(Y)p-(Z)r 3',

or

Formula (XII): 5' L-MDMFMFNMFMMFMFMFMMNMM 3',

wherein
each D is a deoxyribonucleoside (D is a modification of R),
each R is a ribonucleoside, each N is a nucleoside, modified or unmodified (e.g., D, R, M, F, UNA modified, or LNA modified),
each M is a 2'-OMe modified nucleoside,
each F is a 2'-F modified nucleoside,
each L is a 5' phosphate, 5' vinyl phosphonate or 5' OH
each * is a phosphorothioate (PS) linkage,
each Y is two adjacent nucleosides with different modifications (e.g., MD, DM, DF, FD, MF or FM) or a modified nucleoside adjacent to an unmodified nucleoside (e.g., DR, RD, MR or RM),
each Z is two adjacent unmodified nucleosides or two adjacent nucleosides with the same modification or two adjacent unmodified nucleosides (e.g., MM, DD, RR, or FF),
each (5p) is 5'-phosphate,
each n is 6-8,
each p is 3-5,
each r is 1-2,
each v is 0-1,
each s is 2-7,
each t is 0-2, and
wherein no single modification type modifies more than two consecutive nucleotides. The FNM can be FMM.

### Oligomeric Compound Delivery Systems

Oligomeric compounds require entry into target cells to become active. A variety of modalities have been used to traffic oligomeric compounds into target cells including viral delivery vectors, lipid-based delivery, polymer-based delivery, and conjugate-based delivery (Paunovska et al., Drug Delivery Systems for RNA Therapeutics, 2022, Nature Reviews Genetics, 23(5):265-280; Chen et al., 2022, Molecular Therapy, Nucleic Acids, 29:150-160).

Lipid-based particles can form specific structures such as micelles, liposomes and lipid nanoparticles (LPNs) to carry oligomeric compounds into cells. To form these particles, LPNs can include one or more of a cationic or ionizable lipid (e.g., DLin-MC3-DMA, SM-102, ALC-0315), cholesterol, a helper lipid, 1,2-distearoyl-sn-glycero-3-phosphocholine (DSPC), poly(ethylene glycol) (PEG) modified lipid (e.g., PEG-2000-C-DMG, PEG-2000-DMG, ALC-0159), C12-200, cKK-E12 and the like. Different combinations of lipids can be formulated to affect the delivery of the oligomeric compound to different types of cells. In one example, therapeutic siRNA patisiran was formulated in cationic ionizable lipid DLin-MC3-DMA, cholesterol, polar phospholipid DSPC, and PEG-2000-C-DMG for delivery to hepatocytes.

Polymer-based particles are also used in oligomeric compound delivery systems. Such polymers include poly(lactic-co-glycolic acid) (PLGA), polyethylenimine (PEI), poly(l-lysine) (PLL), poly(beta-amino ester) (PBAE), dendrimers (e.g., poly(amidoamine) (PAMAM) or PLL), and other polymers or modified polymers thereof. The polymer composition can be varied depending on the traits desired for delivery of the oligomeric compound.

The oligomeric compounds disclosed herein may be covalently linked to one or more moieties or conjugates which enhance the activity, cellular distribution or cellular uptake of the resulting compound. Conjugate groups can include cholesterols, lipids, carbohydrates, phospholipids, biotin, phenazine, folate, phenanthridine, anthraquinone, acridine, fluoresceins, rhodamines, coumarins, dyes, etc. Conjugate-based delivery can actively deliver oligomeric compounds to specific cell types.

In an example, an N-Acetylgalactosamine (GalNAc)-comprising moiety is conjugated to an oligomeric compound and delivers it into hepatocytes. Various GalNAc conjugates can be found in several publications including the following: Sharma et al., 2018, Bioconjugate Chem, 29:2478-2488; Nair et al., J. Am. Chem. Soc. 2014, 136(49):16958-16961; Keam, 2022, Drugs, 82:1419-1425; US Patent 10,087,208; Prakash et al., 2014, Nucleic Acids Res, 42(13):8796-807; Debacker et al., 2020, Molecular Therapy, 28(8):1759-1771; PCT/US2023/084692; US Patent 11,110,174; US Patent 9,796,756; US Patent 9,181,549; US Patent 10,344,275; US Patent 10,570,169; US Patent 9,506,030; and, US Patent 7,582,744.

In certain embodiments, the following GalNAc conjugate precursor with solid support can be used to conjugate an AGT dsRNA at the 3' end of the sense strand. In a preferred embodiment of the disclosure, the dsRNA is an ARNATAR designed siRNA according to the disclosure selected from Tables 2, 5, 10 or 13.

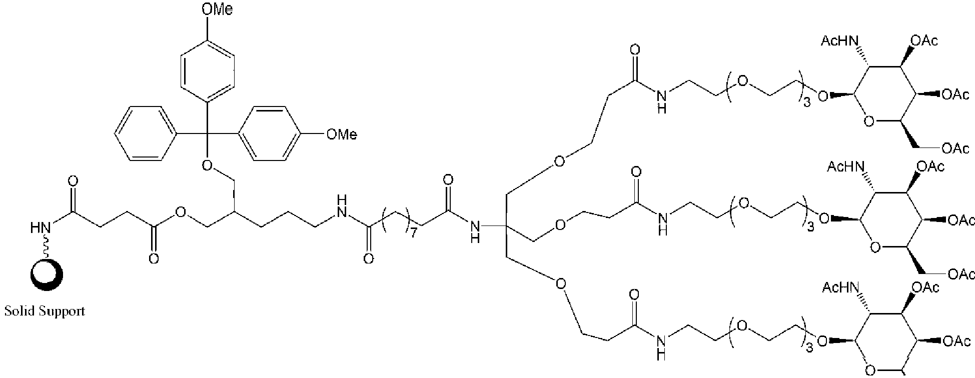

In a preferred embodiment, the GalNAc-comprising moiety conjugated to an oligonucleotide is GalNAc-AN (also known as GA4 or GA-AN) shown as follows:

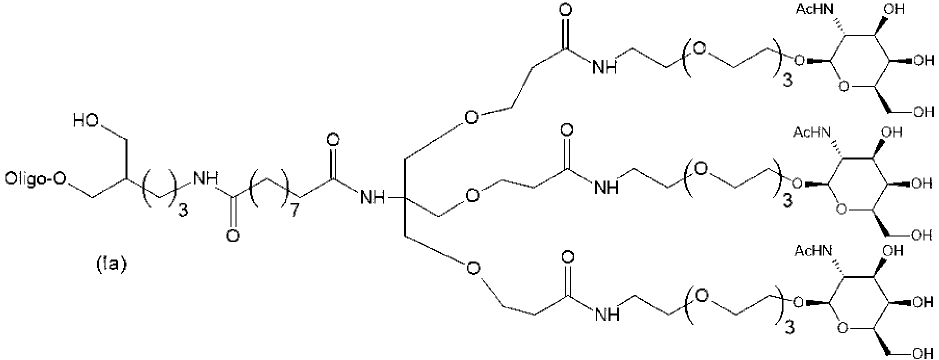

In one embodiment, GalNAc-AN is conjugated to a sense strand of a dsRNA compound. In a preferred embodiment of the disclosure, GalNAc-AN is conjugated to a dsRNA compound selected from any of the compounds according to the disclosure in Tables 2, 5, 10 or 13.

### Oligomeric Compound Synthesis

siRNAs were designed, synthesized, and prepared using methods known in the art.

Solid phase syntheses of oligonucleotides were done on a MerMade^{™} 48x synthesizer (BioAutomation, LGC, Biosearch Technologies, Hoddesdon, UK), which can make up to 48 1µMole or 5µMole scale oligonucleotides per run using standard phosphoramidite chemistry. Phosphoramidite synthesis of oligonucleotides on a solid support is well known in the art (e.g., Beaucage and Caruthers, 1981, Tetrahedron Letters, 22(20): 1859-1862; Roy and Caruthers, 2013, Molecules, 18:14268-14284; and Sandahl et al., 2021, Nature Communications, 12:2760). Solid support is controlled pore glass (500-1400 Å) loaded with universal linkers or loaded with 3'-GalNAc conjugates (e.g., AM Chemicals, Vista, CA, USA; Primetech ALC, Minsk, Belarus; Gene Link, Elmsford, NY, USA; or any GalNAc conjugate disclosed herein) or universal solid support (AM Chemicals, Vista, CA, USA). Ancillary synthesis reagents and standard 2'-cyanoethyl phosphoramidite monomers (2'-fluoro, 2'-O-methyl, RNA, DNA) were obtained from various sources (Hongene Biotech, Shanghai, China; Sigma-Aldrich, St. Louis, MO, USA; Glen Research, Sterling, VA, USA; ThermoFisher Scientific, Waltham, MA, USA; LGC Biosearch Technologies, Hoddesdon, UK). Phosphoramidite mixtures were prepared in anhydrous acetonitrile or 30% DMF:acetonitrile and were coupled using 0.25M 4,5-dicyanoimidazole(DCI) (Sigma-Aldrich, St. Louis, MO, USA) with coupling times ranging from 120-360 seconds. Standard phosphodiester linkages were achieved using 0.02M iodine mixture in Tetrahydrofuran (THF), pyridine and water. Phosphorothiate linkages were generated using 0.05M sulfurizing Reagent II (3-((Dimethylamino-methylidene)amino)-3H-1,2,4-dithiazole-3-thione, DDTT) (40:60,Pyridine/ Acetonitrile) (LGC Biosearch Technologies, Hoddesdon, UK) with an oxidation time of 6 minutes. All sequences were synthesized with Dimethoxy Trityl (DMT) protecting group removed.

Upon completion of solid phase synthesis, the oligonucleotides were cleaved from the solid support and deprotection of base labile groups performed by incubation in ammonium hydroxide at 55°C for 6 hours. Ammonium hydroxide was removed using a centrifugal vacuum concentrator to dryness at room temperature. For sequences containing natural ribonucleotides (2'-OH) protected with tert-butyl dimethyl silyl (TBDMS), a second deprotection was performed using triethylamine; trihydrofluoride (TEA:3HF). To each TBDMS protected oligonucleotide 100 µL DMSO and 125µL TEA:3HF was added and incubated at 65°C for 2.5 hours. After incubation 25µL of 3M sodium acetate was added to the solution which was subsequently precipitated in butanol at -20°C for 30 minutes. The cloudy solution was centrifuged to a cake at which time the supernatant was carefully decanted with a pipette. The standard precipitation process was then completed with 75% ethanol:water then 100% ethanol as supernatant solutions. The oligonucleotide cake was dried for 30 minutes in a centrifugal vacuum concentrator.

Desalting without HPLC purification was performed after precipitation with 3M sodium acetate with a follow on G25 Sephadex^{®} column (Sigma-Aldrich, St. Louis, MO, USA) elution. Purification of oligonucleotides was afforded by anion exchange chromatography on a Gilson GX271 prep HPLC system (Middleton, WI, USA) using BioWorks Q40 resin (Uppsala, Sweden). Final desalt was performed by Sephadex^{®} G25 column. All oligonucleotides were analyzed by ion pairing reverse phase HPLC for purity an Agilent 1200 analytical HPLC (Santa Clara, CA, USA), negative ion mass spectrometry for intact mass on an Agilent 6130 single quad mass spectrometer (Santa Clara, CA, USA), and A260 quantification by UV/Vis on a Tecan Infinite^{®} M Plex plate reader (Zurich, Switzerland).

### Double Stranded Oligomeric Compound Duplex Formation

In general, for a double stranded oligomeric compound such as an siRNA compound, a sense and antisense oligonucleotide is annealed together to form a duplex. The duplex is formed by contacting the sense strand prepared according any one of the processes described herein with the antisense strand prepared according any one of the processes described herein in equimolar concentrations in a solution. Optionally, the solution is heated to a temperature of about 94°C then the temperature is reduced to about 25°C. In an example, duplex formation of 50-300mM can be achieved by heating samples at 94°C for 4 mins in 1x phosphate-buffered saline in a block heater, followed by removal of the heating block containing the samples from the block heater and allowing it to gradually cool down to room temperature over a time course of 1hr.

### Compositions and Methods for Formulating Pharmaceutical Compositions

The dsRNA compounds, such as siRNAs targeting AGT described herein, can be combined with pharmaceutically acceptable active or inert substances, such as a diluent, excipient or carrier, for the preparation of pharmaceutical compositions or formulations.

Compositions and methods for the formulation of pharmaceutical compositions are dependent upon a number of criteria, including, but not limited to, route of administration, extent of disease, or dose to be administered.

The pharmaceutical carrier or excipient can be a pharmaceutically acceptable solvent, suspending agent or any other pharmacologically inert vehicle for delivering one or more nucleic acid compounds to an animal. The excipient can be liquid or solid and can be selected, with the planned manner of administration in mind, so as to provide for the desired bulk, consistency, etc., when combined with a nucleic acid and the other components of a given pharmaceutical composition. Typical pharmaceutical carriers include, but are not limited to, binding agents (e.g., pregelatinized maize starch, polyvinylpyrrolidone or hydroxypropyl methylcellulose, etc.); fillers (e.g., lactose and other sugars, microcrystalline cellulose, pectin, gelatin, calcium sulfate, ethyl cellulose, polyacrylates or calcium hydrogen phosphate, etc.); lubricants (e.g., magnesium stearate, talc, silica, colloidal silicon dioxide, stearic acid, metallic stearates, hydrogenated vegetable oils, corn starch, polyethylene glycols, sodium benzoate, sodium acetate, etc.); disintegrants (e.g., starch, sodium starch glycolate, etc.); and wetting agents (e.g., sodium lauryl sulphate, etc.).

Pharmaceutically acceptable organic or inorganic excipients, which do not deleteriously react with nucleic acid compounds, suitable for parenteral or non-parenteral administration can also be used to formulate the compositions of the present invention. Suitable pharmaceutically acceptable carriers include, but are not limited to, water, salt solutions, alcohols, polyethylene glycols, gelatin, lactose, amylose, magnesium stearate, talc, silicic acid, viscous paraffin, hydroxymethylcellulose, polyvinylpyrrolidone and the like. A pharmaceutically acceptable diluent includes phosphate-buffered saline (PBS). PBS is a diluent suitable for use in compositions to be delivered parenterally. Accordingly, employed in the methods described herein can be a pharmaceutical composition comprising a dsRNA compound targeted to an AGT nucleic acid and a pharmaceutically acceptable diluent. The pharmaceutically acceptable diluent can be PBS. The dsRNA compound can be an siRNA.

Pharmaceutical compositions comprising dsRNA compounds such as siRNAs can encompass any pharmaceutically acceptable salts, esters, or salts of such esters, or any other dsRNA which, upon administration to an animal, including a human, is capable of providing (directly or indirectly) the biologically active metabolite or residue thereof. Accordingly, for example, the disclosure is also drawn to pharmaceutically acceptable salts of dsRNA compounds, prodrugs, pharmaceutically acceptable salts of such prodrugs, and other bioequivalents. Suitable pharmaceutically acceptable salts include, but are not limited to, sodium and potassium salts.

A pharmaceutical composition can bes prepared for administration by injection (e.g., intravenous, subcutaneous, intramuscular, etc.). A pharmaceutical composition can comprise a carrier and is formulated in aqueous solution, such as water or physiologically compatible buffers such as Hanks's solution, Ringer's solution, or physiological saline buffer (e.g., PBS). Other ingredients can be included (e.g., ingredients that aid in solubility or serve as preservatives). Injectable suspensions can be prepared using appropriate liquid carriers, suspending agents and the like. Certain pharmaceutical compositions for injection are presented in unit dosage form, e.g., in ampoules or in multi-dose containers.

### Dosages

For purposes of the disclosure, the amount or dose of the active agent administered should be sufficient to e.g., inhibit the expression of AGT in an animal. In the animal (e.g., human), dose will be determined by the efficacy of the particular active agent and the condition of the animal, as well as the body weight of the animal to be treated.

Many assays for determining an administered dose are known in the art.

The dose of the active agent of the present disclosure also will be determined by the existence, nature and extent of any adverse side effects that might accompany the administration of a particular active agent of the present disclosure. Typically, the attending physician will decide the dosage of the active agent of the present disclosure with which to treat each individual subject, taking into consideration a variety of factors, such as age, body weight, general health, diet, sex, active agent of the present disclosure to be administered, route of administration, and the severity of the condition being treated.

### Dosing

Pharmaceutical compositions can be administered according to a dosing regimen (e.g., dose, dose frequency, and duration) wherein the dosing regimen can be selected to achieve a desired effect i.e., is therapeutically effective in a subject. The desired effect can be, for example, reduction of AGT or the prevention, reduction, amelioration or slowing the progression of a disease, disorder and/or condition, or symptom thereof, associated with AGT or the RAAS pathway in a subject. The variables of the dosing regimen can be adjusted to result in a desired concentration of pharmaceutical composition in a subject. "Concentration of pharmaceutical composition" as used with regard to dose regimen can refer to the dsRNA compound or active ingredient(s) of the pharmaceutical composition. For example, dose and dose frequency can be adjusted to provide a tissue concentration or plasma concentration of a pharmaceutical composition at an amount sufficient to achieve a desired effect.

Dosing is dependent on severity and responsiveness of the disease state to be treated, with the course of treatment lasting from several days to several months, or until a cure is affected or a diminution of the disease state is achieved. Dosing is also dependent on drug potency and metabolism differences between subjects. In certain embodiments, a therapeutically effective dosage in a subject is from 0.01 µg to 50 mg per kg of body weight, 0.01 µg to100 mg per kg of body weight, or within a range of 0.001 mg to 1000 mg dosing, and may be given once or more daily, weekly, monthly, quarterly or yearly, or even once every 2 to 20 years. Following successful treatment, it may be desirable to have the subject undergo maintenance therapy to prevent the recurrence of the disease state, wherein the dsRNA is administered in maintenance doses, ranging from 0.01 µg to 100 mg per kg of body weight, once or more daily, once or more weekly, once or more monthly, once or more quarterly, once or more yearly, to once every 20 years or ranging from 0.001 mg to 1000 mg dosing. In certain embodiments, it may be desirable to administer the dsRNA compound from at most once daily, once weekly, once monthly, once quarterly, once yearly, once every two years, once every three years, once every four years, once every five years, once every ten year, to once every 20 years.

In certain embodiments, the range of therapeutically effective dosing is between any of 1mg-1500mg, 100mg-1400mg, 100mg-1300mg, 100mg-1200mg, 100mg-1100mg, 100mg-1000mg, 100mg-900mg, 200mg-800mg, 300mg-700mg, 400mg-600mg, 100mg-400mg, 200mg-500mg, 300mg-600mg, and 400mg-700mg. In certain embodiments, a therapeutically effective dose is about 100mg, 150mg, 200mg, 250mg, 300mg, 350mg, 400mg, 450mg, 500mg, 550mg, 600mg, 650mg, 700mg, 800mg, 850mg, 900mg, 950mg, 1000mg, 1050mg, 1100mg, 1150mg, 1200mg, 1250mg, 1300mg, 1350mg, 1400mg, 1450mg, or 1500mg. In certain embodiments, a preferred dose is selected from 700mg, 800mg and 900mg.

In certain embodiments, a therapeutically effective amoung of the dsRNA is dosed at any of about 150mg, 200mg, 300mg, 400mg, 500mg, 600mg, 700mg, 800mg, or 900mg twice a year. In certain embodiments, a therapeutically effective amoung of the dsRNA is dosed at about 150mg, 200mg, 300mg, 400mg, 500mg, 600mg, 700mg, 800mg, or 900mg quarterly. In certain embodiments, a therapeutically effective amount of the dsRNA is dosed at about 150mg, 300mg or 600mg once every 3 months. In certain embodiments, a therapeutically effective amount of the dsRNA is dosed at about 150mg, 300mg or 600mg once every 6 months.

### Administration

The dsRNA compounds such as siRNAs or pharmaceutical compositions of the present invention can be administered in a number of ways depending upon whether local or systemic treatment is desired and upon the area to be treated. Administration can be oral, inhaled or parenteral.

In certain embodiments, the compounds and compositions as described herein are administered parenterally. Parenteral administration includes intravenous, intra-arterial, subcutaneous, intraperitoneal or intramuscular injection or infusion; or intracranial, e.g., intrathecal or intraventricular, administration. In certain embodiments, parenteral administration is by infusion. Infusion can be chronic or continuous or short or intermittent. In certain embodiments, infused pharmaceutical agents are delivered with a pump.

In certain embodiments, parenteral administration is by injection. The injection can be delivered with a syringe or a pump. In certain embodiments, the injection is a bolus injection. In certain embodiments, the injection is administered directly to a tissue or organ.

In certain embodiments, formulations for parenteral, intrathecal or intraventricular administration can include sterile aqueous solutions which can also contain buffers, diluents and other suitable additives such as, but not limited to, penetration enhancers, carrier compounds and other pharmaceutically acceptable carriers or excipients.

In certain embodiments, formulations for oral administration of the compounds or compositions can include, but is not limited to, pharmaceutical carriers, excipients, powders or granules, microparticulates, nanoparticulates, suspensions or solutions in water or non-aqueous media, capsules, gel capsules, sachets, tablets or minitablets. Thickeners, flavoring agents, diluents, emulsifiers, dispersing aids or binders can be desirable. In certain embodiments, oral formulations are those in which compounds provided herein are administered in conjunction with one or more penetration enhancers, surfactants and chelators.

### In Vitro Testing of dsRNAs

Described herein are methods for treatment of cells with dsRNAs, for example siRNAs, which can be modified appropriately for treatment with other oligomeric compounds.

Cells may be treated with siRNAs when the cells reach approximately 60-80% confluency in culture.

One reagent commonly used to introduce siRNAs into cultured cells includes the cationic lipid transfection reagent Lipofectamine^{™} RNAiMAX (Invitrogen, Waltham, MA). siRNAs may be mixed with Lipofectamine^{™} RNAiMAX in OPTI-MEM 1 (Thermo Fisher Scientific, Waltham, MA) to achieve the desired final concentration of siRNA and a Lipofectamine^{™} RNAiMAX concentration that may range from 0.001 to 300 nM siRNAs. Transfection procedures are done according to the manufacturer's recommended protocols.

Another technique used to introduce siRNAs into cultured cells includes electroporation.

siRNAs conjugated with a GalNAc-comprising moiety can be introduced to cells through incubation of the siRNAs with cells without transfection reagents, referenced herein as "free uptake". The siRNA-GalNAc conjugates are transported into asialoglycoprotein receptor (ASGR) positive cells such as hepatocytes via endocytosis.

Cells are treated with siRNAs by routine methods. Cells may be harvested 4 -144 hours after siRNAs treatment, at which time mRNA (harvested at 4-144 hrs) or protein levels (extracted at 24-96 hrs) of target nucleic acids are measured by methods known in the art and described herein. In general, treatments are performed in multiple replicates, and the data are presented as the average of the replicate treatments plus the standard deviation.

The concentration of siRNAs used varies from cell line to cell line and target to target. Methods to determine the optimal siRNAs concentration for a particular target in a particular cell line are well known in the art. In general, cells are treated with siRNAs in a dose dependent manner to allow for the calculation of the half-maximal inhibitory concentration value (IC50). siRNAs are typically used at concentrations ranging from 0.001 nM to 300 nM when transfected with Lipofectamine^{™} RNAiMAX. siRNAs are used at higher concentrations ranging from 625 to 20,000 nM when transfected using electroporation or free uptake.

### RNA Isolation

RNA analysis of AGT mRNA levels can be performed on total cellular RNA or poly(A)+ mRNA. Methods of RNA isolation are well known in the art. RNA is prepared using methods well known in the art, for example, using the TRIZOL Reagent (Thermo Fisher Scientific, Waltham, MA), Qiagen RNeasy kit (Qiagen, Hilden, Germany), or AcroPrep Advance 96-well Filter Plates (Pall Corporation, Port Washington, New York) using Qiagen's RLT, RW1 and RPE buffers. RNA extraction procedures are done according to the manufacturer's recommended protocols.

### In Vivo Testing of dsRNA Compounds

dsRNA compounds, for example, siRNAs, are tested in animals to assess their ability to inhibit expression of AGT and/or the RAAS pathway and produce phenotypic changes such as a decrease in one or more RAAS pathway related diseases. Testing may be performed in normal animals, or in experimental disease models. For administration to animals, dsRNAs are formulated in a pharmaceutically acceptable diluent, such as phosphate-buffered saline. Administration includes parenteral routes of administration, such as intraperitoneal, intravenous, and subcutaneous. Calculation of dsRNA dosage and dosing frequency depends upon factors such as route of administration and animal body weight. Following a period of treatment with dsRNAs, mRNA encoding AGT can be isolated from liver tissue and changes in AGT expression are measured. Changes in AGT protein levels can also be measured. Changes in AGT expression can also be measured by determining the level of inhibition of the RAAS pathway in the animal. RAAS pathway related diseases, disorders and/or conditions may be used as markers for determining the level of AGT inhibition in the animal.

### Certain Indications

Described herein are methods of treating a subject comprising administering one or more compounds and/or pharmaceutical compositions of the present invention to the subject. The subject can have, or can be at risk for, a RAAS pathway related disease, disorder and/or condition, or symptom thereof. Described herein are methods for prophylactically reducing AGT expression in a subject. Described herein is treating a subject in need thereof by administering to the subject a therapeutically effective amount of a dsRNA compound such as an siRNA targeted to an AGT nucleic acid.

In certain embodiments, administration to a subject of a therapeutically effective amount of a dsRNA compound targeted to an AGT nucleic acid is accompanied by monitoring of AGT levels in the blood plasma or tissue of the subject, to determine a subject's response to administration of the dsRNA compound. A subject's response to administration of the dsRNA compound is used by a physician to determine the amount and duration of therapeutic intervention.

In certain embodiments, administration to a subject of a dsRNA compound targeted to an AGT nucleic acid results in reduction of AGT expression by at least 15%, 20%, 25%, 30%, 35%, 40%, 45%, 50%, 55%, 60%, 65%, 70%, 75%, 80%, 85%, 90%, 95%, 99% or 100% or a range defined by any two of these values. In certain embodiments, administration to a subject of a dsRNA compound targeted to an AGT nucleic acid results in inhibition of the RAAS pathway by at least 15%, 20%, 25%, 30%, 35%, 40%, 45%, 50%, 55%, 60%, 65%, 70%, 75%, 80%, 85%, 90%, 95%, 99% or 100% or a range defined by any two of these values. In certain embodiments, administration to a subject of a dsRNA compound targeted to an AGT nucleic acid results in a change the RAAS pathway related disease, disorder, condition, symptom or marker (e.g., hypertension or organ damage) in the subject. In certain embodiments, administration to a subject of an AGT dsRNA compound increases or decreases the RAAS related disease, disorder, condition, symptom or marker by at least 15%, 20%, 25%, 30%, 35%, 40%, 45%, 50%, 55%, 60%, 65%, 70%, 75%, 80%, 85%, 90%, 95%, 99% or 100% or a range defined by any two of these values in the subject.

Described herein are pharmaceutical compositions comprising a dsRNA compound targeted to AGT used for the preparation of a medicament for treating a subject suffering or susceptible to a RAAS related disease, disorder or condition.

The dsRNA compound can be an ARNATAR designed siRNA targeting AGT as listed in Tables 2, 5, 7, 9, 10 or 13.

### Certain Combination Therapies

In certain embodiments, a first agent comprising a dsRNA compound provided herein is co-administered with one or more secondary agents to a subject. In certain embodiments of the disclosure, the dsRNA compound is an ARNATAR siRNA according to the disclosure listed in Tables 2, 5, 10 or 13.

In certain embodiments, such second agents are designed to treat the same RAAS pathway related disease, disorder or condition as the first agent described herein. In certain embodiments, such second agents are designed to treat a different disease, disorder, or condition as the first agent described herein. In certain embodiments, such second agents are designed to treat an undesired side effect of one or more pharmaceutical compositions as described herein. In certain embodiments, such first agents are designed to treat an undesired side effect of a second agent. In certain embodiments, second agents are co-administered with the first agent to treat an undesired effect of the first agent. In certain embodiments, second agents are co-administered with the first agent to produce a combinational or additive effect. In certain embodiments, second agents are co-administered with the first agent to produce a synergistic effect.

In certain embodiments, the co-administration of the first and second agents permits use of lower dosages than would be required to achieve a therapeutic or prophylactic effect if the agents were administered as independent therapy. In certain embodiments the dose of a co-administered second agent is the same as the dose that would be administered if the second agent was administered alone. In certain embodiments the dose of a co-administered second agent is greater than the dose that would be administered if the second agent was administered alone.

In certain embodiments, a first agent and one or more second agents are administered at the same time. In certain embodiments, the first agent and one or more second agents are administered at different times. In certain embodiments, the first agent and one or more second agents are prepared together in a single pharmaceutical formulation. In certain embodiments, the first agent and one or more second agents are prepared separately.

In certain embodiments, second agents include, but are not limited to, certain procedures to reduce hypertension, diet changes, lifestyle changes, anti-fibrotic drugs and anti-hypertensive drugs such as RAAS inhibitors, diuretics, calcium channel blockers, adrenergic receptor antagonists, adrenergic agonists and vasodilators.

Examples of procedures that can reduce hypertension include, but are not limited to, renal denervation and baroreceptor activation therapy.

Examples of RAAS inhibitors include, but are not limited to ACE inhibitors (e.g., captopril, enalapril, fosinopril, lisinopril, perindopril, quinapril, ramipril, trandolapril and benazepril), angiotensin II receptor antagonists (e.g., candesartan, eprosartan, irbesartan, losartan, olmesartan, telmisartan and valsartan), renin inhibitors (e.g., aliskiren), and aldosterone receptor antagonists (e.g., eplerenone and spironolactone).

Examples of diuretics include loop diuretics (e.g., bumetanide, ethacrynic acid, furosemide, torsemide), thiazide diuretics (e.g., epitizide, hydrochlorothiazide, chlorothiazide and bendroflumethiazide), thiazide-like diuretics (e.g., indapamide, chlorthalidone and metolazone) and potassium-sparing diuretics (e.g., amiloride, triamterene and spironolactone).

Examples of calcium channel blockers include dihydropyridines (e.g., amlodipine, felodipine, isradipine, lercanidipine, nicardipine, nifedipine, nimodipine and nitrendipine) and non-dihydropyridines (e.g., diltiazem and verapamil).

Examples of adrenergic receptor antagonists include Beta blockers (e.g., atenolol, metoprolol, nadolol, oxprenolol, pindolol, propranolol and timolol), Alpha blockers (e.g., doxazosin, phentolamine, indoramin, phenoxybenzamine, prazosin, terazosin and tolazoline) and mixed Alpha + Beta blockers (e.g., bucindolol, carvedilol and labetalol).

Examples of vasodilators include sodium nitroprusside and hydralazine and its derivatives.

Examples of adrenergic agonists include alpha-2 agonists (e.g., clonidine, guanabenz, methyldopa and moxonidine).

Additional examples of anti-hypertensive drugs include guanethidine, reserpine and the like.

The second agents can be used in combination with the therapeutic compounds described herein to decrease a RAAS pathway related disease, disorder and/or condition such as hypertension, organ damage and the like in the subject.

### Kits of The Invention

According to another aspect of the invention, kits are provided. Kits according to the invention include package(s) comprising any of the compositions of the invention or oligomeric compound of the invention. In various aspects, the kit comprises any of the compositions of the invention as a unit dose. For purposes herein "unit dose" refers to a discrete amount dispersed in a suitable carrier.

The phrase "package" means any vessel containing compositions presented herein. In preferred embodiments, the package can be a box or wrapping. Packaging materials for use in packaging pharmaceutical products are well known to those of skill in the art. Examples of pharmaceutical packaging materials include, but are not limited to, blister packs, bottles, tubes, inhalers, pumps, bags, vials, containers, syringes (including pre-filled syringes), bottles, and any packaging material suitable for a selected formulation and intended mode of administration and treatment.

The kit can also contain items that are not contained within the package but are attached to the outside of the package, for example, pipettes.

Kits may optionally contain instructions for administering compositions of the present invention to a subject having a condition in need of treatment. Kits may also comprise instructions for approved uses of components of the composition herein by regulatory agencies, such as the United States Food and Drug Administration. Kits may optionally contain labeling or product inserts for the present compositions. The package(s) and/or any product insert(s) may themselves be approved by regulatory agencies. The kits can include compositions in the solid phase or in a liquid phase (such as buffers provided) in a package. The kits also can include buffers for preparing solutions for conducting the methods, and pipettes for transferring liquids from one container to another.

The kit may optionally also contain one or more other compositions for use in combination therapies as described herein. In certain embodiments, the package(s) is a container for any of the means for administration such as intravitreal delivery, intraocular delivery, intratumoral delivery, peritumoral delivery, intraperitoneal delivery, intrathecal delivery, intramuscular injection, subcutaneous injection, intravenous delivery, intra-arterial delivery, intraventricular delivery, intrasternal delivery, intracranial delivery, or intradermal injection.

### Methods of Use and Compounds for Use

Described hereon are methods for inhibiting the expression of AGT in a subject and methods of treating a RAAS associated disease, disorder and/or condition or the symptoms thereof in a subject comprising administering an effective amount of a dsRNA compound of the invention or a pharmaceutical composition of the invention, so as to inhibit the expression of AGT in the subject. The invention also provides the compounds for use in treating or preventing a RAAS associated disease, disorder and/or condition in a subject.
In some embodiments of the present disclosure, the subject is a mammal, including, but not limited to, mammals of the order Rodentia, such as mice and hamsters, and mammals of the order Logomorpha, such as rabbits, mammals from the order Carnivora, including Felines (cats) and Canines (dogs), mammals from the order Artiodactyla, including Bovines (cows) and Swines (pigs) or of the order Perissodactyla, including Equines (horses). In some aspects, the mammals are of the order Primates, Ceboids, or Simoids (monkeys) or of the order Anthropoids (humans and apes). In a preferred aspect, the mammal is a human.

### ADVANTAGES OF THE INVENTION

Disclosed herein are dsRNA compounds (e.g., siRNAs) targeting AGT improved with Advanced RNA Targeting (ARNATAR) abilities that enhance their gene silencing activity. The dsRNA compounds utilize ARNATAR motifs in conjunction with AGT targeting sequences to produce stable and durable therapeutic compounds allowing longer lasting benefits for acute and chronic diseases and/or less frequent dosing of the therapeutic compound. In addition to stability and durability, the dsRNA compounds utilizing ARNATAR motifs may have a quicker mode of action (for example, by knocking down AGT expression at an earlier time than the reference compound), which would be beneficial for acute diseases. In some instances, ARNATAR dsRNAs have been found to be more potent than a reference compound.

Another benefit of ARNATAR designed dsRNAs targeting AGT is a shortness of length. This shortness of length allows a shorter synthesis protocol and shorter synthesis time and decreases the cost of manufacturing the compounds.

Additionally, ARNATAR designed dsRNA compounds are very potent inhibitors of AGT. The high potency allows AGT reduction in tissues other than liver.

Accordingly, there is a need for improved dsRNA compounds to treat diseases. ARNATAR dsRNA compounds targeting AGT have been designed to improve speed, stability, specificity, safety and potency in order to produce an improved therapeutic.

### EXAMPLES

While certain compounds, compositions and methods described herein have been described with specificity in accordance with certain embodiments, the following examples serve only to illustrate the compounds described herein and are not intended to limit the same. Only examples are according to the invention which relate to a double-stranded ribonucleic acid, wherein the sense strand comprises the nucleotide sequence of any one of SEQ ID NO: 8, 33 and 55, and wherein the antisense strand comprises the nucleotide sequence of any one of SEQ ID NO: 9 and 19.

### EXAMPLE 1: DESIGNING AGT siRNA WITH VARYING CHEMICAL MOTIFS

An angiotensinogen (AGT) transcript (Table 1) was targeted by siRNAs designed with ARNATAR motifs.

**Table 1: AGT Target Sequence**

| Name | SEQ ID NO: |
|---|---|
| GenBank NM_001382817.3 | 1 |

AGT targeting siRNAs (also known as AGT siRNAs) were designed as shown in Table 2.

The following applies to all modified sequences disclosed herein:
A notation is made before each nucleotide indicating the type of chemical modification, if any, made to the nucleotide. If no modification notation is made before a letter designating a nucleotide, the nucleotide is a deoxyribonucleotide. Notations for the chemical modifications to the strands can be found as follows:
(5p) = 5'-phosphate
r = ribonucleotide
d (or no notation made before a nucleotide) = deoxyribonucleotide which has been substituted for a ribonucleotide
f = 2'-F
m = 2'-OMe
* = phosphorothioate (PS) linkage which has been substituted for a phosphate (PO) linkage
gna = glycol nucleic acid

If more than one sequence is disclosed in one row of the tables, the SEQ ID NO applies to the modified sequence ("Sequence + Chemistry").

Each ARNATAR sense strand is conjugated with a GalNAc-comprising moiety as described by Sharma (Sharma et al., 2018, Bioconjugate Chem, 29:2478-2488; also known as GalNAc3 or GA3 herein; Gene Link, Elmsford, NY, USA) or a GalNAc from AM Chemicals (US Patent 10,087,208; also known herein as GalNAc2 or GA2). As a control, a reference siRNA (also known as ATXL-G or benchmark siRNA herein) was synthesized and included in the studies described herein. This reference siRNA ATXL-G (Table 2A) mirrors a compound known as AD85481 in the prior art (WO2019/222166) and is believed to be Zilebesiran which is currently in clinical trials.

**Table 2. AGT siRNAs with Modifications to Both Strands**

| siRNA Name | Sense or Antisense | Strand Name | Sequence + Chemistry (5' to 3') | Sequence | SEQ ID NO: |
|---|---|---|---|---|---|
| ATsi480 | sense | ATs480 | mC*fC*mAmArC*mArG*mCf UfUmAmCrC*mArA*mGmCf CmU*T*T-GA2 | CCAACAGCUUA CCAAGCCUTT | 2 |
| | Antisense | ATa480 | (5p)mA*G*mGfCmUfUmGmG fUmAmAfGmCfUmGfUmUm GfG*mG*mU | AGGCUUGGUAA GCUGUUGGGU | 3 |
| ATsi481 | sense | ATs481 | mU*fU*mAmA*rC*mA*rA*m GfCfCmUmA*rA*mG*rG*mU mCfUmU*T*T-GA3 | UUAACAAGCCU AAGGUCUUTT | 4 |
| | Antisense | ATa481 | (5p)mA*A*mGfAmCfCmUmU fAmGmGfCmUfUmGfUmUm AfA*mG*mC | AAGACCUUAGG CUUGUUAAGC | 5 |
| ATsi489 | sense | ATs489 | mU*fU*mAmAfCmAfAmGfCf CmUmAfAmGfGmUmCfUmU *T*T-GA3 | UUAACAAGCCU AAGGUCUUTT | 6 |
| | Antisense | ATa489 or ATa597 | (5p)mA*A*mGfAmCfCT*mUf AmGmGfCmUfUmGfUmUmA A*mG*mC | AAGACCTUAGG CUUGUUAAGC | 7 |
| ATsi603 | sense | ATs603 or ATs612 | mU*fG*mCmAfAmGfAmCfUf UmGmAfCmAfCmCmGfAmA *T*T-GA3 | UGCAAGACUUG ACACCGAATT | 8 |
| | Antisense | ATa603, ATa565 or ATa786 | (5p)mU*T*mCfGmGfUG*mUf CmAmAfGmUfCmUfUmGmC A*mG*mC | UTCGGUGUCAA GUCUUGCAGC | 9 |
| ATsi605 | sense | ATs605 | mU*fG*mCmAfAmAfAmCfUf UmGmAfCmAfCmCmGfAmA *T*T-GA3 | UGCAAAACUUG ACACCGAATT | 10 |
| | Antisense | ATa605 | (5p)mU*T*mCfGmGfUG*mUf CmAmAfGmUfUmUfUmGmC A*mG*mC | UTCGGUGUCAA GUUUUGCAGC | 11 |
| ATsi606 | sense | ATs606 | mC*fC*mAmAfCmAfGmCfUf UmAmAfCmAfAmGmCfCmU *T*T-GA3 | CCAACAGCUUA ACAAGCCUTT | 12 |
| | Antisense | ATa606 | (5p)mA*G*mGfCmUfUG*mU fUmAmAfGmCfUmGfUmUm GG*mG*mU | AGGCUUGUUAA GCUGUUGGGU | 13 |
| ATsi609 | sense | ATs600 or ATs484 | mC*fC*mAmAfUmAfGmCfUf UmAmAfCmAfAmGmCfCmU *T*T-GA3 | CCAAUAGCUUA ACAAGCCUTT | 14 |
| | Antisense | ATa609 | (5p)mA*G*mGfCmUfUmGmU fUmAmAfGmCfUmAfUmUm GfG*mU*mU | AGGCUUGUUAA GCUAUUGGUU | 15 |
| ATsi610 | sense | ATs610 | mA*fC*mCmCfAmAfUmAfGf CmUmUfAmAfCmAmAfGmC *C*T-GA3 | ACCCAAUAGCU UAACAAGCCT | 16 |
| | Antisense | ATa610 | (5p)mA*G*mGfCmUfUmGmU fUmAmAfGmCfUmAfUmUm GfGmGT*mA*mG | AGGCUUGUUAA GCUAUUGGGTA G | 17 |
| ATsi611 | sense | ATs483 | mC*fC*mAmA*rU*mA*rG*m CfUfUmAmA*rC*mA*rA*mG mCfCmU*T*T-GA3 | CCAAUAGCUUA ACAAGCCUTT | 18 |
| | Antisense | ATa609 | (5p)mA*G*mGfCmUfUmGmU fUmAmAfGmCfUmAfUmUm GfG*mU*mU | AGGCUUGUUAA GCUAUUGGUU | 15 |
| ATsi612 | sense | ATs612 or ATs603 | mU*fG*mCmAfAmGfAmCfUf UmGmAfCmAfCmCmGfAmA *T*T-GA3 | UGCAAGACUUG ACACCGAATT | 8 |
| | Antisense | ATa612 or ATa787 | (5p)mU*T*mCfGmGfUmGmU fCmAmAfGmUfCmUfUmGm CfA*mG*mC | UTCGGUGUCAA GUCUUGCAGC | 19 |
| ATsi613 | sense | ATs613 | mA*fG*mCmUfCmAfAmCfAf AmGmCfCmUfGmAmGfGmU *T*T-GA3 | AGCUCAACAAG CCUGAGGUTT | 20 |
| | Antisense | ATa613 | (5p)mA*C*mCfUmCfAmGmG fCmUmUfGmUfUmGfAmGm CfU*mG*mU | ACCUCAGGCUU GUUGAGCUGU | 21 |

**Table 2A. Reference siRNA**

| siRNA Name | Sense or Antisense | Strand Name | Sequence + Chemistry (5' to 3') | Sequence | SEQ ID NO: |
|---|---|---|---|---|---|
| ATXL-G | sense | ATXL-Gs | mG*mU*mCmAmU mCfCmAfCfAfAmUmGmAm GmAmGmUmAmCmA- GalNAc | GUCAUCCACAA UGAGAGUACA | 22 |
| | Antisense | ATXL-Ga | mU*fG*mUmAmC(gnaT)mCm UmCmAmUmUmGfUmGfGm AmUmGmAmC*mG*mA | UGUACUCUCAU UGUGGAUGACG A | 23 |

### In Vitro Screening of siRNAs Targeting Human AGT mRNA in Cells

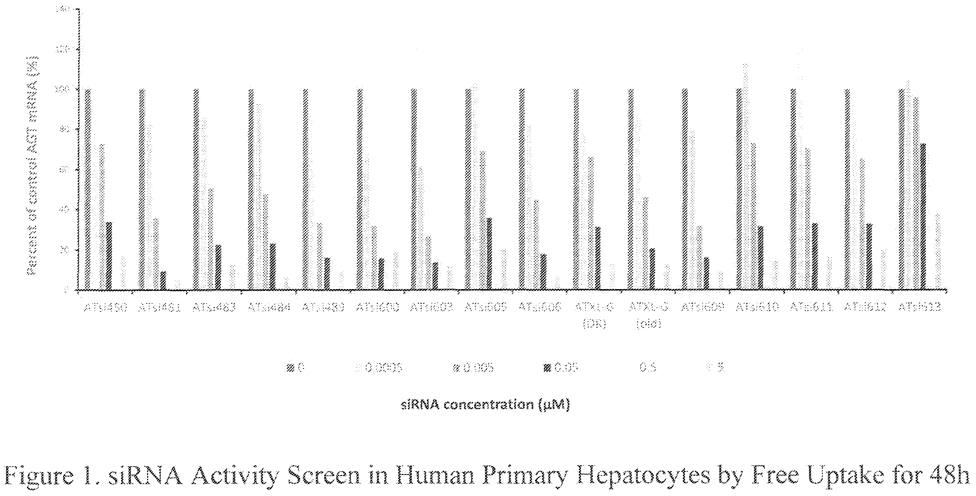

The siRNAs were incubated with human primary hepatocytes (HPH) and taken up by the cells through free uptake, i.e., siRNAs were incubated with cells in the absence of transfection reagents and enter cells through endocytosis via GalNAc conjugate and ASGR receptor interactions. Cells were incubated at different final concentrations for 41-48 hr and total RNA prepared using AcroPrep Advance 96-well Filter Plates (Pall Corporation, Port Washington, New York) and Qiagen's RLT, RW1 and RPE buffers. AGT mRNA levels were determined through qRT-PCR using AGT specific primer probe sets as listed in Table 3. qRT-PCR was performed using AgPath-ID^{™} One-Step RT-PCR Reagents in QS3 real-time PCR system (ThermoFisher Scientific, Waltham, MA, USA). The AGT target RNA levels detected in qRT-PCR assay were normalized to total RNA levels measured with RiboGreen^{™} (ThermoFisher Scientific, Waltham, MA, USA). The results are shown in Figure 1. The IC50s are calculated and shown in Table 4.

**Table 3: Sequences of Primer Probe Set for human AGT**

| Name | Sequence | SEQ ID NO: |
|---|---|---|
| hsAGT-F | CTGATCCAGCCTCACTATGC | 24 |
| hsAGT-R | AGGTCATAAGATCCTTGCAGC | 25 |
| hsAGT-P | AGGGTCTCACTTTCCAGCAAAACTCC | 26 |

**Table 4: AGT siRNA Inhibition in HPH Cells after 48hrs**

| AGT siRNAs | IC50 (uM) |
|---|---|
| ATsi603 | 0.0009 |
| ATsi600^{#} | 0.0015 |
| ATsi609 | 0.0025 |
| ATsi481 | 0.0027 |
| ATsi489 | 0.0031 |
| ATsi606 | 0.0042 |
| ATXL-G (old)* | 0.0058 |
| ATsi484^{#} | 0.0071 |
| ATsi483^{#} | 0.0074 |
| ATXL-G (DR)* | 0.0136 |
| ATsi480 | 0.0213 |
| ATsi610 | 0.0254 |
| ATsi611 | 0.0255 |
| ATsi612 | 0.0269 |
| ATsi605 | 0.032 |
| ATsi613 | 1.672 |

| | |
|---|---|
| *ATXL-G was synthesized in 2 batches: "old" and "DR" ^{#}The sequence and chemistry of these siRNAs are listed in Table 7 | |

The results indicated that some ARNATAR designed siRNAs appeared to be more potent than reference siRNA ATXL-G targeting AGT.

### EXAMPLE 2: IN VITRO COMPARISON OF ARNATAR MODIFICATIONS WITH ESC/ESC+ MODIFICATIONS FOR DIFFERENT siRNA SEQUENCES

The above results suggest that ARNATAR chemical modification motifs produced potent AGT siRNAs. To further determine the effects of different siRNA modification motifs, siRNAs were designed targeting 5 different regions of human AGT. For comparison, the siRNAs were designed with ARNATAR motifs, or with a third party's structure and modification design (ESC or ESC+ as described in Hu et al., Therapeutic siRNA: State of the Art, Signal Transduction and Targeted Therapy, 2020, 5:101; Zilebesiran uses the ESC+ modification motif). Since the ESC/ESC+ motif differed in strand length from ARNATAR's motif, the AGT siRNA antisense sequences were matched from the 5' end, to keep a consistent seed sequence. In addition, the siRNAs were made without GalNAc conjugates in order to only compare the chemical modification motifs. The ARNATAR designed siRNAs have a 5'-phosphate on the antisense strand. The AGT siRNA sequences and chemistry are listed in Table 5. The third party's design is represented by letters "AL" or "AL1".

**Table 5: Oligomeric Compounds Targeting Different Regions of AGT mRNA.**

| siRNA Name | Strand Name | Sense or Antisense | Sequence + Chemistry (5' to 3') | Sequence | SEQ ID NO: |
|---|---|---|---|---|---|
| ATsi-365m | ATs365m | sense | mU*fA*mGmUrC*mGrC*mUfGfC mAmArA*mArC*mUmUfGmA*T* T | UAGUCGCUGCAA AACUUGATT | 27 |
| | ATa365m | Antisense | (5p)mU*C*mAfAmGfUT*mUfUm GmCfAmGfCmGfAmCmUA*mG* mC | UCAAGUTUUGCA GCGACUAGC | 28 |
| 643-AN | ATsi-643s1 | sense | mU*fA*mGmUfCmGfCmUfGfCm GmAfAmAfCmUmUfGmA*T*T | UAGUCGCUGCGA AACUUGATT | 29 |
| | ATa643 | Antisense | (5p)mU*C*mAfAmGfUmUmUfCm GmCfAmGfCmGfAmCmUfA*mG* mC | UCAAGUUUCGCA GCGACUAGC | 30 |
| 643-AL | 643-Als | sense | mG*mC*mUmAmGmUfCmGfCfUf GmCmGmAmAmAmCmUmUmGm A | GCUAGUCGCUGC GAAACUUGA | 31 |
| | 643-Ala | Antisense | mU*fC*mAmAmGfUgnaTfUfCmG mCmAmGfCmGfAmCmUmAmGm C*mA*mC | UCAAGUUUCGCA GCGACUAGCAC | 32 |
| ATsi565 | ATs565 | sense | mU*fG*mCmAfAmGfAmCfUfUm GmAfCmAfCmCmGfAmA*T*T | UGCAAGACUUGA CACCGAATT | 33 |
| | ATa565, ATa603 or ATa786 | Antisense | (5p)mU*T*mCfGmGfUG*mUfCm AmAfGmUfCmUfUmGmCA*mG* mC | UTCGGUGUCAAG UCUUGCAGC | 9 |
| 612-AN | ATs565 | sense | mU*fG*mCmAfAmGfAmCfUfUm GmAfCmAfCmCmGfAmA*T*T | UGCAAGACUUGA CACCGAATT | 33 |
| | ATa612 or ATa787 | Antisense | (5p)mU*T*mCfGmGfUmGmUfCm AmAfGmUfCmUfUmGmCfA*mG* mC | UTCGGUGUCAAG UCUUGCAGC | 19 |
| 612-AL | 612-Als | sense | mG*mC*mUmGmCmAfAmGfAfCf UmUmGmAmCmAmCmCmGmAm A | GCUGCAAGACUU GACACCGAA | 34 |
| | 612-Ala⁺⁺ | Antisense | mU*fU*mCmGmGgnaTmGfUfCm AmAmGmUfCmUfUmGmCmAmG mC*mG*mA | UUCGGUGUCAAG UCUUGCAGCGA | 35 |
| ATsi579 | ATs579 | sense | mG*fA*mCmCfCmUfAmCfCfUm UmCfAmUfAmCmCfUmG*T*T | GACCCUACCUUC AUACCUGTT | 36 |
| | ATa579 | Antisense | (5p)mC*A*mGfGmUfAT*mGfAm AmGfGmUfAmGfGmGmUC*mU* mU | CAGGUATGAAGG UAGGGUCUU | 37 |
| 579-AL1 | 579s-AL1 | sense | mA*mA*mGmAmCmCfCmUfAfCf CmUmUmCmAmUmAmCmCmUm G | AAGACCCUACCU UCAUACCUG | 38 |
| | 579a-AL | Antisense | mC*fA*mGmGmUfAgnaTfGfAmA mGmGmUfAmGfGmGmUmCmUm U*mU*mG | CAGGUAUGAAGG UAGGGUCUUUG | 39 |
| ATsi597 | ATs597 | sense | mU*fU*mAmAfCmAfAmGfCfCm UmAfAmGfGmUmCfUmU*T*T | UUAACAAGCCUA AGGUCUUTT | 40 |
| | ATa597 | Antisense | (5p)mA*A*mGfAmCfCT*mUfAm GmGfCmUfUmGfUmUmAA*mG* mC | AAGACCTUAGGC UUGUUAAGC | 7 |
| 597-AL1 | 597s-AL1 | sense | mG*mC*mUmUmAmAfCmAfAfGf CmCmUmAmAmGmGmUmCmUm U | GCUUAACAAGCC UAAGGUCUU | 41 |
| | 597a-AL | Antisense | mA*fA*mGmAmCfCgnaTfUfAmG mGmCmUfUmGfUmUmAmAmGm C*mU*mG | AAGACCUUAGGC UUGUUAAGCUG | 42 |
| ATsi598 | ATs598 | sense | mC*fC*mAmAfUmAfGmCfUfUm AmAfCmAfAmGmCfCmU*T*T | CCAAUAGCUUAA CAAGCCUTT | 43 |
| | ATa598 or ATa484 | Antisense | (5p)mA*G*mGfCmUfUG*mUfUm AmAfGmCfUmAfUmUmGG*mG* mU | AGGCUUGUUAAG CUAUUGGU | 44 |
| 598-AL1 | 598s-AL1 | sense | mA*mC*mCmCmAmAfUmAfGfCf UmUmAmAmCmAmAmGmCmCm U | ACCCAAUAGCUU AACAAGCCU | 45 |
| | 598a-AL⁺⁺⁺ | Antisense | mA*fG*mGmCmUfUmGfUfUmAm AmGmCfUmAfUmUmGmGmGmU *mA*mG | AGGCUUGUUAAG CUAUUGGGUAG | 46 |

| | | | | | |
|---|---|---|---|---|---|
| ⁺⁺Since gnaG was not available, 612-Ala differs from the normal ESC+ chemistry in placement of the gna modification, however, gna at position 6 has also been widely used in drug discovery (see PCT/US2019/032150). ⁺⁺⁺Since gnaG is not available, 598a-AL does not contain gna at position 7, similar to the ESC chemistry. | | | | | |

### In Vitro Assays - Comparison of ARNATAR Motifs to Third Party Motifs

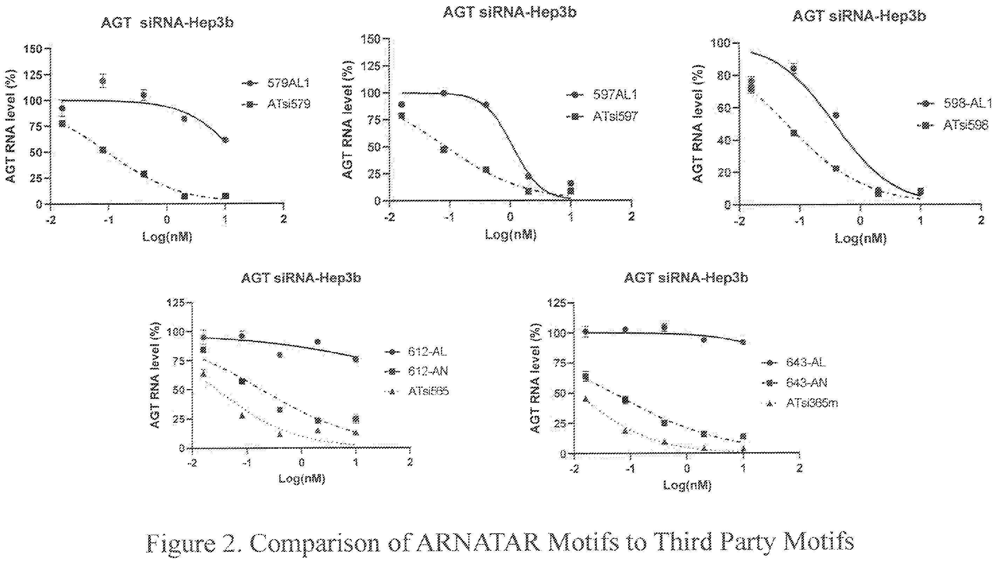

AGT siRNAs without GalNAc conjugate were transfected at 0-10 nM into Hep3B cells with RNAiMAX (Invitrogen, Waltham, MA) and the cells further cultured for 24 hr. siRNA activity was determined by qRT-PCR using the primer probe sets as listed in Table 3. The IC50 of the siRNAs targeting AGT are shown in Table 6 and the dose response curve of siRNA on AGT mRNA levels in Hep3B cells are shown in Figure 2.

**Table 6: siRNA Activity Targeting AGT in Hep3B cells**

| siRNAs | IC50 (nM) |
|---|---|
| 579AL | 15.06 |
| ATsi-579 | 0.0947 |
| 597AL | 1.105 |
| ATsi-597 | 0.087 |
| 598-AL | 0.3776 |
| ATsi-598 | 0.0604 |
| 612-AL | 1233 |
| 612-AN | 0.1821 |
| ATsi-565 | 0.0284 |
| 643-AL | 1172.3 |
| ATsi-365m | 0.04887 |
| 643-AN | 0.01122 |

The results indicate that for all these different sequences, the optimized ARNATAR designs are more potent than the third-party design (P<0.0001), indicating that the optimized ARNATAR motifs are generalizable and the increased activity applies to different sequences.

### EXAMPLE 3: ARNATAR AGT siRNAS SHOWED BETTER ACTIVITY AND LONGER DURATION COMPARED WITH THE REFERENCE siRNA IN ANIMALS

To compare the activity and duration of siRNAs *in vitro* and *in vivo* with reference siRNA ATXL-G, selected ARNATAR siRNAs with different chemistry were designed and synthesized, using GalNAc3 (Table 7).

**Table 7: AGT Oligomeric Compounds with Modifications to Both Strands**

| siRNA Name | Strand Name | Sense or Antisense | Sequence + Chemistry (5' to 3') | Sequence | SEQ ID NO: |
|---|---|---|---|---|---|
| ATsi483 | sense | ATs483 | mC*fC*mAmA*rU*mA*rG* mCfUfUmAmA*rC*mA*rA* mGmCfCmU*T*T-GA3 | CCAAUAGCUU AACAAGCCUTT | 18 |
| | Antisense | ATa483 | (5p)mA*G*mGfCmUfUmGm UfUmAmAfGmCfUmAfUmU mGfG*mG*mU | AGGCUUGUUA AGCUAUUGGG U | 47 |
| ATsi484 | sense | ATs484 or ATs600 | mC*fC*mAmAfUmAfGmCf UfUmAmAfCmAfAmGmCfC mU*T*T-GA3 | CCAAUAGCUU AACAAGCCUTT | 14 |
| | Antisense | ATa484 or ATa598 | (5p)mA*G*mGfCmUfUG*m UfUmAmAfGmCfUmAfUmU mGG*mG*mU | AGGCUUGUUA AGCUAUUGGG U | 44 |
| ATsi600 | sense | ATs484 or ATs600 | mC*fC*mAmAfUmAfGmCf UfUmAmAfCmAfAmGmCfC mU*T*T-GA3 | CCAAUAGCUU AACAAGCCUTT | 14 |
| | Antisense | ATa483 | (5p)mA*G*mGfCmUfUmGm UfUmAmAfGmCfUmAfUmU mGfG*mG*mU | AGGCUUGUUA AGCUAUUGGG U | 47 |

### In Vitro Assay

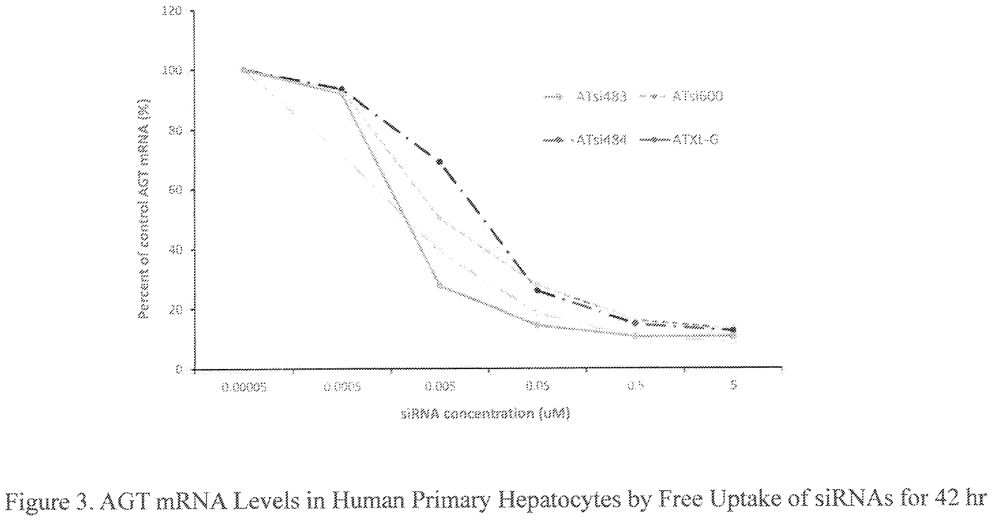

siRNAs were delivered at different final concentrations to human primary hepatocytes (HPH) through free uptake, i.e., siRNAs are incubated with cells in the absence of transfection reagents and enter cells through endocytosis via GalNAc conjugate and ASGR receptor interactions. Cells were incubated for 42 hr and total RNA prepared using AcroPrep Advance 96-well Filter Plates (Pall Corporation, Port Washington, New York) and Qiagen's RLT, RW1 and RPE buffers. AGT mRNA levels were determined through qRT-PCR using AGT specific primer probe sets as listed in Table 3. qRT-PCR was performed using AgPath-ID^{™} One-Step RT-PCR Reagents in QS3 real-time PCR system (ThermoFisher Scientific, Waltham, MA, USA). The AGT RNA levels detected in qRT-PCR assay were normalized to total RNA levels as measured with RiboGreen^{™} (ThermoFisher Scientific, Waltham, MA, USA). The results are shown in Figure 3. The IC50s are calculated and shown in Table 8.

**Table 8. IC50 of siRNA inhibition of AGT mRNA in HPH**

| | ATsi483 | ATsi600 | ATsi484 | ATXL-G |
|---|---|---|---|---|
| IC50 (uM) | 0.00258 | 0.00956 | 0.01543 | 0.00259 |

The results showed that ARNATAR siRNA compounds, such as ATsi483, showed comparable activity with reference siRNA ATXL-G.

### In Vivo Study 1

To assess human AGT siRNA activity *in vivo,* a mouse model expressing human AGT was made. Seven week old BALB/c mice were injected intravenously with adeno-associated virus (AAV) serotype AAV8 expressing human AGT mRNA (GenBank NM_001382817.3; Sands MS, AAV-Mediated Liver-Directed Gene Therapy, 2014, Methods Mol Biol, 807:141-157). At least 3 weeks later after virus administration, blood was collected from mice using lithium heparin as an anticoagulant, and plasma prepared by centrifugation of the blood samples at 2000×g for 10 min at 4°C. Plasma AGT protein levels were then determined using an ELISA kit specific to human AGT (IBL, Minneapolis, MN. Catalog. No 27412). The AGT levels of individual animals at this time point were used as base lines of the AGT (protein) expression.

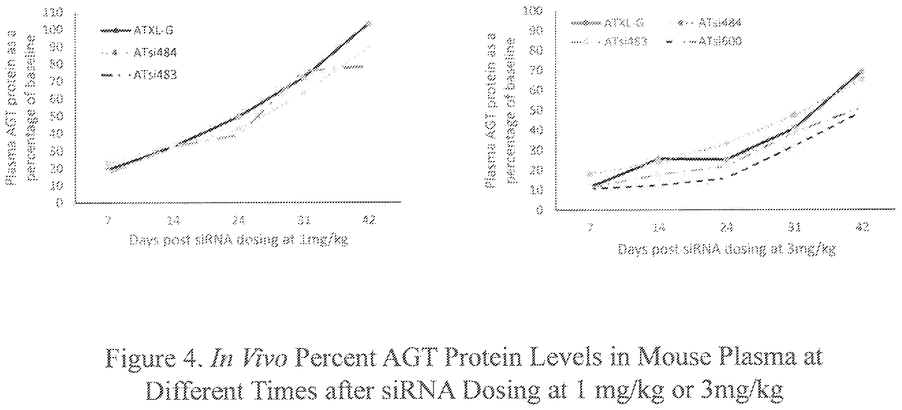

To determine siRNA activity *in vivo,* the next day after baseline blood collection, siRNAs were dosed subcutaneously at 1 mg/kg (ATsi483, ATsi484 and ATXL-G) or 3 mg/kg (ATsi483, ATsi484, ATsi600 and ATXL-G) in the AGT transgenic mice (N=4-6 for each siRNA dose cohort). Blood samples were then collected at indicated times after siRNA dosing, and AGT protein levels in plasma were measured by ELISA. Average AGT protein levels relative to baseline levels (measured at Day -1 before dosing) are shown in Figure 4.

The results showed that ARNATAR siRNAs showed comparable (ATsi483 and ATsi484) or better (ATsi600) activity *in vivo* compared with reference siRNA ATXL-G, with similar duration in the over 6-weeks time frame tested. Interestingly, several siRNAs, e.g., ATsi484 and ATsi600, appear slightly less active than the reference siRNA in cell culture by free uptake, however, similar or better activity than the reference siRNA was observed in animals. These results suggest that some difference exists in activity between cell culture and *in vivo* systems.

### EXAMPLE 4. EVALUATION OF DIFFERENT SEQUENCES AND MODIFICATIONS IN VIVO

To further evaluate the effects of sequences and chemical motifs on AGT siRNA activity, new ARNATAR siRNAs were synthesized that contain either different sequences, different modification motifs, or different lengths. The siRNAs were conjugated with GalNAc3 (GA3 from Gene Link, Elmsford, NY, USA). The siRNAs are listed in Table 9.

**Table 9. AGT Oligomeric Compounds with Modifications to Both Strands**

| siRNA Name | Sense or Antisense | Strand Name | Sequence + Chemistry (5' to 3') | Sequence | SEQ ID NO: |
|---|---|---|---|---|---|
| ATsi610 | sense | ATs610 | mA*fC*mCmCfAmAfUmAfGf CmUmUfAmAfCmAmAfGmC *C*T-GA3 | ACCCAAUAGCU UAACAAGCCT | 16 |
| | Antisense | ATa610 | (5p)mA*G*mGfCmUfUmGmU fUmAmAfGmCfUmAfUmUm GfGmGT*mA*mG | AGGCUUGUUA AGCUAUUGGGT AG | 17 |
| ATsi643 | sense | ATs643 | mU*fA*mGmUfCmGfCmUfGf CmGmAfAmAfCmUmUfGmA *T*T-GA3 | UAGUCGCUGCG AAACUUGATT | 48 |
| | Antisense | ATa643 | (5p)mU*C*mAfAmGfUmUmU fCmGmCfAmGfCmGfAmCm UfA*mG*mC | UCAAGUUUCGC AGCGACUAGC | 30 |
| ATsi644 | sense | ATs644 | mG*fC*mUmUfAmAfCmAfAf GmCmCfUmAfAmGmGfUmC *mU*mU-GA3 | GCUUAACAAGC CUAAGGUCUU | 49 |
| | Antisense | ATa644 | (5p)mA*A*mGfAmCfCmUmU fAmGmGfCmUfUmGfUmUm AfAmGmC*mU*mG | AAGACCUUAG GCUUGUUAAG CUG | 50 |
| ATsi648 | sense | ATs648 | mG*fA*mCmCfCmUfAmCfCf UmUmCfAmUfAmCmCfUmG *T*T-GA3 | GACCCUACCUU CAUACCUGTT | 51 |
| | Antisense | ATa648 | (5p)mC*A*mGfGmUfAmUmG fAmAmGfGmUfAmGfGmGm UfC*mU*mU | CAGGUAUGAA GGUAGGGUCU U | 52 |

To produce a mouse model expressing human AGT, 7 weeks BALB/c mice were injected intravenously with adeno-associated virus (AAV) serotype AAV8 expressing human AGT mRNA (GenBank NM_001382817.3; Sands MS, AAV-Mediated Liver-Directed Gene Therapy, 2014, Methods Mol Biol, 807:141-157). At least 3 weeks later after virus administration, blood was collected from mice, and plasma prepared by centrifugation of the blood samples at 2000×g for 10 min at 4°C. Plasma AGT protein levels were then determined using an ELISA kit specific to human AGT (IBL, Minneapolis, MN. Catalog. No 27412). The AGT levels of individual animals at this time point were used as base lines of the AGT (protein) expression.

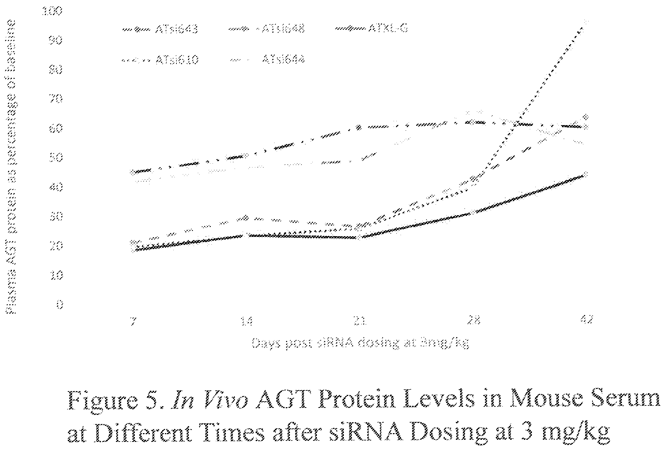

To determine siRNA activity *in vivo,* the next day after baseline blood collection, siRNAs were dosed subcutaneously at 3 mg/kg (N=3-4). Blood samples were then collected at indicated times after siRNA dosing, and AGT protein levels in plasma were measured by ELISA. Average AGT protein levels relative to baseline levels (measured at Day -1 before dosing) are shown in Figure 5. In the previous experiment (Example 3, Figure 4) for the reference compound ATXL-G at the last time point (31 days), the AGT level was ~40%, similar here to ATsi610 and ATsi643. The results showed that certain siRNA compounds, such as ATsi643 and ATsi610, are comparable to the reference siRNA in terms of both activity and duration.

### EXAMPLE 5: EVALUATION OF ADDITIONAL SEQUENCES AND MODIFICATIONS IN VITRO AND IN VIVO

To further evaluate the effects of sequences and chemical motifs on siRNA activity, new ARNATAR siRNAs were synthesized that contain either different sequences, different modification motifs, different lengths or with natural or TT overhangs. For example, the sequence for ATsi603 and ATsi612 contains a mismatched mutation to SEQ ID NO: 1. The siRNAs were conjugated with GalNAc3 (GA3 from Gene Link, Elmsford, NY, USA). The siRNAs are listed in Table 10.

**Table 10: AGT Oligomeric Compounds with Modifications to Both Strands**

| siRNA Name | Sense or Antisense | Strand Name | Sequence + Chemistry (5' to 3') | Sequence | SEQ ID NO: |
|---|---|---|---|---|---|
| ATsi603 | sense | ATs603 or ATs612 | mU*fG*mCmAfAmGfAm CfUfUmGmAfCmAfCmC mGfAmA*T*T-GA3 | UGCAAGACUUG ACACCGAATT | 8 |
| | Antisense | ATa603, ATa565 or ATa786 | (5p)mU*T*mCfGmGfUG* mUfCmAmAfGmUfCmUf UmGmCA*mG*mC | UTCGGUGUCAA GUCUUGCAGC | 9 |
| ATsi612 | sense | ATs612 or ATs603 | mU*fG*mCmAfAmGfAm CfUfUmGmAfCmAfCmC mGfAmA*T*T-GA3 | UGCAAGACUUG ACACCGAATT | 8 |
| | Antisense | ATa612 or ATa787 | (5p)mU*T*mCfGmGfUmG mUfCmAmAfGmUfCmUf UmGmCfA*mG*mC | UTCGGUGUCAA GUCUUGCAGC | 19 |
| ATsi633 | sense | ATs633 | mA*fC*mCmCfAmAfUm AfGfCmUmUfAmAfCmA mAfGmCmCfU*T*T-GA3 | ACCCAAUAGCU UAACAAGCCUT T | 53 |
| | Antisense | ATa633 | (5p)mA*G*mGfCmUfUmG mUfUmAmAfGmCfUmAf UmUmGfGmGmU*T*T | AGGCUUGUUAA GCUAUUGGGUT T | 54 |
| ATsi665 | sense | ATs633 | mA*fC*mCmCfAmAfUm AfGfCmUmUfAmAfCmA mAfGmCmCfU*T*T-GA3 | ACCCAAUAGCU UAACAAGCCUT T | 53 |
| | Antisense | ATa610 | (5p)mA*G*mGfCmUfUmG mUfUmAmAfGmCfUmAf UmUmGfGmGT*mA*mG | AGGCUUGUUAA GCUAUUGGGTA G | 17 |

### In Vitro Assay

For a better comparison, two siRNAs (ATsi600 and ATsi610) evaluated above were also included in this assessment of additional ARNATAR siRNAs.

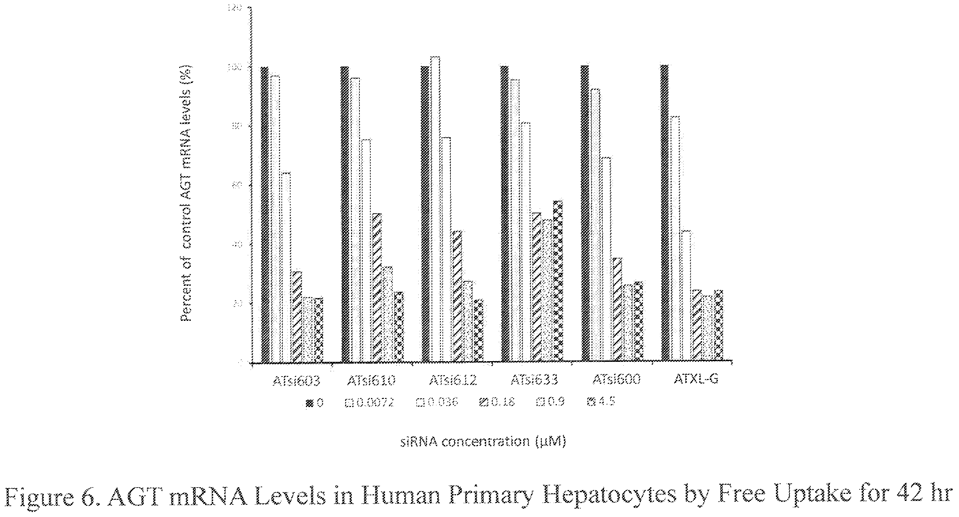

siRNAs were delivered at different final concentrations to human primary hepatocytes (HPH) through free uptake, i.e., siRNAs are incubated with cells in the absence of transfection reagents and enter cells through endocytosis via GalNAc conjugate and ASGR receptor interactions. Cells were incubated for 42 hr and total RNA prepared using AcroPrep Advance 96-well Filter Plates (Pall Corporation, Port Washington, New York) and Qiagen's RLT, RW1 and RPE buffers. AGT mRNA levels were determined through qRT-PCR using AGT specific primer probe sets as listed in Table 3. qRT-PCR was performed using AgPath-ID^{™} One-Step RT-PCR Reagents in QS3 real-time PCR system (ThermoFisher Scientific, Waltham, MA, USA). The AGT RNA levels detected in qRT-PCR assay were normalized to total RNA levels as measured with RiboGreen^{™} (ThermoFisher Scientific, Waltham, MA, USA). The results are shown in Figure 6. The IC50s are calculated and shown in Table 11.

**Table 11: IC50 of siRNA Inhibition of AGT mRNA in Human Primary Hepatocytes**

| | ATsi603 | ATsi610 | ATsi612 | ATsi633 | ATsi600 | ATXL-G |
|---|---|---|---|---|---|---|
| IC50 (uM) | 0.101 | 0.267 | 0.201 | 1.617 | 0.137 | 0.043 |

### In Vivo Study

To produce a mouse model expressing human AGT, 7 weeks BALB/c mice were injected intravenously with adeno-associated virus (AAV) serotype AAV8 expressing human AGT mRNA (GenBank NM_001382817.3; Sands MS, AAV-Mediated Liver-Directed Gene Therapy, 2014, Methods Mol Biol, 807:141-157). At least 3 weeks later after virus administration, blood was collected from mice, and plasma prepared by centrifugation of the blood samples at 2000×g for 10 min at 4°C. Plasma AGT protein levels were then determined using an ELISA kit specific to human AGT (IBL, Minneapolis, MN. Catalog. No 27412). The AGT levels of individual animals at this time point were used as baselines of the AGT (protein) expression.

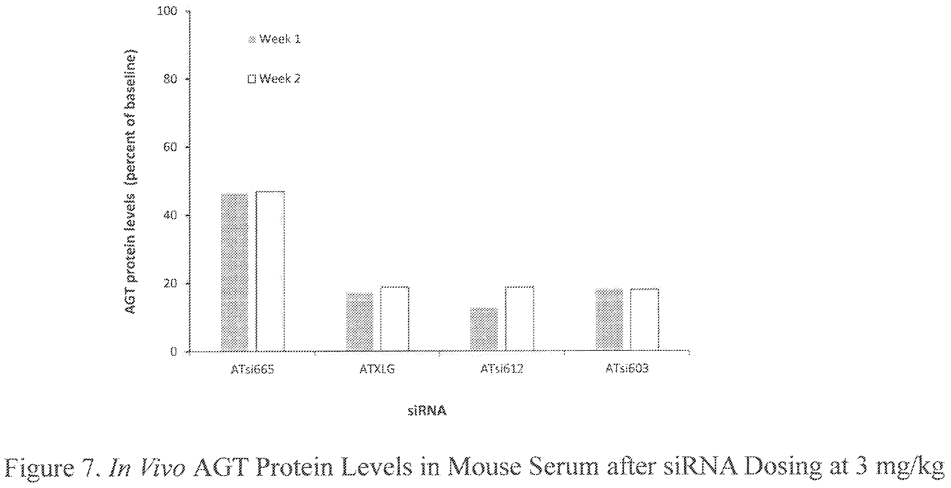

To determine siRNA activity *in vivo,* the next day after baseline blood collection, selected siRNAs ATsi603, ATsi612, ATsi665 and benchmark siRNA ATXL-G were dosed subcutaneously at 3 mg/kg (N=2-4). Blood samples were then collected at indicated times after siRNA dosing, plasma prepared, and AGT protein levels in plasma were measured by ELISA. Average AGT protein levels relative to baseline levels (measured at Day -1 before dosing) are shown in Figure 7 and Table 12.

**Table 12. Plasma AGT protein level in percentage relative to base line AGT protein level**

| | ATsi665 | ATXL-G | ATsi612 | ATsi603 |
|---|---|---|---|---|
| Week 1 | 46.38 | 17.03 | 12.77 | 18.00 |
| Week 2 | 46.8 | 18.7 | 18.6 | 17.9 |

The results indicate that, again, although certain siRNAs, such as ATsi612, showed slightly lower activity *in vitro,* however, *in vivo,* this siRNA compound showed slightly better or comparable to the reference siRNA in terms of both activity and duration.

### EXAMPLE 6: EVALUATION OF SELECTED OLIGOMERIC COMPOUNDS IN VITRO AND IN VIVO

To further evaluate the effects of GalNAc conjugates on siRNA activity, new ARNATAR siRNAs were designed that contain the sequence and chemical motif of ATsi603 or ATsi612 (see Table 10), but, were conjugated to a different GalNAc-comprising moiety (GalNAc-AN also known as GA-AN or GA4, see *supra* for a decription of GA4). The new siRNAs are listed in Table 13.

**Table 13: AGT Oligomeric Compounds with Modifications to Both Strands**

| siRNA Name | Sense or Antisense | Strand Name | Sequence + Chemistry (5' to 3') | Sequence | SEQ ID NO: |
|---|---|---|---|---|---|
| ATsi786 | Sense | ATs786 or ATs787 | mU*fG*mCmAfAmGfAm CfUfUmGmAfCmAfCmC mGfAmA*T*T-GA4 | UGCAAGACUUG ACACCGAATT | 55 |
| | Antisense | ATa786, ATa565 or ATa603 | (5p)mU*T*mCfGmGfUG* mUfCmAmAfGmUfCmUf UmGmCA*mG*mC | UTCGGUGUCAA GUCUUGCAGC | 9 |
| ATsi787 | Sense | ATs787 or ATs786 | mU*fG*mCmAfAmGfAm CfUfUmGmAfCmAfCmC mGfAmA*T*T-GA4 | UGCAAGACUUG ACACCGAATT | 55 |
| | Antisense | ATa787 or ATa612 | (5p)mU*T*mCfGmGfUmG mUfCmAmAfGmUfCmUf UmGmCfA*mG*mC | UTCGGUGUCAA GUCUUGCAGC | 19 |

### In Vitro Assay: Time Course in Hep3B Cells

AGT targeting siRNAs ATsi603, ATsi786, ATsi787 and benchmark siRNA ATXL-G were transfected at 0 nM, 0.008 nM, 0.04 nM, 0.2 nM, 1 nM and 5 nM into Hep3B cells with RNAiMAX (Invitrogen, Waltham, MA) and the cells further cultured for 8 hr or 13 hr. siRNA activity was determined by qRT-PCR using the primer probe sets as listed in Table 3.

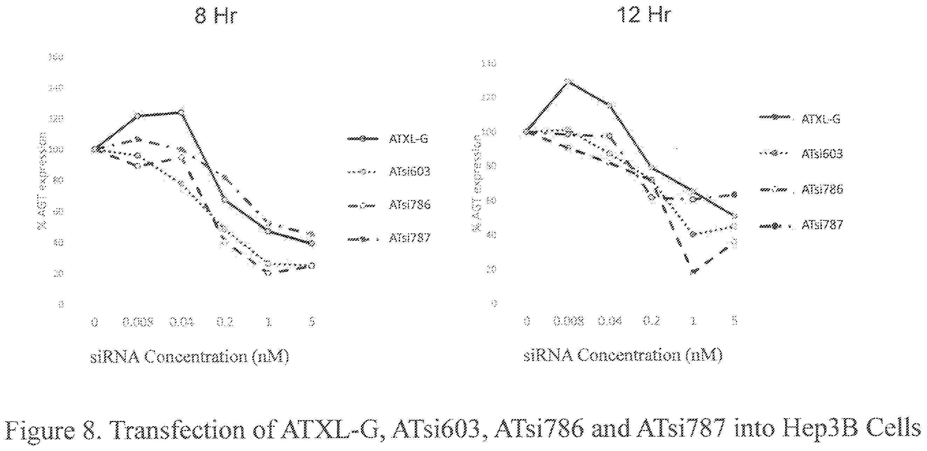

The AGT RNA levels detected in qRT-PCR assay were normalized to total RNA levels as measured with RiboGreen^{™} (ThermoFisher Scientific, Waltham, MA, USA). The percent of AGT mRNA levels are shown in Figure 8. ATsi786 and ATsi603 are more potent than ATXL-G, with P=0.0047 and 0.0033 at 8hrs; P=0.0019 and 0.0385 at 12hrs.

### In Vivo Assay

To produce a mouse model expressing human AGT, 7 weeks BALB/c mice were injected intravenously with adeno-associated virus (AAV) serotype AAV8 expressing human AGT mRNA (GenBank NM_001382817.3; Sands MS, AAV-Mediated Liver-Directed Gene Therapy, 2014, Methods Mol Biol, 807:141-157). At least 3 weeks later after virus administration, blood was collected from mice, and plasma prepared by centrifugation of the blood samples at 2000×g for 10 min at 4°C. Plasma AGT protein levels were then determined using an ELISA kit specific to human AGT (IBL, Minneapolis, MN. Catalog. No 27412). The AGT levels of individual animals at this time point were used as baselines of the AGT (protein) expression.

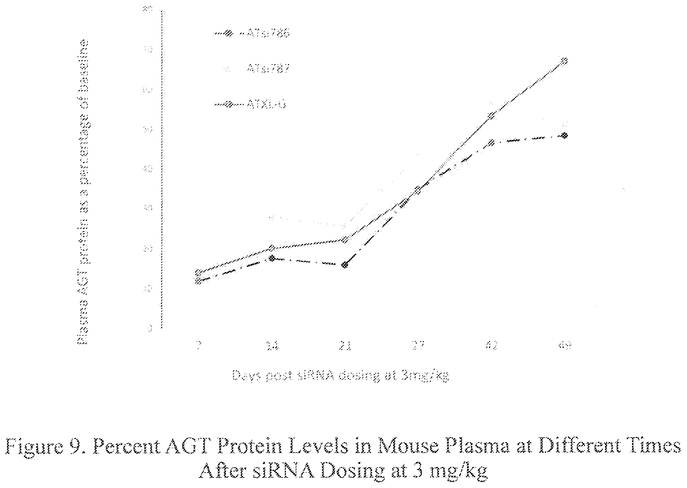

To determine siRNA activity *in vivo,* the next day after baseline blood collection, AGT targeting siRNAs ATsi786, ATsi787 and benchmark siRNA ATXL-G were dosed subcutaneously at 3 mg/kg (N=3-4). Blood samples were then collected weekly for up to 6 or 7 weeks after siRNA dosing, plasma prepared, and AGT protein levels in plasma were measured by ELISA. Average AGT protein levels relative to baseline levels (measured at Day -1 before dosing) are shown in Figure 9. As shown in Figure 9, ATsi786 was slightly more potent at several time points than benchmark ATXL-G in reducing AGT expression.

### EXAMPLE 7: EVALUATION OF SELECTED OLIGOMERIC COMPOUNDS IN AGT TRANSGENIC MICE

To assess the early kinetics of human AGT siRNA activity *in vivo,* a mouse model expressing human AGT was made. 7-8 week old BALB/c mice were injected intravenously with adeno-associated virus (AAV) serotype AAV8 expressing human AGT mRNA (GenBank NM_001382817.3; Sands MS, AAV-Mediated Liver-Directed Gene Therapy, 2014, Methods Mol Biol, 807:141-157) at 2.5 X 10¹¹ virus genome per animal. About 3 weeks after virus administration, blood was collected from mice using lithium heparin as an anticoagulant, and plasma prepared by centrifugation of the blood samples at 2000×g for 10 min at 4°C. Plasma AGT protein levels were then determined using an ELISA kit specific to human AGT (IBL, Minneapolis, MN. Catalog. No 27412). The AGT levels of individual animals at this time point were used as base lines of the AGT (protein) expression.

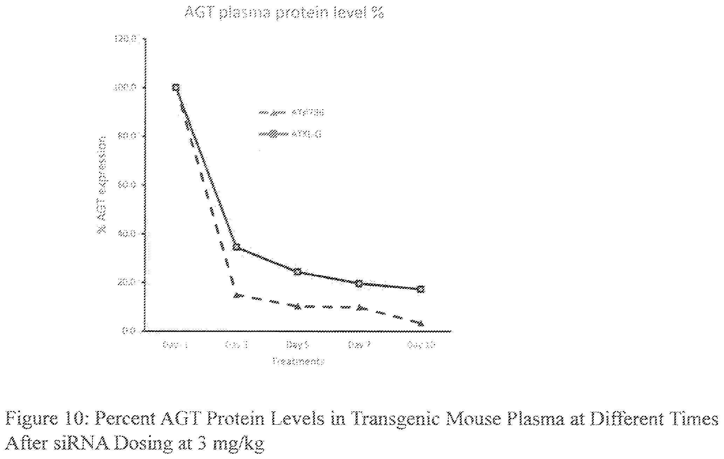

To determine siRNA activity *in vivo,* human AGT siRNA ATsi786 or benchmark ATXL-G was administrated at 3 mg/kg through subcutaneous (SC) injection on Day 0 (N=3 for each group). Approximately 50 µl blood was collected through cheek bleeding on day -1 (base line) and on day 3, 5, 7 and 10 into Microvette^{®} CB 300 Lithium heparin LH tubes (Sarstedtstr, Nümbrecht Germany) and kept on ice for over 20 mins. Plasma was prepared by centrifugation of the blood sample at 2000×g for 20 minutes at 4°C, using a 5425R centrifuge (Eppendorf, Hamburg, Germany), and supernatant was collected. Human AGT protein levels in plasma (1: 1000 dilution) from day -1 and day 10 were determined using Human Total Angiotensinogen Assay Kit (product number 27412; IBL-America, Minneapolis, MN, USA), based on the manufacturer's protocol. The relative AGT protein levels at different times were calculated in Excel as percentage of the AGT protein levels at day -1 of the individual animals. The average percentage in each group was calculated in Excel as shown in Table 14 and plotted as shown in Figure 10.

**Table 14: Mean Expression of AGT Protein Levels at Various Time Points**

| Treatment | Mean Expression % | | | | |
|---|---|---|---|---|---|
| | Day -1 | Day 3 | Day 5 | Day 7 | Day 10 |
| ATsi786 | 100.0 | 14.9 | 10.3 | 9.9 | 3.4 |
| ATXL-G | 100.0 | 34.4 | 24.2 | 19.6 | 17.3 |

The results show that the level of human AGT protein in plasma was quickly reduced upon ATsi786 siRNA administration in animals. ATsi786 reduced human AGT protein faster than benchmark ATXL-G (P=0.0201).

### SEQUENCES

**Table 15: Sequences**

| SEQ ID NO: | SEQUENCE | DESCRIPTION |
|---|---|---|
| 1 | AGAAAGTAGACCCTCACCAGGCATGGAATCTGCCAGTG CCTTGATCTTGGACTTCCAGCTTCCAGAACTGGTATGCG GAAGCGAGCACCCCAGTCTGAGATGGCTCCTGCCGGTG TGAGCCTGAGGGCCACCATCCTCTGCCTCCTGGCCTGG GCTGGCCTGGCTGCAGGTGACCGGGTGTACATACACCC CTTCCACCTCGTCATCCACAATGAGAGTACCTGTGAGC AGCTGGCAAAGGCCAATGCCGGGAAGCCCAAAGACCC CACCTTCATACCTGCTCCAATTCAGGCCAAGACATCCCC TGTGGATGAAAAGGCCCTACAGGACCAGCTGGTGCTAG TCGCTGCAAAACTTGACACCGAAGACAAGTTGAGGGCC GCAATGGTCGGGATGCTGGCCAACTTCTTGGGCTTCCGT ATATATGGCATGCACAGTGAGCTATGGGGCGTGGTCCA TGGGGCCACCGTCCTCTCCCCAACGGCTGTCTTTGGCAC CCTGGCCTCTCTCTATCTGGGAGCCTTGGACCACACAGC TGACAGGCTACAGGCAATCCTGGGTGTTCCTTGGAAGG ACAAGAACTGCACCTCCCGGCTGGATGCGCACAAGGTC CTGTCTGCCCTGCAGGCTGTACAGGGCCTGCTAGTGGC CCAGGGCAGGGCTGATAGCCAGGCCCAGCTGCTGCTGT CCACGGTGGTGGGCGTGTTCACAGCCCCAGGCCTGCAC CTGAAGCAGCCGTTTGTGCAGGGCCTGGCTCTCTATACC CCTGTGGTCCTCCCACGCTCTCTGGACTTCACAGAACTG GATGTTGCTGCTGAGAAGATTGACAGGTTCATGCAGGC TGTGACAGGATGGAAGACTGGCTGCTCCCTGATGGGAG CCAGTGTGGACAGCACCCTGGCTTTCAACACCTACGTC CACTTCCAAGGGAAGATGAAGGGCTTCTCCCTGCTGGC CGAGCCCCAGGAGTTCTGGGTGGACAACAGCACCTCAG TGTCTGTTCCCATGCTCTCTGGCATGGGCACCTTCCAGC ACTGGAGTGACATCCAGGACAACTTCTCGGTGACTCAA GTGCCCTTCACTGAGAGCGCCTGCCTGCTGCTGATCCAG CCTCACTATGCCTCTGACCTGGACAAGGTGGAGGGTCT CACTTTCCAGCAAAACTCCCTCAACTGGATGAAGAAAC TATCTCCCCGGACCATCCACCTGACCATGCCCCAACTGG TGCTGCAAGGATCTTATGACCTGCAGGACCTGCTCGCC CAGGCTGAGCTGCCCGCCATTCTGCACACCGAGCTGAA CCTGCAAAAATTGAGCAATGACCGCATCAGGGTGGGGG AGGTGCTGAACAGCATTTTTTTTGAGCTTGAAGCGGAT GAGAGAGAGCCCACAGAGTCTACCCAACAGCTTAACAA GCCTGAGGTCTTGGAGGTGACCCTGAACCGCCCATTCC TGTTTGCTGTGTATGATCAAAGCGCCACTGCCCTGCACT TCCTGGGCCGCGTGGCCAACCCGCTGAGCACAGCATGA GGCCAGGGCCCCAGAACACAGTGCCTGGCAAGGCCTCT GCCCCTGGCCTTTGAGGCAAAGGCCAGCAGCAGATAAC AACCCCGGACAAATCAGCGATGTGTCACCCCCAGTCTC CCACCTTTTCTTCTAATGAGTCGACTTTGAGCTGGAAAG | AGT sequence GenBank |
| | | NM_001382817.3 |
| | CAGCCGTTTCTCCTTGGTCTAAGTGTGCTGCATGGAGTG AGCAGTAGAAGCCTGCAGCGGCACAAATGCACCTCCCA GTTTGCTGGGTTTATTTAGAGAATGGGGGTGGGAGG CAAGAACCAGTGTTTAGCGCGGACTACTGTTCCAAAA AGAATTCCAACCGACCAGCTTGTTTGTGAAACAAAAAA GTGTTCCCTTTTCAAGTTGAGAACAAAAATTGGGTTTTA AAATTAAAGTATACATTTTTGCATTGCCTTCGGTTTGTA TTTAGTGTCTTGAATGTAAGAACATGACCTCCGTGTAGT GTCTGTAATACCTTAGTTTTTCCACAGATGCTTGTGAT TTTTGAACAATACGTGAAAGATGCAAGCACCTGAATTT CTGTTTGAATGCGGAACCATAGCTGGTTATTTCTCCCTT GTGTTAGTAATAAACGTCTTGCCACAATAAGCCTCCAA AAA | |
| 56 | CCAACAGCUUACCAAGCCUTT | Sense strand ATs480 |
| 57 | AGGCUUGGUAAGCUGUUGGGU | Antisense strand ATa480 |
| 58 | UUAACAAGCCUAAGGUCUUTT | Sense strand ATs481, ATs489, ATs597 |
| 59 | AAGACCUUAGGCUUGUUAAGC | Antisense strand ATa481 |
| 60 | AAGACCTUAGGCUUGUUAAGC | Antisense strand ATa489, ATa597 |
| 61 | UGCAAGACUUGACACCGAATT | Sense strand ATs565, ATs603, ATs612, ATs786, ATs787 |
| 62 | UTCGGUGUCAAGUCUUGCAGC | Antisense strand ATa565, ATa603, ATa612, ATa786, ATa787 |
| 63 | GCUGCAAGACUUGACACCGAA | Sense strand 612-Als |
| 64 | UUCGGUGUCAAGUCUUGCAGCGA | Antisense strand 612-Ala |
| 65 | GACCCUACCUUCAUACCUGTT | Sense strand ATs579, ATs648 |
| 66 | CAGGUATGAAGGUAGGGUCUU | Antisense strand ATa579 |
| 67 | AAGACCCUACCUUCAUACCUG | Sense strand 579s-AL1 |
| 68 | CAGGUAUGAAGGUAGGGUCUUUG | Antisense strand 579a-AL |
| 69 | GCUUAACAAGCCUAAGGUCUU | Sense strand 597s-AL1 |
| 70 | AAGACCUUAGGCUUGUUAAGCUG | Antisense strand 597a-AL |
| 71 | CCAAUAGCUUAACAAGCCUTT | Sense strand ATs483, ATs484, ATs598, ATs600 |
| 72 | AGGCUUGUUAAGCUAUUGGGU | Antisense strand ATa483, ATa484, ATa598 |
| 73 | UGCAAAACUUGACACCGAATT | Sense strand ATs605 |
| 74 | UTCGGUGUCAAGUUUUGCAGC | Antisense strand ATa605 |
| 75 | CCAACAGCUUAACAAGCCUTT | Sense strand ATs606 |
| 76 | AGGCUUGUUAAGCUGUUGGGU | Antisense strand ATa606 |
| 77 | AGGCUUGUUAAGCUAUUGGUU | Antisense strand ATa609 |
| 78 | ACCCAAUAGCUUAACAAGCCT | Sense strand ATs610 |
| 79 | AGGCUUGUUAAGCUAUUGGGTAG | Antisense strand ATa610 |
| 80 | AGCUCAACAAGCCUGAGGUTT | Sense strand ATs613 |
| 81 | ACCUCAGGCUUGUUGAGCUGU | Antisense strand ATa613 |
| 82 | GUCAUCCACAAUGAGAGUACA | Sense strand ATXL-Gs |
| 83 | UGUACUCUCAUUGUGGAUGACGA | Antisense strand ATXL-Ga |
| 84 | CTGATCCAGCCTCACTATGC | Human AGT forward primer hsAGT-F |
| 85 | AGGTCATAAGATCCTTGCAGC | Human AGT reverse primer hsAGT-R |
| 86 | AGGGTCTCACTTTCCAGCAAAACTCC | Human AGT probe hsAGT-P |
| 87 | UAGUCGCUGCAAAACUUGATT | Sense strand ATs365m |
| 88 | UCAAGUTUUGCAGCGACUAGC | Antisense strand ATa365m |
| 89 | UAGUCGCUGCGAAACUUGATT | Sense strand ATs643, ATsi-643s1 |
| 90 | UCAAGUUUCGCAGCGACUAGC | Antisense strand ATa643 |
| 91 | GCUAGUCGCUGCGAAACUUGA | Sense strand 643-Als |
| 92 | UCAAGUUUCGCAGCGACUAGCAC | Antisense strand 643-Ala |
| 93 | ACCCAAUAGCUUAACAAGCCU | Sense strand 598s-AL1 |
| 94 | AGGCUUGUUAAGCUAUUGGGUAG | Antisense strand 598a-AL |
| 95 | GCUUAACAAGCCUAAGGUCUU | Sense strand ATs644 |
| 96 | AAGACCUUAGGCUUGUUAAGCUG | Antisense strand ATa644 |
| 97 | CAGGUAUGAAGGUAGGGUCUU | Antisense strand ATa648 |
| 98 | ACCCAAUAGCUUAACAAGCCUTT | Sense strand ATs633 |
| 99 | AGGCUUGUUAAGCUAUUGGGUTT | Antisense strand ATa633 |

## Claims

1. A double-stranded ribonucleic acid (dsRNA) compound for inhibiting expression of angiotensinogen (AGT) in a cell, wherein the dsRNA compound comprises a sense strand and an antisense strand forming a double stranded portion, wherein the sense strand comprises the nucleotide sequence of any one of SEQ ID NO: 8, 33 and 55, and wherein the antisense strand comprises the nucleotide sequence of any one of SEQ ID NO: 9 and 19.

2. The double-stranded ribonucleic acid (dsRNA) compound of claim 1, wherein the dsRNA compound is a shRNA or siRNA compound.

3. The double-stranded ribonucleic acid (dsRNA) of any preceding claim, further comprising a conjugate moiety, preferably wherein the conjugate moiety is an N-Acetylgalactosamine (GalNAc)-comprising moiety, and/or the conjugate moiety is attached to the 3' end of the sense strand.

4. The double-stranded ribonucleic acid (dsRNA) compound of any preceding claim, wherein the compound inhibits expression of angiotensinogen (AGT) in a cell by at least about 70%, 75%, 80%, 85%, 90%, 95%, 98% or 99%.

5. The double-stranded ribonucleic acid (dsRNA) compound of any preceding claim, wherein the dsRNA is a salt of the dsRNA.

6. A pharmaceutical composition for inhibiting expression of angiotensinogen (AGT) in a cell, wherein the pharmaceutical composition comprises the double-stranded ribonucleic acid (dsRNA) compound of any preceding claim, alone or in combination with a pharmaceutically acceptable carrier or excipient.

7. A kit comprising the compound of any one of claims 1-5, or the pharmaceutical composition of claim 6, and optionally, a label.

8. A double stranded ribonucleic acid (dsRNA) compound as defined in any of claims 1 to 5 for use in medicine.

9. A double stranded ribonucleic acid (dsRNA) compound as defined in any of claims 1 to 5 for use in treating or preventing a renin-angiotensin-aldosterone system (RAAS) associated disease, disorder and/or condition in a subject.

10. The double stranded ribonucleic acid (dsRNA) compound for use of claim 9, wherein the RAAS associated disease, disorder and/or condition is selected from the group consisting of high blood pressure, hypertension, borderline hypertension, primary hypertension, secondary hypertension isolated systolic or diastolic hypertension, pregnancy-associated hypertension, diabetic hypertension, resistant hypertension, refractory hypertension, paroxysmal hypertension, renovascular hypertension, Goldblatt hypertension, hypertension associated with low plasma renin activity or plasma renin concentration, ocular hypertension, glaucoma, pulmonary hypertension, portal hypertension, systemic venous hypertension, systolic hypertension, labile hypertension; hypertensive heart disease, hypertensive nephropathy, atherosclerosis, arteriosclerosis, vasculopathy, diabetic nephropathy, diabetic retinopathy, chronic heart failure, cardiomyopathy, diabetic cardiac myopathy, glomerulosclerosis, coarctation of the aorta, aortic aneurism, ventricular fibrosis, heart failure, myocardial infarction, angina, stroke, renal disease, renal failure, systemic sclerosis, intrauterine growth restriction (IUGR), fetal growth restriction, obesity, liver steatosis/ fatty liver, non-alcoholic Steatohepatitis (NASH), non-alcoholic fatty liver disease (NAFLD); glucose intolerance, type 2 diabetes (non-insulin dependent diabetes), and metabolic syndrome.

## Patentansprüche

1. Doppelsträngige Ribonukleinsäure-(dsRNA)-Verbindung zur Hemmung der Expression von Angiotensinogen (AGT) in einer Zelle, wobei die dsRNA-Verbindung einen Sense-Strang und einen Antisense-Strang umfasst, die einen doppelsträngigen Abschnitt bilden, wobei der Sense-Strang die Nukleotidsequenz einer der SEQ ID NO: 8, 33 und 55 umfasst und wobei der Antisense-Strang die Nukleotidsequenz einer der SEQ ID NO: 9 und 19 umfasst.

2. Doppelsträngige Ribonukleinsäure-(dsRNA)-Verbindung nach Anspruch 1, wobei die dsRNA-Verbindung eine shRNA- oder siRNA-Verbindung ist.

3. Doppelsträngige Ribonukleinsäure-(dsRNA)-Verbindung nach einem der vorhergehenden Ansprüche, ferner umfassend eine Konjugat-Einheit, wobei die Konjugat-Einheit vorzugsweise eine N-Acetylgalactosamin (GalNAc)-umfassende Einheit ist und/oder die Konjugat-Einheit an das 3'-Ende des Sense-Strangs gebunden ist.

4. Doppelsträngige Ribonukleinsäure-(dsRNA)-Verbindung nach einem der vorhergehenden Ansprüche, wobei die Verbindung die Expression von Angiotensinogen (AGT) in einer Zelle um mindestens etwa 70 %, 75 %, 80 %, 85 %, 90 %, 95 %, 98 % oder 99 % hemmt.

5. Doppelsträngige Ribonukleinsäure-(dsRNA)-Verbindung nach einem der vorhergehenden Ansprüche, wobei die dsRNA ein Salz der dsRNA ist.

6. Pharmazeutische Zusammensetzung zur Hemmung der Expression von Angiotensinogen (AGT) in einer Zelle, wobei die pharmazeutische Zusammensetzung die doppelsträngige Ribonukleinsäure-(dsRNA)-Verbindung nach einem der vorhergehenden Ansprüche allein oder in Kombination mit einem pharmazeutisch verträglichen Träger oder Hilfsstoff umfasst.

7. Kit, umfassend die Verbindung nach einem der Ansprüche 1 bis 5 oder die pharmazeutische Zusammensetzung nach Anspruch 6 und optional ein Etikett.

8. Doppelsträngige Ribonukleinsäure-(dsRNA)-Verbindung wie in einem der Ansprüche 1 bis 5 definiert zur Verwendung in der Medizin.

9. Doppelsträngige Ribonukleinsäure-(dsRNA)-Verbindung wie in einem der Ansprüche 1 bis 5 definiert zur Verwendung bei der Behandlung oder Vorbeugung einer/eines mit dem Renin-Angiotensin-Aldosteron-System (RAAS) assoziierten Krankheit, Störung und/oder Zustands bei einem Subjekt.

10. Doppelsträngige Ribonukleinsäure-(dsRNA)-Verbindung zur Verwendung nach Anspruch 9, wobei die/der RAAS-assoziierte Krankheit, Störung und/oder Zustand ausgewählt ist aus der Gruppe bestehend aus hohem Blutdruck, Hypertonie, grenzwertiger Hypertonie, primärer Hypertonie, sekundärer Hypertonie, isolierter systolischer oder diastolischer Hypertonie, schwangerschaftsassoziierter Hypertonie, diabetischer Hypertonie, resistenter Hypertonie, refraktärer Hypertonie, paroxysmaler Hypertonie, renovaskulärer Hypertonie, Goldblatt-Hypertonie, Hypertonie in Verbindung mit niedriger Plasma-Renin-Aktivität oder Plasma-Renin-Konzentration, okulärer Hypertonie, Glaukom, pulmonaler Hypertonie, portaler Hypertonie, systemischer venöser Hypertonie, systolischer Hypertonie, labiler Hypertonie; hypertensiver Herzerkrankung, hypertensiver Nephropathie, Atherosklerose, Arteriosklerose, Vaskulopathie, diabetischer Nephropathie, diabetischer Retinopathie, chronischer Herzinsuffizienz, Kardiomyopathie, diabetischer Kardiomyopathie, Glomerulosklerose, Koarktation der Aorta, Aortenaneurysma, ventrikulärer Fibrose, Herzinsuffizienz, Myokardinfarkt, Angina, Schlaganfall, Nierenerkrankung, Nierenversagen, systemischer Sklerose, intrauteriner Wachstumsrestriktion (IUGR), fetaler Wachstumsrestriktion, Adipositas, Lebersteatose/Fettleber, nichtalkoholischer Steatohepatitis (NASH), nichtalkoholischer Fettlebererkrankung (NAFLD); Glukoseintoleranz, Typ-2-Diabetes (nicht insulinabhängiger Diabetes) und metabolischem Syndrom.

## Revendications

1. Composé d'acide ribonucléique double brin (ARNdb) destiné à inhiber l'expression de l'angiotensinogène (AGT) dans une cellule, dans lequel le composé d'ARNdb comprend un brin sens et un brin antisens formant une portion double brin, dans lequel le brin sens comprend la séquence nucléotidique de l'une quelconque des SEQ ID NO: 8, 33 et 55, et dans lequel le brin antisens comprend la séquence nucléotidique de l'une quelconque des SEQ ID NO: 9 et 19.

2. Composé d'acide ribonucléique double brin (ARNdb) selon la revendication 1, dans lequel le composé d'ARNdb est un composé shRNA ou siRNA.

3. Composé d'acide ribonucléique double brin (ARNdb) selon l'une quelconque des revendications précédentes, comprenant en outre une fraction conjuguée, de préférence dans lequel la fraction conjuguée est une fraction comprenant de la N-acétylgalactosamine (GalNAc), et/ou la fraction conjuguée est attachée à l'extrémité 3' du brin sens.

4. Composé d'acide ribonucléique double brin (ARNdb) selon l'une quelconque des revendications précédentes, dans lequel le composé inhibe l'expression de l'angiotensinogène (AGT) dans une cellule d'au moins environ 70 %, 75 %, 80 %, 85 %, 90 %, 95 %, 98 % ou 99 %.

5. Composé d'acide ribonucléique double brin (ARNdb) selon l'une quelconque des revendications précédentes, dans lequel l'ARNdb est un sel de l'ARNdb.

6. Composition pharmaceutique destinée à inhiber l'expression de l'angiotensinogène (AGT) dans une cellule, dans laquelle la composition pharmaceutique comprend le composé d'acide ribonucléique double brin (ARNdb) selon l'une quelconque des revendications précédentes, seul ou en combinaison avec un véhicule ou excipient pharmaceutiquement acceptable.

7. Kit comprenant le composé selon l'une quelconque des revendications 1 à 5, ou la composition pharmaceutique selon la revendication 6, et éventuellement une étiquette.

8. Composé d'acide ribonucléique double brin (ARNdb) tel que défini dans l'une quelconque des revendications 1 à 5 pour une utilisation en médecine.

9. Composé d'acide ribonucléique double brin (ARNdb) tel que défini dans l'une quelconque des revendications 1 à 5 pour une utilisation dans le traitement ou la prévention d'une maladie, d'un trouble et/ou d'une affection associés au système rénine-angiotensine-aldostérone (SRAA) chez un sujet.

10. Composé d'acide ribonucléique double brin (ARNdb) pour une utilisation selon la revendication 9, dans lequel la maladie, le trouble et/ou l'affection associés au SRAA sont choisis dans le groupe constitué par l'hypertension artérielle, l'hypertension, l'hypertension limite, l'hypertension primaire, l'hypertension secondaire, l'hypertension systolique ou diastolique isolée, l'hypertension associée à la grossesse, l'hypertension diabétique, l'hypertension résistante, l'hypertension réfractaire, l'hypertension paroxystique, l'hypertension rénovasculaire, l'hypertension de Goldblatt, l'hypertension associée à une faible activité rénine plasmatique ou à une faible concentration de rénine plasmatique, l'hypertension oculaire, le glaucome, l'hypertension pulmonaire, l'hypertension portale, l'hypertension veineuse systémique, l'hypertension systolique, l'hypertension labile, la cardiopathie hypertensive, la néphropathie hypertensive, l'athérosclérose, l'artériosclérose, la vasculopathie, la néphropathie diabétique, la rétinopathie diabétique, l'insuffisance cardiaque chronique, la cardiomyopathie, la myopathie cardiaque diabétique, la glomérulosclérose, la coarctation de l'aorte, l'anévrisme aortique, la fibrose ventriculaire, l'insuffisance cardiaque, l'infarctus du myocarde, l'angine, l'accident vasculaire cérébral, la maladie rénale, l'insuffisance rénale, la sclérose systémique, le retard de croissance intra-utérin (RCIU), le retard de croissance fœtale, l'obésité, la stéatose hépatique/la stéatose du foie gras, la stéatohépatite non alcoolique (NASH), la stéatose hépatique non alcoolique (NAFLD), l'intolérance au glucose, le diabète de type 2 (diabète non insulinodépendant) et le syndrome métabolique.
